# EUROPEAN PATENT APPLICATION

(11) **EP 3 712 170 A1**
(43) Date of publication of application: **23.09.2020**
(21) Application number: 18875520.1
(22) Date of filing: 09.11.2018
(51) Int. Cl.: C07K 16/28, C12N 15/13, C12N 15/63, C12N 5/10, A61K 39/395, A61P 35/00, A61P 31/00

(54) **CD96 ANTIBODY, ANTIGEN-BINDING FRAGMENT AND PHARMACEUTICAL USE THEREOF**

(30) Priority: 10.11.2017 CN 201711107331
(71) Applicant: Jiangsu Hengrui Medicine Co., Ltd., Jiangsu 222047 (CN); Shanghai Hengrui Pharmaceutical Co., Ltd, Shanghai 200245 (CN)
(72) Inventor: CAO, Zhuoxiao, Shanghai 200245 (CN); FU, Yayuan, Shanghai 200245 (CN); YU, Cunjing, Shanghai 200245 (CN); HU, Qiyue, Shanghai 200245 (CN); TAO, Weikang, Shanghai 200245 (CN)
(74) Representative: dompatent von Kreisler Selting Werner - Partnerschaft von Patent- und Rechtsanwälten mbB
(86) International application number: PCT/CN2018/114797
(87) International publication number: WO 2019/091449

(57) **Abstract**

Provided are a CD96 antibody, an antigen-binding fragment and a pharmaceutical use thereof. Further provided are a murine antibody, a chimeric antibody and a humanized antibody comprising the CDR region of the CD96 antibody, and a pharmaceutical composition comprising the CD96 antibody and the antigen-binding fragment thereof, and the use of same as a drug. In particular, provided is a use of a humanized CD96 antibody in the preparation of a drug for treating CD96-related diseases or conditions.

## Description

The present disclosure claims the priority of Chinese patent application 201711107331.5, filed on November 10, 2017, the contents of which are incorporated herein by its entirety.

### Field of the invention

The present disclosure relates to a CD96 antibody and an antigen-binding fragment thereof. Further, the present disclosure also relates to a chimeric antibody and a humanized antibody comprising the CDR region of CD96 antibody. The present disclosure also relates to a pharmaceutical composition comprising the CD96 antibody and the antigen-binding fragment thereof, and the use of same as a diagnostic agent and a therapeutic agent for CD96 related diseases.

### Background

The statements herein merely provide background information related to the present invention and do not necessarily constitute prior arts.

In 1992, Wang and his colleagues discovered and cloned a new cell membrane-type molecule, which was then named TACTILE (T cell activation increased late expression). The molecule was subsequently named CD96 at an international workshop and conference on Human Leukocyte Differentiation Antigens. CD96 molecules are expressed on normal T cells, T cell clones and certain transformed T cells. Peripheral blood T cells expressed low levels of CD96 molecules, which was significantly upregulated after activation, and reached an expression peak on day 6-9 after stimulation. Under the stimulation of alloantigen, the expression of CD96 on NK cells was also upregulated. CD96 belongs to the immunoglobulin superfamily, and its extracellular membrane region contains three immunoglobulin-like domains, with a total of 15 N-linked glycosylation sites and a stem-like domain having highly O-linked glycosylation rich in serine/threonine/proline residues. CD96 has a transmembrane region containing 24 amino acid residues, a cytoplasmic region containing 44 amino acid residues, and a region rich in basic animo acid/proline. In the more than ten years since the CD96 molecule was cloned, there has been no significant progress in the study of the molecule because its ligand is not clear. CD96 is widely expressed in hematopoietic and non-hematopoietic cell lines. CD96 is also expressed in normal human CD4⁺T cells, CD8⁺T cells, monocytes and NK cells. After PHA stimulation, CD96 expression in CD4⁺T cells, CD8⁺T cells and NK cells was significantly upregulated, confirming that CD96 is a molecule with increased activation expression. However, PHA did not significantly upregulate the expression of CD96 in monocytes, suggesting that different cells has different characteristics in regulating the expression of CD96 molecules. Until 2004, Fuchs and his colleagues discovered that NK cells can recognize PVR (CD155) through CD96, promote the adhesion of NK cells to target cells which express CD155, promote the cytotoxicity of NK cells and mediate the internalization of CD155 on the surface of target cells. Since PVR (CD155) is highly expressed in certain tumor cells, this receptor system may play an important role in the recognition and killing of tumors by NK cells. However, so far the understanding of the expression and function of CD96 is still very limited. It is believed that with the continuous understanding of the function of this molecule, CD96 will surely attract more attention.

The cytoplasmic region of the CD96 molecule contains an immunoreceptor tyrosine inhibitory motif (ITIM), which is highly conserved among species. It is generally believed that ITIM is involved in inhibiting signal transmission. Recent studies have found that certain molecules can transmit activation signals through ITIM. Receptors containing ITIM motifs play a key role in the activation of signal transmission and the recruitment of SHP-2. Therefore, CD96 may mediate the inhibition of NK cell activity. In addition, CD96 and CD226 have certain homology in sequence. According to the current research, it is believed that those two molecules can inhibit or activate NK cells through binding to CD155, respectively, so as to achieve the function of regulating NK cells.

So far, only CD96 antibody molecules reported in CN105636983 and CN105636985, etc. show a reduced immunosuppression in animals, or CD96 antibody molecules in EP2097535 are used to eliminate CD96+ AMLSC cells. No specific CD96 antibody molecules have been reported yet, and no CD96 antibody molecules have been clinically studied. Therefore, it is necessary to develop CD96 antibodies with high affinity and targeting specific epitopes for studying the functions of CD96 and CD96 antibody drugs.

### Content of the invention

The present disclosure provides a monoclonal antibody or an antigen-binding fragment (also referred to as CD96-binding molecules) that specifically binds the amino acid sequence or three-dimensional structure of CD96.

In one aspect, the present disclosure provides a monoclonal antibody or an antigen-binding fragment thereof that specifically binds to human CD96, wherein the monoclonal antibody comprises a heavy chain variable region and a light chain variable region, wherein:
(i) the heavy chain variable region comprises HCDR variants selected from the group consisting of 3, 2, 1 or 0 amino acid mutations on the basis of the HCDR1, HCDR2 and HCDR3, respectively, wherein the HCDR1, HCDR2 and HCDR3 are shown in SEQ ID NO: 16, 17 and 18, respectively; the light chain variable region comprises LCDR variants selected from the group consisting of 3, 2, 1 or 0 amino acid mutations on the basis of the LCDR1, LCDR2 and LCDR3, respectively, wherein the LCDR1, LCDR2 and LCDR3 are shown in SEQ ID NO: 19, 20 and 21, respectively; or
(ii) the heavy chain variable region comprises HCDR variants selected from the group consisting of 3, 2, 1 or 0 amino acid mutations on the basis of the HCDR1, HCDR2 and HCDR3, respectively, wherein the HCDR1, HCDR2 and HCDR3 are shown in SEQ ID NO: 22, 23 and 24, respectively; the light chain variable region comprises LCDR variants selected from the group consisting of 3, 2, 1 or 0 amino acid mutations on the basis of the LCDR1, LCDR2 and LCDR3, respectively, wherein the LCDR1, LCDR2 and LCDR3 are shown in SEQ ID NO: 25, 26 and 27, respectively; or
(iii) the heavy chain variable region comprises HCDR variants selected from the group consisting of 3, 2, 1 or 0 amino acid mutations on the basis of the HCDR1, HCDR2 and HCDR3, respectively, wherein the HCDR1, HCDR2 and HCDR3 are shown in SEQ ID NO: 28, 29 and 30, respectively; the light chain variable region comprises LCDR variants selected from the group consisting of 3, 2, 1 or 0 amino acid mutations on the basis of the LCDR1, LCDR2 and LCDR3, respectively, wherein the LCDR1, LCDR2 and LCDR3 are shown in SEQ ID NO: 31, 32 and 33, respectively; or
(iv) the heavy chain variable region comprises HCDR variants selected from the group consisting of 3, 2, 1 or 0 amino acid mutations of the HCDR1, HCDR2 and HCDR3, respectively, wherein the HCDR1, HCDR2 and HCDR3 are shown in SEQ ID NO: 34, 35 and 36, respectively; the light chain variable region comprises LCDR variants selected from the group consisting of 3, 2, 1 or 0 amino acid mutations of the LCDR1, LCDR2 and LCDR3, respectively, wherein the LCDR1, LCDR2 and LCDR3 are shown in SEQ ID NO: 37, 38 and 39, respectively; or
(v) the heavy chain variable region comprises HCDR variants selected from the group consisting of 3, 2, 1 or 0 amino acid mutations on the basis of the HCDR1, HCDR2 and HCDR3, respectively, wherein the HCDR1, HCDR2 and HCDR3 are shown in SEQ ID NO: 40, 41 and 42, respectively; the light chain variable region comprises LCDR variants selected from the group consisting of 3, 2, 1 or 0 amino acid mutations on the basis of the LCDR1, LCDR2 and LCDR3, respectively, wherein the LCDR1, LCDR2 and LCDR3 are shown in SEQ ID NO: 43, 44 and 45, respectively. The amino acid mutation is preferably amino acid substitution, and more preferably conservative amino acid substitution.

In some embodiments, CDR variants of the monoclonal antibody or the antigen-binding fragment (including 3 heavy chain CDRs and 3 light chain CDRs) having 3, 2 or 1 amino acid mutations is the CDR variants having 3, 2 or 1 amino acid mutations screened by affinity maturation methods.

In some embodiments, the monoclonal antibody or the antigen-binding fragment has an affinity (KD) to CD96 of less than 10⁻⁷M, less than 10⁻⁸M, less than 10⁻⁹M, less than 10⁻¹⁰M or less than 10⁻¹¹M.

In some embodiments, the present disclosure provides a monoclonal antibody or an antigen-binding fragment thereof that specifically binds human CD96, wherein the monoclonal antibody comprises a heavy chain variable region and a light chain variable region, wherein:
(vi) the heavy chain variable region comprises HCDR1, HCDR2 and HCDR3 regions having the amino acid sequences of SEQ ID NO: 16, 17 and 18, respectively; the light chain variable region comprises LCDR1, LCDR2 and LCDR3 regions having the amino acid sequences of SEQ ID NO: 52, 20 and 21, respectively, wherein the LCDR1 is preferably selected from any one of SEQ ID NO: 19, 108, 109 and 110; or
(vii) the heavy chain variable region comprises HCDR1, HCDR2 and HCDR3 regions having the amino acid sequences of SEQ ID NO: 22, 23 and 64, respectively; the light chain variable region comprises LCDR1, LCDR2 and LCDR3 regions having the amino acid sequences of SEQ ID NO: 25, 26 and 27, respectively, wherein the HCDR3 is preferably selected from any one of SEQ ID NO: 24, 111, 112, 113, 114, 115 and 116; or
(viii) the heavy chain variable region comprises CDR1, HCDR2 and HCDR3 regions having the amino acid sequences of SEQ ID NO: 28, 29 and 30, respectively; the light chain variable region comprises LCDR1, LCDR2 and LCDR3 regions having the amino acid sequences of SEQ ID NO: 31, 32 and 33, respectively; or
(ix) the heavy chain variable region comprises HCDR1, HCDR2 and HCDR3 regions having the amino acid sequences of SEQ ID NO: 34, 35 and 36, respectively; the light chain variable region comprises LCDR1, LCDR2 and LCDR3 regions having the amino acid sequences of SEQ ID NO: 37, 38 and 39, respectively; or
(x) the heavy chain variable region comprises HCDR1, HCDR2 and HCDR3 regions having the amino acid sequences of SEQ ID NO: 40, 119 and 42, respectively; the light chain variable region comprises LCDR1, LCDR2 and LCDR3 regions shown as the amino acid sequences of SEQ ID NO: 43, 44 and 45, respectively, wherein the HCDR2 is preferably selected from SEQ ID NO: 41, 120 or 121.

In some embodiments, the monoclonal antibody or the antigen-binding fragment is a recombinant antibody, preferably selected from a murine antibody, a chimeric antibody and a humanized antibody.

In some embodiments, the monoclonal antibody or the antigen-binding fragment thereof comprises a light chain variable region, or a heavy chain variable region, or a light chain variable region and a heavy chain variable region selected from the following:
a. the heavy chain variable region having the sequence of any one of SEQ ID NO: 6, 46, 49, 50 and 51, or a variant thereof, and/or the light chain variable region having the sequence of any one of SEQ ID NO: 7, 47, 48, 53, 54, 55, 56, 57 and 58, or a variant thereof; or
b. the heavy chain variable region having the sequence of any one of SEQ ID NO: 8, 59, 62, 63, 65, 66, 67, 68, 69 and 70, or a variant thereof, and/or the light chain variable region having the sequence of any one of SEQ ID NO: 9, 60 and 61, or a variant thereof; or
c. the sequence of the heavy chain variable region having the sequence of any one of SEQ ID NO: 10, or a variant thereof, and/or the light chain variable region having the sequence of any one of SEQ ID NO: 11, or a variant thereof;
d. the heavy chain variable region having the sequence of any one of SEQ ID NO: 12, 71, 75, 76 and 77, or a variant thereof, and/or the light chain variable region having the sequence of any one of SEQ ID NO: 13, 72, 73 and 74, or a variant thereof; or
e. the heavy chain variable region having the sequence of any one of SEQ ID NO: 14, 78, 82, 83, 84, 122 and 123, or a variant thereof, and/or the light chain variable region having the sequence of any one of SEQ ID NO: 15, 79, 80, and 81, or a variant thereof;

Wherein the variant in a to e has 1, 2, 3, 4, 5, 6, 7, 8, 9 or 10 amino acid mutations in the sequence of the framework region of the light chain variable region or the heavy chain variable region.

In some embodiments, the monoclonal antibody or the antigen-binding fragment thereof comprises a heavy chain variable region selected from the group consisting of:
(a) a heavy chain variable region or a variant thereof having the amino acid sequence of shown in any one of SEQ ID NO: 6, 46, 49, 50 and 51;
(b) a heavy chain variable region or a variant thereof having the amino acid sequence of any one of SEQ ID NO: 8, 59, 62, 63, 65, 66, 67, 68, 69 and 70;
(c) a heavy chain variable region or a variant thereof having the amino acid sequence of SEQ ID NO: 10;
(d) a heavy chain variable region or a variant thereof having the amino acid sequence of any one of SEQ ID NO: 12, 71, 75, 76 and 77;
(e) a heavy chain variable region or a variant thereof having the amino acid sequence of any one of SEQ ID NO: 14, 78, 82, 83, 84, 122 and 123;

Wherein the variant in (a) to (e) has 1, 2, 3, 4, 5, 6, 7, 8, 9 or 10 amino acid mutations in the framework region sequence of the light chain variable region or the heavy chain variable region.

In some embodiments, the monoclonal antibody or antigen-binding fragment thereof comprises a light chain variable region selected from the group consisting of:
(a) a light chain variable region or a variant thereof having the amino acid sequence of any one of SEQ ID NO: 7, 47, 48, 53, 54, 55, 56, 57 and 58;
(b) a light chain variable region or a variant thereof having the amino acid sequence of any one of SEQ ID NO: 9, 60 and 61;
(c) a light chain variable region or a variant thereof having the amino acid sequence of SEQ ID NO: 11;
(d) a light chain variable region or a variant thereof having the amino acid sequence of any one of SEQ ID NO: 13, 72, 73 and 74;
(e) a light chain variable region or a variant thereof having the amino acid sequence of any one of SEQ ID NO: 15, 79, 80 and 81;

Wherein the variant in (a) to (e) has 1, 2, 3, 4, 5, 6, 7, 8, 9 or 10 amino acid mutations in the framework region sequence of the light chain variable region or the heavy chain variable region, the amino acid mutation is preferably a back mutation of a framework region amino acid.

In some embodiments, the antibody or antigen-binding fragment comprises:
f. the heavy chain variable region having the sequence selected from any one of SEQ ID NOs: 6, 46, 49, 50 and 51, and the light chain variable region having the sequence selected from any one of SEQ ID NO: 7, 47, 48, 53, 54, 55, 56, 57 and 58; or
g. the heavy chain variable region having the sequence selected from the sequences shown in any one of SEQ ID NOs: 8, 59, 62, 63, 65, 66, 67, 68, 69 and 70, and the light chain variable region having the sequence selected from any one of SEQ ID NO: 9, 60, and 61; or
h. the heavy chain variable region having the sequence of SEQ ID NO: 10, and the light chain variable region having the sequence of SEQ ID NO: 11;
i. the heavy chain variable region having the sequence selected from any one of SEQ ID NOs: 12, 71, 75, 76 and 77, and the light chain variable region having the sequence selected from any one of SEQ ID NOs: 13, 72, 73 and 74; or
j. the heavy chain variable region having the sequence selected from any one of 14, 78, 82, 83, 84, 122 and 123, and the light chain variable region having the sequence selected from any one of SEQ ID NOs: 15, 79, 80 and 81.

In some embodiments, the antibody is a full-length antibody, further comprising a human antibody constant region, preferably comprising a human antibody heavy chain constant region of SEQ ID NO: 117 and/or a human antibody light chain constant region of SEQ ID NO: 118.

In some embodiments, the antigen-binding fragment is selected from the group consisting of Fab, Fab', F(ab')2, single chain variable fragment (scFv), dimerized domain V (diabody), disulfide stabilized Fv (dsFv) and antigen-binding fragments of CDR-containing peptides.

In some embodiments, the antibody or antigen-binding fragment binds to human CD96 with an affinity of a KD value of 1× 10⁻⁷M to 1× 10⁻¹²M as determined by, for example, surface plasmon resonance (BIACORE).

In another aspect, the present disclosure also provides an isolated monoclonal antibody or an antigen-binding fragment thereof that competes with said monoclonal antibodies (i)-(x) or a-j or an antigen-binding fragment thereof to bind to human CD96.

In some embodiments, the monoclonal antibody or the antigen-binding fragment thereof has at least one of the following characteristics:
i. binding to human CD96 with an affinity of a KD value of 1×10⁻⁷M to 1×10⁻¹²M as determined by, for example, surface plasmon resonance (BIACORE);
ii. cross-reacting with CD96 of cynomolgus monkey or rhesus monkey;
iii. blocking the binding of human CD96 to human CD155;
iv. increased activation of NK cells and/or T cells;
v. blocking the inhibition of NK cell activation induced by the binding of CD96 to CD155.

In some embodiments, the monoclonal antibody or the antigen-binding fragment thereof binds to a region in the extracellular region of human CD96 (SEQ ID NO: 3) as shown by IAVYHPQYGFYCAYGRPCES.

In another aspect, the present disclosure also provides a multispecific antibody comprising a light chain variable region and/or a heavy chain variable region of anti-CD96 antibody or the antigen-binding fragment thereof described above.

In another aspect, the present disclosure also provides a single chain antibody comprising a light chain variable region and/or a heavy chain variable region of the anti-CD96 antibody or the antigen-binding fragment thereof described above.

In another aspect, the present disclosure also provides a pharmaceutical composition comprising a therapeutically effective amount of the monoclonal antibody or the antigen-binding fragment thereof, the multispecific antibody or the single chain antibody described above, and one or more pharmaceutically acceptable carriers, diluents, buffers, or excipients, preferably the pharmaceutical composition may contain 0.01 to 99% by weight of the anti-CD96 antibody or the antigen-binding fragment thereof in a unit dose, or the amount of the monoclonal antibody or the antigen-binding fragment thereof in a unit dose of the pharmaceutical composition is 0.1-2000 mg, more preferably 1-1000 mg.

In another aspect, the present disclosure also provides an isolated nucleic acid molecule encoding the monoclonal antibody or the antigen-binding fragment thereof, or the multispecific antibody, or the single chain antibody as defined in any one of the preceding aspects.

In another aspect, the present disclosure also provides a recombinant vector comprising said isolated nucleic acid molecule.

In another aspect, the present disclosure also provides a host cell transformed with said recombinant vector, wherein the host cell was selected from a prokaryotic cell and a eukaryotic cell, preferably a eukaryotic cell, more preferably a mammalian cell.

In another aspect, the present disclosure also provides a method for producing the monoclonal antibody or the antigen-binding fragment thereof as defined in any one of the preceding aspects, wherein the method comprises culturing said host cell in a medium to produce and accumulate the monoclonal antibody or the antigen-binding fragment thereof, the multispecific antibody or the single chain antibody as defined in any one of the preceding aspects, and harvesting the monoclonal antibody or the antigen-binding fragment thereof from a culture.

In another aspect, the present disclosure also provides a method for detecting or measuring human CD96 in vitro, the method comprises using the monoclonal antibody or the antigen-binding fragment thereof, or the multispecific antibody, or the single chain antibody as defined in any one of the preceding aspects.

In another aspect, the present disclosure also provides a use of the monoclonal antibody or the antigen-binding fragment thereof, or the multispecific antibody, or the single chain antibody as defined in any one of the preceding aspects in the preparation of a reagent for detecting or measuring human CD96.

In another aspect, the present disclosure also provides a method of reducing or alleviating immunosuppression, the method comprises administering to a subject a therapeutically effective amount of the monoclonal antibody or the antigen-binding fragment thereof, or the multispecific antibody, or the single chain antibody, or the pharmaceutical composition or the isolated nucleic acid molecule as defined in any one of the preceding aspects.

In another aspect, the present disclosure also provides a method for enhancing the activity of NK cells, wherein the method comprises a step of reducing CD96 activity using the monoclonal antibody or the antigen-binding fragment thereof, or the multispecific antibody or the single chain antibody, or the pharmaceutical composition or the isolated nucleic acid molecule as defined in any one of the preceding aspects.

In another aspect, the present disclosure also provides a use of the monoclonal antibody or the antigen-binding fragment thereof, the multispecific antibody, or the single chain antibody, or the pharmaceutical composition or the isolated nucleic acid molecule as defined in any one of the preceding aspects in the preparation of a medicament for enhancing the activity of NK cells.

In another aspect, the present disclosure also provides a method for treating diseases associated with human CD96, the method comprises administering to a subject a therapeutically effective amount of the monoclonal antibody or the antigen-binding fragment thereof, or the multispecific antibody, or the single chain antibody, or the pharmaceutical composition or the isolated nucleic acid molecule as defined in any one of the preceding aspects, to treat diseases associated with human CD96, wherein the disease is preferably a tumor, a cancer or an infectious disease.

In another aspect, the present disclosure also provides a use of the monoclonal antibody or the antigen-binding fragment thereof, the multispecific antibody, the single chain antibody, the pharmaceutical composition or the isolated nucleic acid molecule as defined in any one of the preceding aspects, or a combination thereof, in the preparation of a medicament for treating or preventing a disease or disorder, wherein the disease or disorder is a disease associated with human CD96, the disease or disorder is preferably a tumor, a cancer or an infectious disease.

In another aspect, the present disclosure also provides a medicament of the monoclonal antibody or the antigen-binding fragment thereof, the multispecific antibody, the single chain antibody, the pharmaceutical composition or the isolated nucleic acid molecule as defined in any one of the preceding aspects.

In another aspect, the present disclosure also provides the monoclonal antibody or the antigen-binding fragment thereof, the multispecific antibody, the single chain antibody, or the pharmaceutical composition or the isolated nucleic acid molecule as defined in any one of the preceding aspects as a medicament, wherein the medicament is used for the treatment of diseases associated with human CD96, wherein the disease is preferably a tumor, a cancer or an infectious disease.

Alternatively, the cancer can be any cancer that responds to at least partially blocked CD96-mediated immunosuppression, suppression, or peripheral tolerance. Examples of cancer include, but are not limited to, carcinoma, lymphoma, blastoma, sarcoma, and leukemia or lymphoid malignancy. More specific examples of the cancers include squamous cell carcinoma, myeloma, small cell lung cancer, non-small cell lung cancer (NSCLC), head and neck squamous cell carcinoma (HNSCC), glioma, Hodgkin's lymphoma , Non-Hodgkin's lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), T-cell/histocyte-rich large B-cell lymphoma, multiple myeloma, myeloid cell leukelia -1 protein (Mcl-1), myelodysplastic syndrome (MDS), gastrointestinal cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric carcinoma, bone cancer, Ewing's sarcoma, cervical cancer, brain cancer, gastric carcinoma, bladder cancer, hepatocellular carcinoma, breast cancer, colon cancer, hepatocellular carcinoma (HCC), clear cell renal cell carcinoma (RCC), head and neck cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative tumor, neuroendocrine neoplasm, Merkel cell carcinoma, testicular cancer, and skin cancer. In some embodiments, the cancer is metastatic cancer that has the ability to migrate to another site, tissue or organ within the body and form a tumor at that site.

Typically, a disease or condition that responds to at least partially blocked CD96-mediated immunosuppression, suppression, or peripheral tolerance is any disease or condition that enhances or restores immune surveillance to benefit a subject suffering from the disease or condition. The diseases and conditions may include persistent diseases or conditions that can be controlled or suppressed by cell-mediated immunity. Non-limiting examples include tumors, cancer or infectious diseases. Specifically, examples of cancers related to the present disclosure include, but are not limited to, carcinoma, lymphoma, blastoma, sarcoma, and leukemia or lymphoid malignancy. More specific examples of the cancers include squamous cell carcinoma, myeloma, small cell lung cancer, non-small cell lung cancer (NSCLC), head and neck squamous cell carcinoma (HNSCC), glioma, Hodgkin's lymphoma , Non-Hodgkin's lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), T-cell/histocyte-rich large B-cell lymphoma, multiple myeloma, myeloid cell leukelia -1 protein (Mcl-1), myelodysplastic syndrome (MDS), gastrointestinal cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric carcinoma, bone cancer, Ewing's sarcoma, cervical cancer, brain cancer, gastric carcinoma, bladder cancer, hepatocellular carcinoma, breast cancer, colon cancer, hepatocellular carcinoma (HCC), clear cell renal cell carcinoma (RCC), head and neck cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative tumor, neuroendocrine neoplasm, Merkel cell carcinoma, testicular cancer, and skin cancer. In some embodiments, the cancer is metastatic cancer that has the ability to migrate to another site, tissue or organ within the body and form a tumor at that site.

The infectious diseases involved include all diseases caused by virus or pathogen infection which can be infected through respiratory organs, blood contact or skin contact. Non-limiting examples of these infectious diseases include, but are not limited to, hepatitis B, hepatitis C, human papilloma virus (HPV) infection, cytomegalovirus infection, viral respiratory disease, influenza, and Epstein-Barr virus (EBV) infection , Herpes simplex virus (HSV includes HSV1 and HSV2) infection, varicella-zoster virus (VSV) infections, cytomegalovirus (CMV) infections, and the like.

The CD96 monoclonal antibody or the antigen-binding fragment of the present disclosure has high specificity and high affinity to CD96, and the immunogenicity of the humanized antibody is greatly reduced, while the specificity, high affinity and activity of the murine antibody are completely retained.

### Brief description of the drawings

Figure 1: Expreiments of CD96 humanized antibody blocking hCD96-CHOs cell binding.
Figure 2: Expreiments of CD96 humanized antibody blocking hCD96 antigen and hCD155-CHO cell binding.
Figure 3: Antibody competition experiments. In Figure 3A the coated antibody (A antibody) is h1718-012; In Figure 3B the coated antibody is h1719-014; In Figure 3C the coated antibody is h1721-003; In Figure 3D the coated antibody is ch1720; In Figure 3E the coated antibody is h1722- 010.
Figure 4: CD96-CHOs cell binding experiment (FACS test) of the CD96 chimeric antibody/peptide complex. Figure 4A shows the testing results of ch1718 with 3# peptides. Figure 4B shows the testing results of ch1719 with 3# peptides. Figure 4C shows the testing results of ch1720 with different peptides. Figure 4D shows the testing results of ch1721 with different peptides.
Figure 5: ch1718, ch1719 and ch1720 promote the killing effect of NK cells on tumor cells, E:T=1:1.

### Detailed description of the invention

### Term

In order to more easily understand the present invention, certain technical and scientific terms are specifically defined below. Unless otherwise defined herein, all other technical and scientific terms used herein have the meaning commonly understood by those skilled in the art to which this invention belongs.

Three-letter codes and one-letter codes of amino acids used in the present disclosure are described in J.biol.chem,243,p3558(1968).

The 'antibody' in the present disclosure refers to an immunoglobulin, which is a tetrapeptide chain structure formed by connecting two identical heavy chains and two identical light chains through interchain disulfide bonds. The amino acid composition and sequence of the constant region of the immunoglobulin heavy chain are different, so is their antigenicity. According to this, immunoglobulins can be divided into five classes, or called isotypes, that is, IgM, IgD, IgG, IgA and IgE, and the corresponding heavy chains are µ chain, δ chain, γ chain, α chain, and ε chain, respectively. Ig of the same class can be divided into different subclasses according to the difference in amino acid composition of hinge regions and the number and position of heavy chain disulfide bonds, for example, IgG can be classified into IgG1, IgG2, IgG3 and IgG4. The light chain is divided into a κ chain or a λ chain by the difference of constant regions. Each of the five classes of Ig can have a κ chain or a λ chain.

In the present disclosure, the antibody light chain as defined in the present disclosure may further comprise a light chain constant region, and the light chain constant region comprises a human or murine κ, λ chain, or a variant thereof.

In the present disclosure, the antibody heavy chain as defined in the present disclosure may further comprise a heavy chain constant region, and the heavy chain constant region comprises a human or murine IgG1, IgG2, IgG3, IgG4 or a variant thereof.

The variable region (Fv region) comprises about 110 amino acids with great sequence variation, which are located at the N-terminus of the heavy chain and the light chain of the antibody; the constant region comprising the remaining amino acids is relatively stable in sequence and located at the C-terminus of that of antibody. The variable region comprises three hypervariable regions (HVRs) and four relatively conserved framework regions (FRs). Three hypervariable regions determine the specificity of the antibody, which are also known as complementarity determining regions (CDRs). Each light chain variable region (LCVR) and heavy chain variable region (HCVR) consists of 3 CDR regions and 4 FR regions. The order from amino terminal to carboxy terminal is: FR1, CDR1, FR2, CDR2, FR3, CDR3, FR4. Three CDR regions of the light chain are referred to as LCDR1, LCDR2 and LCDR3; and three CDR regions of the heavy chain are referred to as HCDR1, HCDR2 and HCDR3. The number and location of CDR amino acid residues in the LCVR regions and HCVR regions of the antibody or antigen-binding fragment as define in the present disclosure conform to the known Kabat numbering system (LCDR1-3, HCDR1-3).

The antibodies of the present disclosure include murine antibodies, chimeric antibodies, humanized antibodies, preferably humanized antibodies.

The term 'murine antibody' in this disclosure is an anti human CD96 monoclonal antibody prepared according to the knowledge and skill in the art. Test subjects are injected with CD96 antigen during preparation, and hybridomas expressing antibodies with the desired sequence or functional characteristics are isolated. In a preferred embodiment of the present disclosure, the anti-murine CD96 antibody or the antigen-binding fragment thereof may further comprise a light chain constant region comprising a murine κ, λ chain or variants thereof, or further comprise a heavy chain constant region of murine IgG1, IgG2 , IgG3 or variants thereof.

The term 'chimeric antibody' is an antibody obtained by fusing the variable region of a murine antibody with the constant region of a human antibody, which can reduce the immune response induced by the murine antibody. To construct a chimeric antibody, the first thing is to establish a hybridoma that secrets murine specific monoclonal antibody, then clone the variable region gene from the murine hybridoma cell, and then clone the constant region gene of the human antibody as required. The murine variable region gene is linked with the human constant region gene to form a chimeric gene and subsequently the chimeric gene is inserted into an expression vector. Finally, the chimeric antibody molecule is expressed in a eukaryotic system or a prokaryotic system. In a certain embodiment of the present disclosure, the antibody light chain of the CD96 chimeric antibody further comprises a light chain constant region comprising a human κ, λ chain or a variant thereof. The antibody heavy chain of the CD96 chimeric antibody further comprises a heavy chain constant region of human IgG1, IgG2, IgG3, IgG4 or a variant thereof, preferably a human IgG1, IgG2 or IgG4 heavy chain constant region, or a virant of IgG1, IgG2 or IgG4 heavy chain constant region with amino acid mutation (Eg, YTE mutation or back mutation).

The term 'humanized antibody' refers to an antibody produced by grafting a murine CDR sequence into a framework of human antibody variable region, that is, an antibody produced from different type of human germline antibody framework sequences. It can overcome the heterogeneous response induced by the chimeric antibody which carries a large amount of murine protein components. Such framework sequences can be obtained from a public DNA database containing germline antibody gene sequences or published references. For example, germline DNA sequences of human heavy and light chain variable region genes can be obtained from the 'VBase' human germline sequence database (Website: www.mrccpe.com.ac.uk/vbase), and in Kabat, EA, etc., 1991 Sequences of Proteins of Immunological Interest, 5th edition. In order to avoid the decrease in activity caused by the decrease in immunogenicity, the framework sequence of human antibody variable region may be subjected to minimal reverse mutation or back mutation to maintain the activity. The humanized antibodies of the present disclosure also include humanized antibodies with affinity maturation of CDRs by phage display. In a preferred embodiment of the present disclosure, the murine CDR sequence of the CD96 humanized antibody is selected from the group consisting of SEQ ID NO: 16-21, 22-27, 28-33, 34-39 or 40-45; the human antibody variable region framework is designed and selected, wherein the heavy chain FR sequence on the antibody heavy chain variable region is derived from the human germline heavy chain sequence and the human germline light chain sequence. In order to avoid the decrease in activity caused by the decrease in immunogenicity, the human antibody variable region can be subjected to minimum of reverse mutation (back mutation, that is, amino acid residues in FR from the human antibody is mutated to the amino acid residues in corresponding position of original antibody) to retain activation.

The grafting of CDRs may result in a decrease in the affinity of the produced CD96 antibody or the antigen-binding fragment thereof to the antigen due to changes in the framework residues in contact with the antigen. Such interactions can be the result of highly mutated somatic cells. Therefore, it may still be necessary to graft such donor framework amino acids to the framework of a humanized antibody. Amino acid residues involved in antigen binding from non-human CD96 antibody or the antigen-binding fragment thereof can be identified by examining the sequence and structure of the variable region of murine monoclonal antibody. Residues in the CDR donor framework that differ from the germline can be considered related. If the closest germline cannot be determined, the sequence can be compared to the consensus sequence of subtype or the consensus sequence of murine sequence with a high percentage of similarity. Rare framework residues are believed to be the result of high frequency mutations of somatic cells and thus play an important role in binding.

In the description similar to 'CDR variants with 3, 2, 1 or 0 amino acid mutations of the CDR1, CDR2 and CDR3 having SEQ ID NO: X, SEQ ID NO: Y and SEQ ID NO: Z, respectively', an exemplary explanation is the mutations of CDR may comprise 3, 2, 1 or 0 amino acid mutations, and the same or different number of amino acid residues may be optionally selected between CDR1, CDR2 and CDR3 for mutation. For example, on the basis of the CDR1, CDR2 and CDR3 shown in SEQ ID NO: X, SEQ ID NO: Y and SEQ ID NO: Z, 1 amino acid of mutation is made to CDR1 and 0 amino acid of mutation is made to CDR2 and CDR3. When 0 amino acid of mutation is made to a CDR, the CDR variant with 0 amino acid mutation is still the CDR itself.

The term 'antigen-binding fragment' or 'functional fragment' of an antibody refers to one or more fragments of an antibody that retain the ability to specifically bind an antigen (eg, CD96). It has been shown that fragments of a full-length antibody can be used to achieve the antigen-binding function of the antibody. Examples of binding fragments indicated in the term 'antigen-binding fragment' of an antibody include (i) a monovalent Fab fragment consisting of VL, VH, CL and CHI domains; (ii) a F(ab')₂ fragment including bivalent fragment of two Fab fragments connected by a disulfide bridge on the hinge region, (iii) Fd fragment consisting of VH and CHI domains; (iv) Fv fragment consisting of VH domain and VL domain of single-armed of the antibody; (v) a single domain or dAb fragment (Ward et al. (1989)Nature341: 544-546) consisting of a VH domain; and (vi) an isolated complementary determining region (CDR) or (vii) optionally a combination of two or more separate CDRs connected by a synthetic linker. In addition, although the two domains VL and VH of Fv fragment are encoded by separate genes, these genes can be combined through a synthetic linker using recombinant methods, thus producing a single protein chain that is a monovalent molecule formed by pairing VL and VH regions (referred to as single-chain Fv (scFv); see refs, eg, Bird et al. (1988)Science 242: 423-426; and Huston et al. (1988)Proc. Natl. Acad. Sci USA 85: 5879-5883). Such scFvs are also intended to be included in the term "antigen-binding fragment" of an antibody. Such antibody fragments are obtained using conventional techniques known to those skilled in the art, and screened by their functionality in the same manner as intact antibodies. Antigen-binding moieties can be produced by recombinant DNA technology or by enzymatic or chemical cleavage of intact immunoglobulins. The antibodies may be antibodies of different isotypes, for example, IgG (eg, IgG1, IgG2, IgG3 or IgG4 subtypes), IgA1, IgA2, IgD, IgE or IgM antibodies.

The antigen-binding fragments of the present disclosure include Fab, F(ab')2, Fab', single chain variable fragment (scFv), dimerized domain V (diabody), disulfide stabilized Fv (dsFv), CDR-containig peptides, etc.

Fab is an antibody fragment having a molecular weight of about 50,000 and antigen-binding activity of the fragments obtained by treating IgG antibody molecule with a papain (cleaves the amino acid residue at position 224 of the H chain), wherein about half of the N-terminal side of the H chain and the entire L chain are connected together by disulfide bonds.

The Fab of the present disclosure can be produced by treating the monoclonal antibody of the present disclosure that specifically recognizes and binds to the amino acid sequence of the extracellular region of human CD96 or its three-dimensional structure with papain. In addition, the Fab can be produced by inserting DNA encoding the Fab of the antibody into a prokaryotic expression vector or a eukaryotic expression vector and transforming the vector into a prokaryote or eukaryote to express the Fab.

F(ab')2 is an antibody fragment having a molecular weight of about 100,000, antigen-binding activity and two Fab regions connected at hinge positions obtained by cleaving the lower portions of two disulfide bonds in IgG hinge region with enzyme pepsin.

F(ab')2 of the present disclosure can be produced by treating the monoclonal antibody of the present disclosure that specifically recognizes and binds to the amino acid sequence of the extracellular region of human CD96 or its three-dimensional structure with pepsin. In addition, the F(ab')2 can be produced by connecting Fab's described below with a thioether bond or a disulfide bond.

Fab' is an antibody fragment having a molecular weight of about 50,000 and antigen-binding activity obtained by cleaving the disulfide bond of the hinge region of F(ab')2 described above. The Fab' of the present disclosure can be produced by treating F(ab')2 of the present disclosure that specifically recognizes and binds to the amino acid sequence of the extracellular region of CD96 or its three-dimensional structure with a reducing agent such as dithiothreitol.

In addition, the Fab' can be produced by inserting DNA encoding a Fab' fragment of the antibody into a prokaryotic expression vector or a eukaryotic expression vector and transforming the vector into a prokaryote or eukaryote to express the Fab'.

The term 'single-chain antibody', 'single-chain Fv' or 'scFv' refers to the molecule including an antibody heavy chain variable domain (or region; VH) and an antibody light chain variable domain (or region; VL) conjugated by a linker. Such scFv molecules may have a general structure: NH₂-VL-linker-VH-COOH or NH₂-VH-linker-VL-COOH. Suitable linkers of prior arts consist of repeated GGGGS amino acid sequences or variants thereof, for example using 1-4 repeated variants (Holliger et al. (1993), Proc. Natl. Acad. Sci. USA90:6444-6448). Other linkers can be used for the present disclosure are described by Alfthan et al. (1995), Protein Eng.8:725-731, Choi et al. (2001), Eur.J.Immuno 1.31:94-106, Hu et al. (1996), Cancer Res.56:3055-3061, Kipriyanov et al. (1999), J. Mol. Biol. 293:41-56 and Roovers et al. (2001), Cancer Immunol.

The scFv of the present disclosure can be produced using the following steps: obtaining the cDNA encoding VH and VL of monoclonal antibody of the present disclosure that specifically recognizes and binds to the amino acid sequence of the extracellular region of human CD96 or its three-dimensional structure, and constructing the DNA encoding scFv, inserting the DNA into a prokaryotic or eukaryotic expression vector, and then transforming the expression vector into a prokaryote or eukaryote to express the scFv.

Diabodies are antibody fragments in which scFv is dimerized and possess bivalent antigen-binding activity. In a bivalent antigen-binding activity, the two antigens may be the same or different.

The diabody of the present disclosure can be produced using the following steps: obtaining the cDNA encoding VH and VL of the monoclonal antibody of the present disclosure that specifically recognizes and binds to the amino acid sequence of the extracellular region human of CD96 or its three-dimensional structure, and constructing the DNA encoding scFv so that the length of the amino acid sequence of the peptide linker is 8 residues or less, inserting the DNA into a prokaryotic or eukaryotic expression vector, and then transforming the expression vector into a prokaryote or eukaryote to express the diabody.

dsFv is obtained by linking a polypeptide in which one amino acid residue in each of VH and VL is substitued by a cysteine residue via disulfide bond between the cysteine residues. The amino acid residues substituted by cysteine residues can be selected according to a known method (Protein Engineering, 7,697(1994)) based on the three-dimensional structure prediction of the antibody.

The dsFv of the present disclosure can be produced using the following steps: obtaining the cDNA encoding VH and VL of the monoclonal antibody of the present disclosure that specifically recognizes and binds to the amino acid sequence of the extracellular region of human CD96 or its three-dimensional structure, and constructing DNA encoding dsFv, inserting the DNA into a prokaryotic or eukaryotic expression vector, and then transforming the expression vector into a prokaryote or eukaryote to express the dsFv.

A CDR-containing peptide consists of one or more CDRs that contain VH or VL. Peptides containing multiple CDRs can be conjugated directly or via a suitable peptide linker.

The CDR-containing peptides of the present disclosure can be produced using the following steps: constructing DNA encoding CDRs containing VH and VL of the monoclonal antibody of the present disclosure that specifically recognizes and binds to the amino acid sequence of the extracellular region of human CD96 or its three-dimensional structure, inserting the DNA into a prokaryotic or eukaryotic expression vector, and then transforming the expression vector into a prokaryote or eukaryote to express the peptide. The CDR-containing peptide can also be produced by a chemical synthesis method such as the Fmoc method or the tBoc method.

The term 'antibody framework' as used herein refers to a part of variable domain VL or VH, which serves as a scaffold for the antigen-binding loop (CDR) of the variable domain. In essence, it is a variable domain without CDR.

The term 'amino acid mutation' refers to mutation of certain or some animo acid positions on a fragment of polypeptide and variants thereof, wherein the variant can be obtained by subtituting, inserting or deleting amino acids at one or some sites on the polypeptide.

The term 'epitope' or 'antigenic determinant' refers to a part on an antigen to which an immunoglobulin or antibody specifically binds (eg, certain parts on CD96 molecule). An epitope typically includes at least 3, 4, 5, 6, 7, 8, 9, 10, 11, 12, 13, 14 or 15 consecutive or non-consecutive amino acids in a unique spatial conformation. See, for example, Epitope Mapping Protocols in Methods in Molecular Biology, vol.66, G. E. Morris, Ed. (1996).

The terms 'specifically bind', 'selectively bind', 'bind selectively' and 'bind specifically' refer to the binding of an antibody to epitopes on a predetermined antigen. Generally, antibodies bind antigens with an affinity (KD) of less than about 10⁻⁸M, such as about less than 10⁻⁹M, 10⁻¹⁰M, 10⁻¹¹M, or less.

The term 'KD' refers to the dissociation equilibrium constant of a particular antibody-antigen interaction. Generally, antibodies of the present disclosure bind CD96 with a dissociation equilibrium constant (KD) of less than about 10⁻⁷M, such as less than about 10⁻⁸M, 10⁻⁹M, or 10⁻¹⁰M or less, for example, using surface plasmon resonance (SPR) technology to measure the constant by a BIACORE instrument.

When the term 'competition' is used in the case of antigen-binding proteins competing for the same epitope (such as neutralizing antigen-binding proteins or neutralizing antibodies or specifically binding antibodies), it means competition between the antigen-binding proteins, which is determined by by the following assay: the antigen-binding protein (e.g., an antibody or an immunologically functional fragment thereof) to be tested prevents or inhibits (e.g., reduces) the binding of a reference antigen-binding protein (e.g., a ligand or reference antibody) to a common antigen (e.g., a CD96 antigen or a fragment thereof). Numerous types of competitive binding assays can be used to determine whether one antigen-binding protein competes with another, such as: solid-phase direct or indirect radioimmunoassay (RIA), solid-phase direct or indirect enzyme immunoassay (EIA), sandwich competition assay (see, eg, Stahli et al., 1983, Methodsin Enzymology 9:242-253); solid phase direct biotin-avidin EIA (see, eg, Kirkland et al., 1986, J. Immunol. 137:3614-3619), solid phase direct labeling assay, solid phase direct labeling sandwich assay (see, eg, Harlow and Lane, 1988, (Antibodies, A Laboratory Manual), Cold Spring Harbor Press); solid phase direct labeling RIA using 1-125 (see, for example, Morel et al., 1988, Molec. Immunol.25:7-15); solid phase direct biotin-avidin EIA (see, for example, Cheung, et al., 1990, Virology176:546-552); and direct labeling RIA (Moldenhauer et al., 1990, Scand.J.Immunol.32:77-82); or by a method such as Emobodiment 7 of the present disclosure. Generally, the assays involve the use of a purified antigen (the antigen is on a solid surface or a cell surface) capable of binding to an unlabeled detecting antigen-binding protein and a labeled reference antigen-binding protein. Competitive inhibition is measured by measuring the amount of label binding to a solid surface or cell in the presence of the test antigen-binding protein. Or, as provided in Embodiment 7 herein of the method for determining competitive binding, competitive inhibition is determined by immobilizing antigen-binding protein A and detecting the change of the labeled antigen signal which pre-binds to the antigen-binding protein. Usually, this competitive inhibition test is confirmed by swapping the positions of antigen-binding protein A and antigen-binding protein B. Antigen-binding proteins identified by the competitive assays (competing antigen-binding protein) include: an antigen-binding protein that binds to the same epitope as a reference antigen-binding protein; and an antigen-binding protein that binds to an epitope adjacent to an epitope that is sufficiently close to the reference antigen-binding protein, while the two epitopes spatially hinder the binding of each other. Gnerally, when there is an excess of competing antigen-binding proteins, part of the competitive antigen-binding proteins inhibit (e.g., reduce) at least 40%, at least 50%, at least 55%, at least 60%, at least 65%, at least 70%, at least 75% of the specific binding of the reference antigen-binding protein to the common antigen. In the case of complete competition, the binding of the reference antigen-binding protein to the antigen is inhibited by at least 80%, at least 85%, at least 90%, at least 95%, at least 97%, at least 98%, at least 99% or more. Inhibition of less than 30% is considered non-competitive. In the method for testing competitive binding provided in Embodiment 7, it is considered as competition or partial competition, when the the competitive antigen-binding protein can inhibit the binding of the reference antigen-binding protein in the degree defined above whether it is as antigen-binding protein A or as antigen-binding protein B.

'Conservative amino acid modification' or 'conservative amino acid substitution' or 'conservative amino acid replacement' refers to amino acids in proteins that are substituted by other amino acids that share similar characteristics (such as charge, side chain size, hydrophobicity/hydrophilicity, main chain conformation and rigidity, etc.), thus, the changes can be made frequently without altering the biological activity or other desired properties (such as antigen affinity and/or specificity) of the proteins. Those skilled in the art recognize that, in general, single amino acid substitutions in non-essential regions of a polypeptide do not substantially alter biological activity of the polypeptide (see, eg, Watson et al. (1987) Molecular Biology of the Gene, The Benjamin/Cummings Pub. Co., P224 (4th edition)). In addition, the substitution of structurally or functionally similar amino acid is unlikely to disrupt its biological activity. Exemplary conservative substitutions are set forth in the following table 'Exemplary conservative amino acid substitutions'.

**Exemplary Conservative Amino Acid Substitutions**

| Original Residue | Conservative Substitution |
|---|---|
| Ala(A) | Gly; Ser |
| Arg(R) | Lys; His |
| Asn(N) | Gln; His; Asp |
| Asp(D) | Glu; Asn |
| Cys(C) | Ser; Ala; Val |
| Gln(Q) | Asn; Glu |
| Glu(E) | Asp; Gln |
| Gly(G) | Ala |
| His(H) | Asn; Gln |
| Ile(I) | Leu; Val |
| Leu(L) | Ile; Val |
| Lys(K) | Arg; His |
| Met(M) | Leu; Ile; Tyr |
| Phe(F) | Tyr; Met; Leu |
| Pro(P) | Ala |
| Ser(S) | Thr |
| Thr(T) | Ser |
| Trp(W) | Tyr; Phe |
| Tyr(Y) | Trp; Phe |
| Val(V) | Ile; Leu |

The term 'nucleic acid molecule', as used herein, refers to both DNA molecules and RNA molecules. The nucleic acid molecule may be single-stranded or double-stranded, preferably double-stranded DNA. A nucleic acid is 'effectively linked' when it is placed in a functional relationship with another nucleic acid sequence. For example, if a promoter or enhancer affects transcription of a coding sequence, the promoter or enhancer is operatively linked to the coding sequence.

The term 'vector' refers to a nucleic acid molecule capable of transporting another nucleic acid to which it is linked. In one embodiment, the vector is a 'plasmid', which refers to a circular double-stranded DNA loop to which other DNA segments can be linked. In another embodiment, the vector is a viral vector, wherein other DNA segments can be linked into the viral genome. The vectors disclosed herein are capable of autonomous replication in host cells into which they have been introduced (e.g., bacterial vectors with bacterial origins of replication and episomal mammalian vectors) or can be integrated into the host cell's genome after transfecting into the host cell, thereby replicating together with the host genome (eg, non-episomal mammalian vectors).

Methods for producing and purifying antibodies and antigen-binding fragments are well known in the prior art, such as Cold Spring Harbor's Antibody Experiment Technical Guide, Chapters 5-8 and 15. For example, mice can be immunized with human CD96 or fragments thereof, and the resulting antibodies can be renatured, purified, and the amino acid can be sequenced using conventional methods. Antigen-binding fragments can also be prepared by conventional methods. The antibody or the antigen-binding fragment as defined in the invention is genetically engineered to add one or more human FR regions into a non-human CDR region. The human FR germline sequence can be obtained by aligning the IMGT human antibody variable region germline gene database and MOE software from ImMunoGeneTics (IMGT) website http://imgt.cines.fr, or from the Journal of Immunoglobulins, 2001ISBN012441351.

The term 'host cell' refers to a cell into which an expression vector has been introduced. Host cells maybe microorganism (eg, bacteria), plant or animal cells. Bacteria that are easy to be transformed include members of the *enterobacteriaceae*, such as strains of *Escherichia coli* or *Salmonella*; *Bacilillaceae*, such as *Bacillus subtilis*; *Pneumococcus*; *Streptococcus* and *Haemophilus influenzae.* Suitable microorganisms include *Saccharomyces cerevisiae* and *Pichia pastoris.* Suitable animal host cell lines include CHO (Chinese Hamster Ovary Cell Line), HEK293 and NSO cells.

The engineered antibodies or the antigen-binding fragments of the present disclosure can be prepared and purified using conventional methods. For example, cDNA sequences encoding heavy and light chains can be cloned and recombined into a GS expression vector. The recombinant immunoglobulin expression vector can be stably transfected into CHO cells. As a more recommended prior art, mammalian expression systems may cause glycosylation of antibodies, especially the highly conserved N-terminal site in the Fc region. Stable clones are obtained by expressing antibodies that specifically bind to human CD96. Positive clones were expanded in serum-free medium in the bioreactor to produce antibodies. The culture medium in which the antibody is secreted can be purified by conventional techniques, for example, an A or G Sepharose FF column with adjusted buffer. Non-specifically bound components are washed away. Then bound antibody was eluted by pH gradient method, and antibody fragments were detected by SDS-PAGE and pooled. The antibody can be concentrated by filtration using a conventional method. Soluble mixtures and polymers can also be removed by conventional methods, such as molecular sieve, ion exchange. The resulting product needs to be immediately frozen, such as -70°C, or lyophilized.

'Alleviation of immunosuppression' means to inhibit or suppress the immune function of one or more cells that normally express CD96 and at least partially eliminate, remove or overcome the normal activity or function of CD96. Typically, one or more cells that normally express CD96 are T cells, including CD4⁺ and CD8⁺ T cells, γδ T cells, NKT cells, and natural killer (NK) cells. In some embodiments, alleviation of immunosuppression may include or involve the abolition of peripheral tolerance to foreign pathogens, host cells exhibiting foreign pathogens (e.g., displaying foreign pathogen-derived polypeptides in autologous MHC) and/or the cancer cells or tissues of the host.

The term 'blocking' refers to the ability of the antibody or the antigen-binding fragment thereof of the present disclosure to block, partially block, interfere with, decrease, inhibit, reduce or inactivate a target protein, ie, CD96 and/or its ligands (Such as CD155). Thus, those skilled in the art understand that the term 'blocking' may cover the loss of all and/or part of the activity of the ligand or receptor. The activity of the ligand or receptor can be suppressed or inhibited by a compound that binds to the active site of the ligand/receptor protein, or by other means, such as inactivating a second protein that activates the inhibited first protein. For example, all and/or partial inhibition of the interaction between CD96 and CD155 can be characterized by increased NK cell activation.

The term 'tumor' refers to all the growth and proliferation of neoplastic cells, no matter malignant or benign, and all pre-cancerous and cancerous cells and tissues.

The terms 'cancer' and 'cancerous' refer to or describe a physiological disease in mammals that is typically characterized by unregulated cell growth. The terms 'cancer', 'cancerous', 'cell proliferative disorder', 'proliferative disorder' and 'tumor' are not mutually exclusive when mention in the present invention.

When applied to an animal, human, subject, cell, tissue, organ or biological fluid, 'give', 'administer' and 'treat'refer to the contact of an exogenous drug, therapeutic agent, diagnostic agent or composition to animal, human, subject, cell, tissue, organ or biological fluid. 'give', 'administer' and 'treat' may refer to, for example, treatment, pharmacokinetics, diagnosis, research and experimental methods. Treatment of a cell includes contact of a reagent with a cell, and contact of a reagent with a fluid, wherein the fluid is in contact with the cell. 'Give', 'administer' and 'treat' also mean treating cells *in vitro* and *ex vivo* by an agent, diagnosis, binding composition, or by another cell. When applied to a human, veterinary or research subject, 'treat' refers to therapeutic treatment, preventive or prophylactic measures, research and diagnostic applications.

'Therapy' means the administration to a patient of an internal or external therapeutic agent, such as a composition comprising any one of the antibodies or the antigen-binding fragments thereof, or the nucleic acid molecules encoding the antibodies or the antigen-binding fragments thereof, said patient has one or a variety of disease symptoms, and the therapeutic agents are known to have therapeutic effect on these symptoms. Generally, a therapeutic agent is administered to a patient or population under treatment in an amount effectively alleviate the symptoms of one or more diseases to induce the deterioration of such symptoms or inhibit the development of such symptoms to any clinically measurable degree. The amount of therapeutic agent (also known as a 'therapeutically effective amount') that is effective in alleviating the symptoms of any particular disease can vary depending on lots of factors, such as the patient's disease state, age and weight, and the ability of the drug to give the desired effect in the patient. Whether the symptoms of the disease have been alleviated can be evaluated by any clinical test method that a doctor or other health care professional usually uses to assess the severity or progression of the symptoms. Although embodiments of the present disclosure (e.g., treatment methods or articles) may not be effective in alleviating each symptom of target disease, they should reduce symptoms of the target disease in a statistically significant number of patients confirmed by any statistical test method known in the art such as Student t-test, Chi-square test, Mann and Whitney's The U test, Kruskal-Wallis test (H test), Jonckheere-Terpstra test and Wilcoxon test.

An 'effective amount' includes an amount sufficient to ameliorate or prevent the symptoms or conditions of a medical disease. An effective amount also means an amount sufficient to allow or facilitate diagnosis. An effective amount for a particular patient or veterinary subject can vary depending on factors such as the condition to be treated, the patient's overall health, the route and dosage of administration, and the severity of side effects. An effective amount can be the maximum dose or dosage regimen to avoid significant side effects or toxic effects.

'Exogenous' refers to a substance that is produced outside the organism, cell or human as appropriate. 'Endogenous' refers to a substance that is produced in a cell, organism or human body as appropriate.

'Homology' refers to sequence similarity between two polynucleotide sequences or between two polypeptides. When positions in two compared sequences are occupied by the same bases or amino acid monomer subunits, for example, if each position of two DNA molecules is occupied by adenine, then the molecules are homologous at that position. The percent of homology between two sequences is a function of the number of matching or homologous positions shared by the two sequences divided by the number of compared positions ×100. For example, when the sequences are optimally compared, if 10 positions in the two sequences have 6 matches or homology, then the two sequences are 60% homologous; if 100 positions in the two sequences have 95 matches or homology, then the two sequences are 95% homologous. In general, comparisons are made when the two sequences are compared for the greatest percentage of homology.

As used herein, the expressions 'cell', 'cell line' and 'cell culture' are used interchangeably, and all such names include offspring. Thus, the words 'transformants' and 'transformed cells' include primary test cells and cultures derived therefrom regardless of the number of passages. It should also be understood that due to intentional or unintentional mutations, all offspring cannot have the exactly same DNA content. The mutant offsprings that have the same functional or biological activity as those originally screened in the transformed cells are included. Where different names are meant, the meaning of which are clearly understood from the context.

As used herein, 'polymerase chain reaction' or 'PCR' refers to a procedure or technique in which a specific amount of nucleic acid, RNA and/or DNA is amplified as described in, for example, US Patent No.4,683,195. Generally, it is necessary to obtain sequence information from the terminal of or ouside the target region so that oligonucleotide primers can be designed; these primers are identical or similar in sequence to the corresponding strands of the template to be amplified. The 5'terminal nucleotides of the two primers may coincide with the terminal of the material to be amplified. PCR can be used to amplify specific RNA sequences, specific DNA sequences from total genomic DNA and cDNA, phage or plasmid sequences transcribed from total cellular RNA. See generally Mullis et al. (1987) Cold Spring Harbor Symp. Ouant. Biol. 51:263; Edited by Erlich, (1989) PCR TECHNOLOGY (Stockton Press, N.Y.). The PCR used herein is considered as an example, but not the only example, of a nucleic acid polymerase reaction method for amplifying the test nucleic acid sample. The method includes using known nucleic acids as primers and nucleic acid polymerases to amplify or produce specific parts of nucleic acids.

'Optional' or 'optionally' means that the event or environment described later may, but not necessarily, occur, and the description includes situations where the event or environment occurs or not. For example, 'optionally comprising 1-3 antibody heavy chain variable regions' means that an antibody heavy chain variable region of a particular sequence may, but not necessarily, be present.

'Pharmaceutical composition' means a mixture containing one or more of the compounds or a physiological/pharmaceutically acceptable salt or prodrug thereof described herein with other chemical components, such as physiological/pharmaceutically acceptable carriers and excipients. The purpose of the pharmaceutical composition is to promote the administration to the organism, which is beneficial to the absorption of the active ingredient and exerts the biological activity.

In addition, the present disclosure includes a medicament for treating a disease associated with CD96, comprising a monoclonal antibody or an antibody fragment thereof of the present disclosure as an active ingredient.

There is no limitation on the diseases related to CD96, as long as it is a disease associated with CD96, for example, the therapeutic response induced by the molecules disclosed in the present disclosure can be reduced by binding human CD96 for blocking the binding of CD96 to its ligand CD155, thereby reducing NK inhibition of cells. Therefore, the molecules of the present disclosure are very useful for those who suffer a tumor, cancer or infectious disease when in preparations and formulations suitable for therapeutic applications.

Further, the present disclosure relates to a method for immunodetection or measurement of CD96, a reagent for immunodetection or measurement of CD96, a method for immunodetection or measurement of cells expressing CD96, and a diagnostic agent for diagnosing a disease associated with CD96. It contains the monoclonal antibody or the antibody fragment that specifically recognizes and binds to the amino acid sequence of the extracellular region or its three-dimensional structure of human CD96 as an active ingredient.

In the present disclosure, the method for detecting or determining the amount of CD96 may be any known method. For example, it includes immunological detection or measurement method.

The immunodetection or measurement method is a method for detecting or measuring the amount of antibody or antigen using a labeled antigen or antibody. Examples of the immunodetection or measurement method include radioimmunoassay using radioactive substance-labeled immune antibody method (RIA), enzyme immunoassay (EIA or ELISA), fluorescent immunoassay (FIA), luminescent immunoassay, western blotting, physicochemical method, etc.

The above-mentioned diseases associated with CD96 can be diagnosed by detecting or measuring cells expressing CD96 with the monoclonal antibodies or antibody fragments of the present disclosure.

In order to detect cells expressing a polypeptide, a known immunodetection method can be used, preferably an immunoprecipitation method, a fluorescent cell staining method, an immunotissue staining method, or the like. In addition, a fluorescent antibody staining method using the FMAT8100HTS system (Applied Biosystem) can be used.

In the present disclosure, there is no particular restriction on the living sample for detecting or measuring CD96, as long as it has the possibility of including cells expressing CD96, such as tissue cells, blood, plasma, serum, pancreatic juice, urine, feces, tissue fluid or culture fluid.

The diagnostic agent containing the monoclonal antibody or the antibody fragment thereof of the present disclosure may further contain an agent for performing an antigen-antibody reaction or an agent for testing the reaction according to a required diagnostic method. Reagents for performing the antigen-antibody reaction include buffers, salts and the like. The reagent for measurement includes reagents commonly used in immunodetection or measurement methods, such as a labeled second antibody that recognizes the monoclonal antibody, the antibody fragment thereof or conjugate thereof and a substrate corresponding to the label, and the like.

### Detailed description of the preferred embodiment

The disclosure is further described below with reference to the embodiments, but these embodiments are not intended to limit the scope of the disclosure. Experimental methods without specifying certain conditions in the embodiments of the present disclosure are generally in accordance with the conventional conditions, such as Using Antibodies: A Laboratory Manual, and Molecular Cloning: A Laboratory Manual, Cold Spring Harbor; or the conditions proposed by the manufacturers of raw materials or commodities. Reagents are commercially available conventional reagents unless otherwised specified.

### Example 1. Design and expression of CD96 antigen protein

Human CD96 protein (Uniprot: P40200-2) was used as a template for the CD96 of the present disclosure to design the amino acid sequences of the antigens and proteins for following assays involved in the present disclosure. Optionally, the CD96 proteins fused with different tags were cloned into the pHr vector (self-made) or the pXC-17.4 vector (LONZA) respectively, and transiently expressed in 293 cells or stably expressed in CHO cells, thus purifying the encoded antigen and the protein to be tested of the present disclosure. The following CD96 antigens refer to human CD96 unless otherwise specified.

CD96 extracellular region with Flag tag (SEQ ID NO: 1): CD96-Flag, for immunizing mice

MEKKWKYCAVYYIIQIHFVKGVWEKTVNTEENVYATLGSDVNLTCQTQTVGFFV
QMQWSKVTNKIDLIAVYHPQYGFYCAYGRPCESLVTFTETPENGSKWTLHLRNMSCS
VSGRYECMLVLYPEGIQTKIYNLLIQTHVTADEWNSNHTIEIEINQTLEIPCFQNSSSKISS
EFTYAWSVEDNGTQETLISQNHLISNSTLLKDRVKLGTDYRLHLSPVQIFDDGRKFSCHI
RVGPNKILRSSTTVKVFAKPEIPVIVENNSTDVLVERRFTCLLKNVFPKANITWFIDGSFL
HDEKEGIYITNEERKGKDGFLELKSVLTRVHSNKPAQSDNLTIWCMALSPVPGNKVWN
ISSEKITFLLGSEISSTDPPLSVTESTLDTQPSPASSVSPARYPATSSVTLVDVSALRPNTTP
QPSNSSMTTRGFNYPWTSSGTDTKKSVSRIPSETYSSSPSGAGSTLHDNVFTSTARAFSE
VPTTANGSTKTNHVHITGIVVNKPKDGMDYKDDDDK

Note: The underlined part is the signal peptide and the italic part is the Flag-tag.

Fusion protein of CD96 extracellular domain and mIgG2a Fc (SEQ ID NO: 2): CD96-mFc for immunization

MEFGLSWLFLVAILKGVQCVWEKTVNTEENVYATLGSDVNLTCQTQTVGFFVQM
QWSKVTNKIDLIAVYHPQYGFYCAYGRPCESLVTFTETPENGSKWTLHLRNMSCSVSG
RYECMLVLYPEGIQTKIYNLLIQTHVTADEWNSNHTIEIEINQTLEIPCFQNSSSKISSEFT
YAWSVEDNGTQETLISQNHLISNSTLLKDRVKLGTDYRLHLSPVQIFDDGRKFSCHIRV
GPNKILRSSTTVKVFAKPEIPVIVENNSTDVLVERRFTCLLKNVFPKANITWFIDGSFLH
DEKEGIYITNEERKGKDGFLELKSVLTRVHSNKPAQSDNLTIWCMALSPVPGNKVWNIS
SEKITFLLGSEISSTDPPLSVTESTLDTQPSPASSVSPARYPATSSVTLVDVSALRPNTTPQP
SNSSMTTRGFNYPWTSSGTDTKKSVSRIPSETYSSSPSGAGSTLHDNVFTSTARAFSEVP
TTANGSTKTNHVHITGIVVNKPKDGMEPRGPTIKPCPPCKCPAPNLLGGPSVFIFPPKIKD
VLMISLSPIVTCVVVDVSEDDPDVQISWFVNNVEVHTAQTQTHREDYNSTLRVVSALPIQHQ

DWMSGKEFKCKVNNKDLPAPIERTISKPKGSVRAPQVYVLPPPEEEMTKKQVTLTCMVTDF
MPEDIYVEWTNNGKTELNYKNTEPVLDSDGSYFMYSKLRVEKKNWVERNSYSCSVVHEGLH
NHHTTKSFSRTPGK

Note: The underlined part is the signal peptide and the italic part is the mFc.

Full-length CD96 (SEQ ID NO: 3): for constructiing CD96 overexpressing cell lines for immunization and test

MEKKWKYCAVYYIIQIHFVKGVWEKTVNTEENVYATLGSDVNLTCQTQTVGFF
VQMQWSKVTNKIDLIAVYHPQYGFYCAYGRPCESLVTFTETPENGSKWTLHLRNMSC
SVSGRYECMLVLYPEGIQTKIYNLLIQTHVTADEWNSNHTIEIEINQTLEIPCFQNSSSKI
SSEFTYAWSVEDNGTQETLISQNHLISNSTLLKDRVKLGTDYRLHLSPVQIFDDGRKFS
CHIRVGPNKILRSSTTVKVFAKPEIPVIVENNSTDVLVERRFTCLLKNVFPKANITWFID
GSFLHDEKEGIYITNEERKGKDGFLELKSVLTRVHSNKPAQSDNLTIWCMALSPVPGN
KVWNISSEKITFLLGSEISSTDPPLSVTESTLDTQPSPASSVSPARYPATSSVTLVDVSAL
RPNTTPQPSNSSMTTRGFNYPWTSSGTDTKKSVSRIPSETYSSSPSGAGSTLHDNVFTST
ARAFSEVPTTANGSTKTNHVHITGIVVNKPKDGMSWPVIVAALLFCCMILFGLGVRKWC
QYQKEIMERPPPFKPPPPPIKYTCIQEPNESDLPYHEMETL

Note: The underlined part is the signal peptide, the normal part is the extracellular region, and the italic part is the transmembrane region and the intracellular region.

CD96 extracellular region with His tag (SEQ ID NO: 4): CD96-His, for following assays

MEKKWKYCAVYYIIQIHFVKGVWEKTVNTEENVYATLGSDVNLTCQTQTVGFFV
QMQWSKVTNKIDLIAVYHPQYGFYCAYGRPCESLVTFTETPENGSKWTLHLRNMSCS
VSGRYECMLVLYPEGIQTKIYNLLIQTHVTADEWNSNHTIEIEINQTLEIPCFQNSSSKISS
EFTYAWSVEDNGTQETLISQNHLISNSTLLKDRVKLGTDYRLHLSPVQIFDDGRKFSCHI
RVGPNKILRSSTTVKVFAKPEIPVIVENNSTDVLVERRFTCLLKNVFPKANITWFIDGSFL
HDEKEGIYITNEERKGKDGFLELKSVLTRVHSNKPAQSDNLTIWCMALSPVPGNKVWN
ISSEKITFLLGSEISSTDPPLSVTESTLDTQPSPASSVSPARYPATSSVTLVDVSALRPNTTP
QPSNSSMTTRGFNYPWTSSGTDTKKSVSRIPSETYSSSPSGAGSTLHDNVFTSTARAFSE
VPTTANGSTKTNHVHITGIVVNKPKDGMHHHHHH

Note: The underlined part is the signal peptide, the italic part is His tag.

Fusion protein of CD96 extracellular region and hIgG1Fc (SEQ ID NO: 5): CD96-Fc, for following assays

MEFGLSWLFLVAILKGVQCVWEKTVNTEENVYATLGSDVNLTCQTQTVGFFVQM
QWSKVTNKIDLIAVYHPQYGFYCAYGRPCESLVTFTETPENGSKWTLHLRNMSCSVSG
RYECMLVLYPEGIQTKIYNLLIQTHVTADEWNSNHTIEIEINQTLEIPCFQNSSSKISSEFT
YAWSVEDNGTQETLISQNHLISNSTLLKDRVKLGTDYRLHLSPVQIFDDGRKFSCHIRV
GPNKILRSSTTVKVFAKPEIPVIVENNSTDVLVERRFTCLLKNVFPKANITWFIDGSFLH
DEKEGIYITNEERKGKDGFLELKSVLTRVHSNKPAQSDNLTIWCMALSPVPGNKVWNIS
SEKITFLLGSEISSTDPPLSVTESTLDTQPSPASSVSPARYPATSSVTLVDVSALRPNTTPQP
SNSSMTTRGFNYPWTSSGTDTKKSVSRIPSETYSSSPSGAGSTLHDNVFTSTARAFSEVP
TTANGSTKTNHVHITGIVVNKPKDGMEPKSSDKTHTCPPCPAPELLGGPSVFLFPPKPKD
TLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFSCSVMHEALH
NHYTQKSLSLSPGK

Note: The underlined part is the signal peptide and the italic part is the Fc.

### Embodiment 2. Purification of CD96 associated recombinant proteins, and purification of hybridoma antibodies and recombinant antibodies

### 1. Purification steps of CD96-Flag recombinant protein:

The sample was centrifuged at high speed to remove impurities and concentrate to an appropriate volume. The flag affinity column was equilibrated with 2-5 column volumes of 0.5×PBS. The cell expression supernatant sample from which impurities have been removed was loaded onto the column. The column was washed with 0.5×PBS until the readout at A280 was reduced to baseline. The column was washed with PBS containing 0.3M NaCl to remove the impurity proteins and efflux was collected. The target protein was eluted with 0.1M acetic acid (pH3-4) or Flag peptide solution and the elution peak of the target product was collected, and the pH was adjusted to neutral.

The collected elution was concentrated and may further purified by, for example gel chromatography Superdex 200 (GE) and the mobile phase was PBS, and the peaks of the multimer and heteroprotein were removed and the elution peak of the target product was collected. The obtained protein was identified as correct by electrophoresis, peptide mapping, and LC-MS, and aliquoted for later use.

### 2. Purification steps of His96-tagged CD96-His recombinant protein:

The samples of cell expression supernatant were centrifuged at high speed to remove impurities, the buffer was replaced with PBS, and imidazole was added to a final concentration of 5 mM. The nickel column was equilibrated with 2-5 column volumes of PBS containing 5 mM imidazole. After the replacement of PBS, the samples of supernatant were loaded onto the column for binding, and the medium may choose nickel columns from different companies. The column was washed with PBS containing 5 mM imidazole until the readout at A280 was reduced to baseline. The column was then washed with PBS+10 mM imidazole to remove non-specifically bound heteroproteins, and the effluent was collected. The target protein was eluted with PBS containing 300 mM imidazole, and the elution peak was collected.

The collected elution was concentrated and may further purified by, for example gel chromatography Superdex 200 (GE) and the mobile phase was PBS, and the peaks of the multimer and heteroprotein were removed, and the elution peak of the target product was collected. The obtained protein was identified as correct by electrophoresis, peptide mapping, and LC-MS, and aliquoted for later use.

### 3. Isolation and purification of hybridoma supernatants/ProteinG affinity chromatography: purification of recombinant antibodies and Fc fusion proteins

Protein G is preferred for purification of mouse hybridoma supernatants for affinity chromatography. The cultured hybridomas were centrifuged to collect the supernatant, and 10-15% by volume of 1M Tris-HCl (pH 8.0-8.5) was added to adjust the pH of supernatant. The Protein G column was washed with 3-5 column volumes of 6 M guanidine hydrochloride, and then 3-5 column volumes of pure water. The column was equilibrated with 3-5 column volumes of a buffer system such as 1×PBS (pH7.4). The cell supernatant was loaded with low flow rate for binding, and the flow rate was controlled to maintain a retention time of about 1 minute or longer. The chromatography column was washed with 3-5 column volumes of 1×PBS (pH7.4) until the UV absorption fell back to baseline. The sample was eluted with 0.1 M acetic acid/sodium acetate (pH 3.0) buffer, the elution peak was collected according to UV detection, and the eluted product was temporarily stored after the pH of which was rapidly adjusted to pH 5-6 using 1 M Tris-HCl (pH8.0). The eluted product can be subjected to solution replacement by methods well known to those skilled in the art, such as ultrafiltration concentration with ultrafiltration tube and solution replacement with a desired buffer system, or molecular exclusion such as G-25 desalting to replace with a desired buffer system, or high-resolution molecular exclusion columns such as Superdex 200 to remove multimer components from the eluted product to improve the purity of sample.

### 4. Protein A affinity chromatography to extract fusion proteins or antibodies with Fc-tag

First, the culture supernatant of cells expressing the Fc fusion protein or antibody was centrifuged at high speed to collect the supernatant. ProteinA affinity column was washed with 3-5 column volumes of 6M guanidine hydrochloride, and then 3-5 column volumes of pure water. The column was equilibrated with 3-5 column volumes of a buffer system such as 1×PBS (pH 7.4). The cell supernatant was loaded with a low flow rate for binding, and the flow rate was controlled to maintain a retention time of about 1 minute or longer. After the binding was completed, the chromatography column was washed with 3-5 column volumes of 1×PBS (pH7.4) until the UV absorption fell back to baseline. The sample was eluted with 0.1 M acetic acid/sodium acetate (pH3.0-3.5) buffer, and the eluted peak was collected according to UV detection. The eluted product was temporatily stored after the pH of which was rapidly adjusted to pH 5-6 using 1M Tris-HCl (pH 8.0). The eluted product can be subjected to solution replacement by methods well known to those skilled in the art, such as using ultrafiltration concentration with ultrafiltration tube and solution replacement with a desired buffer system, or molecular exclusion such as G-25 desalting to replace with a desired buffer system, or high-resolution molecular exclusion columns such as Superdex 200 to remove multimer components from the eluted product to improve the purity of sample.

### Embodiment 3. Preparation of Anti-human CD96 Hybridoma Monoclonal antibodies

### 1. Immunization

The anti-human CD96 monoclonal antibody was produced by immunizing mice. SJL white mice, female, 6-8 weeks old (Beijing Vital River Laboratory Animal Technology Co., Ltd., animal permit number: SCXK (Beijing) 2012-0001) were used in this experiment and raised in a SPF laboratory. After purchase, the mice were kept in the laboratory for 1 week under 12/12 hours light/dark cycle, at a temperature of 20-25°C, and humidity 40-60%. The mice that had been adapted to the environment were immunized according to the following protocol. Immunogens were human CD96 extracellular region (SEQ ID NO: 1 or 2) with Flag tag or mFc tag, and CHO cell line overexpressing human CD96.

Immunization protocol A: TiterMax® Gold Adjuvant (Sigma Cat No. T2684) and Thermo Imject® Alum (Thermo Cat No. 77161) adjuvant were used for cross-immunization. The ratio of antigen to adjuvant (TiterMax® Gold Adjuvant) was 1:1, the ratio of antigen to adjuvant (Thermo Imject® Alum) is 3: 1, 50 µg/mouse for the primary immunization, and 25 µg/mouse for the booster immunization. The antigen was emulsified and inoculated on day 0, 14, 28, 42, 56. On day 0, each mouse was intraperitoneally (IP) injected with 50 µg emulsified antigens. On day 14, each mouse was subcutaneously (SC) multiply injected (usually 6-8 points on the back) with 25 µg. On day 28 and day 42, the antigen was back or intraperitoneally injected according to the swelling conditions on the back and abdomen. Blood was sampled on day 21, 35, 49 and 63, and antibody titer in mouse serum were determined by ELISA. After 4-5 times of immunization, mice with high antibody titer in the serum and with the titer tending to be stationary were selected for splenocyte fusion. Immunization was boosted 3 days before splenocyte fusion, and each mouse was intraperitoneally (IP) with antigen solution prepared with physiological saline.

Immunization protocol B: Mice were immunized with Quick Antibody-Mouse 5W (KX0210041) adjuvant. The ratio of antigen to adjuvant was 1:1, 25 µg/mouse for both primary immunization and booster immunization. The antigen and adjuvant were quickly and thoroughly mixed, and then inoculated on day 0, 21 and 35. On day 0, the hind leg of each mouse was intramuscularly (IM) injected with 25 µg antigen. On the day 21 and 35, each mouse was injected with 25 µg in the same manner (depending on the titer, whether or not the third immunization was performed). Blood was sampled on day 28 and 42 and the antibody titer in mouse serum was determined by ELISA. Mice with high antibody titers in the serum and with the titer tending to be stationary were selected for splenocyte fusion. Immunization was boosted 3 days before splenocyte fusion, and each mouse was intraperitoneally (IP) with antigen solution prepared with physiological saline.

### 2. Spleen cell fusion

The spleen lymphocytes and myeloma cells Sp2/0 (ATCC® CRL-8287™) were fused to obtain hybridoma cells by optimized PEG-mediated fusion procedure. The fused hybridoma cells were re-suspended in complete medium (DMEM medium containing 20% FBS, 1×HAT, 1×OPI) at a density of 0.5-1 × 10^6/ml, then aliquoted into a 96-well cell culture plate (1 × 10⁵/150µ/well). After incubation at 37°C and 5% CO₂ for 3-4 days, 100 µl/well of HAT complete medium was supplemented. The culture was continued for 3-4 days until needle-like clones were formed. After removing supernatant, 200 µl/well of HT complete medium (RPMI-1640 medium containing 20% FBS, 1×HT and 1×OPI) was added, incubating at 37 °C, 5% CO₂ for 3 days, and then ELISA test was performed.

### 3. Screening of hybridoma cells

Based on the hybridoma cell growth density, ELISA tests for the hybridoma culture supernatant was performed. Cell-binding experiments and cell-blocking experiments were performed with supernatant of positive cells tested by ELISA. The wells that were positive for both binding and blocking were expanded in time for cryopreservation and subcloning was performed two to three times until a single cell clone was obtained.

CD96-binding ELISA, cell-binding assay and cell-blocking assay were also performed for each subcloned cell. Hybridoma clones were screened through the above experiments, and antibodies were further prepared by serum-free cell culture. The antibodies were purified according to the purification embodiments (item 3 of Embodiment 2) for use in the test examples.

### 4. Sequencing of positive hybridoma clones

The process of cloning sequences from positive hybridomas are as follows. The logarithmic growth phase hybridoma cells were collected, RNA was extracted using Trizol (Invitrogen, Cat No. 15596-018) according to instructions of the Kit, and reverse transcription was performed using PrimeScript™ Reverse Transcriptase Kit (Takara, Cat No. 2680A). The reverse-transcribed cDNA was amplified by PCR using a mouse Ig-Primer Kit (Novagen, TB326 Rev.B 0503) and sent to a sequencing company for sequencing. The amino acid sequences of the heavy and light chain variable region DNA sequences corresponding to the murine antibodies m1718, m1719, m1720, m1721 and m1722 were obtained (the amino acid residues of the CDRs in VH/VL are numbered and annotated according to the Kabat numbering system, and the underlined sequence is CDR):

m1718 VH（SEQ ID NO：6）
EFQLQQSGPELVKPGASVKISCKASAYSITDYNMNWVKQSNGKSLEWIGVINPSY
GITDYNQNFKDKATLTVDQSSSTAYMQLNSLTSEDSAVYYCAIQLRLPGYFDVWGTG
TTVIVSS

m1718VL（SEQ ID NO：7）
DIKMTQSPSSMYASLGERVTITCKASQDINNYLNWFQQKPGKSPKTLIYRANRLV
DGVPSRFSGSGSGQDYSLTISSLEYEDMGIYYCLKYDEFPYTFGGGTKVEIK

m1719VH（SEQ ID NO：8）
QVQLKESGPGLVAPSQSLSITCTVSGFSLISYGVSWVRQPPGKGLEWLGVIWGDG
NTNYHSVLISRLSIRKDDSKSQVFLKLNSLQTDDTATYYCAKNNYYGSRYGYPMDYW
GQGTSVTVSS

m1719VL（SEQ ID NO：9）
DIVMTQSHKFMSTSVGDRVSITCKATQDVGTAVAWYQQKPGHSPKLLIYWASTR
HTGVPERFTGSGSGTDFTLTIDNVQSEDLADYFCQQYGSSVLTFGAGTKVELR

m1720VH（SEQ ID NO：10）
EFQLQQSGPELVKPGASVKISCKASAYSLTDYNMNWVKQSNGKSLEWIGVINPSY
GISSYNQKFKGKATLTVDQSSSTAYMHLNSLTSEDSAVYYCARQLRLPGYFDVWGTG
TTVTVSS

m1720VL（SEQ ID NO：11）
DIKMTQSPSSKNASLGERVTITCKASQDINSYLNWFQQKPGKSPKTLIYRASRLVD
GVPSRFSGSGSGQDYSLTISSLEYEDMGIYYCLKYDEFPYTFGGGTKLEIK

m1721VH（SEQ ID NO：12）

QVQLKESGPGLVAPSQSLSITCTVSGFSLISYGVNWVRQPPGKGLEWLGVIWGDG
STNYHSALISRLSISKDDSKSQVFLNLNSLQTDDTATYYCAKNYFYGSRYGYAMDSW
GQGISVTVSS

m1721VL（SEQ ID NO：13）
DIQMTQSPASLSAAVGETVTITCRASENIYSSLAWYQQKQGKSPQLLVYNAKTLIE
TVASRFSGSGSGTQYSLKINSLQPEDFGSFYCQHHYGTPYTFGGGTKLEIK

m1722VH（SEQ ID NO：14）
QVQLQQPGAELVKPGTSVKLSCKASGNTFIDYWMHWVKQRPGQGLEWIGMLHP
NSGTTSFNEKFKIKTTLTIDKSSSTAYMQLSSLTSEDSAIYYCASDYSGPFAYWGQGTL
VTVSA

m1722VL（SEQ ID NO：15）

QIVLTQSPGIMSAFPGEKVTMTCSASSSVNYIHWYQQKSGTSPKRWIFDTSKLASG

VPARFSGSGSGTSYSLTISSMEAEDAATYYCQQWRSNPLTFGAGTKLELK

The CDR sequences in the light chains and heavy chains of each antibody are shown in Table 1.

**Table 1 CDR sequence of heavy chains and light chains of each antibody**

| Antibody | CDR of heavy chain | | CDR of light chain | |
|---|---|---|---|---|
| m1718 | HCDR1 | DYNMN SEQ ID NO: 16 | LCDR1 | KASQDINNYLN SEQ ID NO: 19 |
| | HCDR2 | VINPSYGITDYNQNFKD SEQ ID NO: 17 | LCDR2 | RANRLVD SEQ ID NO: 20 |
| | HCDR3 | QLRLPGYFDV SEQ ID NO: 18 | LCDR3 | LKYDEFPYT SEQ ID NO: 21 |
| m1719 | HCDR1 | SYGVS SEQ ID NO: 22 | LCDR1 | KATQDVGTAVA SEQ ID NO: 25 |
| | HCDR2 | VIWGDGNTNYHSVLIS SEQ ID NO: 23 | LCDR2 | WASTRHT SEQ ID NO: 26 |
| | HCDR3 | NNYYGSRYGYPMDY SEQ ID NO: 24 | LCDR3 | QQYGSSVLT SEQ ID NO: 27 |
| m1720 | HCDR1 | DYNMN SEQ ID NO: 28 | LCDR1 | KASQDINSYLN SEQ ID NO: 31 |
| | HCDR2 | VINPSYGISSYNQKFKG SEQ ID NO: 29 | LCDR2 | RASRLVD SEQ ID NO: 32 |
| | HCDR3 | QLRLPGYFDV | LCDR3 | LKYDEFPYT |
| | | SEQ ID NO: 30 | | SEQ ID NO: 33 |
| m1721 | HCDR1 | SYGVN SEQ ID NO: 34 | LCDR1 | RASENIYSSLA SEQ ID NO: 37 |
| | HCDR2 | VIWGDGSTNYHSALIS SEQ ID NO: 35 | LCDR2 | NAKTLIE SEQ ID NO: 38 |
| | HCDR3 | NYFYGSRYGYAMDS SEQ ID NO: 36 | LCDR3 | QHHYGTPYT SEQ ID NO: 39 |
| m1722 | HCDR1 | DYWMH SEQ ID NO: 40 | LCDR1 | SASSSVNYIH SEQ ID NO: 43 |
| | HCDR2 | MLHPNSGTTSFNEKFKI SEQ ID NO: 41 | LCDR2 | DTSKLAS SEQ ID NO: 44 |
| | HCDR3 | DYSGPFAY SEQ ID NO: 42 | LCDR3 | QQWRSNPLT SEQ ID NO: 45 |

The light chain and heavy chain variable regions of the murine antibody were linked to the light chain constant region (SEQ ID NO: 117) and the heavy chain constant region (SEQ ID NO: 118) of the human antibody, respectively, to form a chimeric antibody. The chimeric antibody corresponding to m1718 antibody was named ch1718, and other antibodies were named by analogy.

### Embodiment 4. Humanization of murine anti-CD96 antibody

By aligning germline gene database of IMGT human antibody heavy chain and light chain variable region and MOE software, the heavy chain and light chain variable region germline genes with high homology with murine antibodies were selected as templates, respectively. The CDRs of murine antibodies were respectively grafted into the corresponding human templates to form variable region sequences with the order of FR1-CDR1-FR2-CDR2-FR3-CDR3-FR4. According to the experimental requirements, the key amino acids in the backbone sequence were back-mutated into the amino acid corresponding to the murine antibody to ensure the original affinity, and the humanized anti-CD96 monoclonal antibody was obtained. The amino acid residues were identified and annotated according to the natural numbers when perfoming back mutations.

### 4.1 Humanization of hybridoma clone m1718

(1) Selection of humanized framework for m1718
For mouse antibody m1718, the template of humanized light chain is IGKV1-16^{∗}01 and hjk4.1, and the template of humanized heavy chain is IGHV1-3^{∗}01 and hjh6.1. After CDR grafting, humanized antibody h1718-001 was obtained and its sequences of humanized variable region are as follows:
h1718-001 VH-CDR graft 
EVQLVQSGAEVKKPGASVKVSCKASGYTFTDYNMNWVRQAPGQRLEWMGVINPSYG
ITDYNQNFKDRVTITRDTSASTAYMELSSLRSEDTAVYYCARQLRLPGYFDVWGQGTTVTVS
S
SEQ ID NO: 46
h1718-001 VL-CDR graft

DIQMTQSPSSLSASVGDRVTITCKASQDINNYLNWFQQKPGKAPKSLIYRANRLVDG
VPSRFSGSGSGTDFTLTISSLQPEDFATYYCLKYDEFPYTFGGGTKVEIK
SEQ ID NO: 47

Note: The order of various regions is FR1-CDR1-FR2-CDR2-FR3-CDR3-FR4, the italic part is FR sequence, and underlined part is CDR sequence.
(2) The design of h1718 series back mutations are as follows:

**Table 2. Back mutations of h1718 antibody**

| h1718-**VL** | | h1718-**VH** | |
|---|---|---|---|
| h1718-VL1 | **Grafted** | h1718-VH1 | **Grafted** |
| h1718-VL2 | S46T, T69Q, F71Y | h1718-VH2 | M48I, R72V |
| | | h1718-VH3 | F29I, M48I, R72V, R98I |
| | | h1718-VH4 | F29I, R38K, M48I, R67K, R72V, R98I |

Note: For example, S46T is numbered according to the natural sequence of the amino acid sequence, 'S' at position 46 is mutated back to 'T'. 'Grafted' represents that the murine antibody CDR sequence is inserted into the human germline FR region.
(3) The combinations of variable region sequences of h1718 series humanized antibodies are as follows:

**Table 3. combinations of variable region sequences of h1718 series humanized antibodies**

| | h1718-VH1 | h1718-VH2 | h1718-VH3 | h1718-VH4 |
|---|---|---|---|---|
| M1718-VL1 | h1718-001 | h1718-002 | h1718-003 | h1718-004 |
| h1718-VL2 | h1718-005 | h1718-006 | h1718-007 | h1718-008 |

Note: This table shows the sequences obtained from various combinations of mutations. As shown by h1718-006, the humanized antibody h1718-006 contains a light chain variable region h1718-VL2 and a heavy chain variable region h1718-VH2, and so on.
(4) The specific sequences of the variable regions of h1718 series humanized antibodies are as follows:
>h1718-VL1 (The same as H1718-001VL-CDR graft) 
DIQMTQSPSSLSASVGDRVTITCKASQDINNYLNWFQQKPGKAPKSLIYRANRLVDGVP
SRFSGSGSGTDFTLTISSLQPEDFATYYCLKYDEFPYTFGGGTKVEIK
SEQ ID NO: 47
>h1718-VL2 
DIQMTQSPSSLSASVGDRVTITCKASQDINNYLNWFQQKPGKAPKTLIYRANRLVDGVP
SRFSGSGSGQDYTLTISSLQPEDFATYYCLKYDEFPYTFGGGTKVEIK
SEQ ID NO: 48
> h1718-VH1 (The same as H1718-001VH-CDR graft) 
EVQLVQSGAEVKKPGASVKVSCKASGYTFTDYNMNWVRQAPGQRLEWMGVINPSYGI
TDYNQNFKDRVTITRDTSASTAYMELSSLRSEDTAVYYCARQLRLPGYFDVWGQGTTVTVSS
SEQ ID NO: 46
>h1718-VH2 
EVQLVQSGAEVKKPGASVKVSCKASGYTFTDYNMNWVRQAPGQRLEWIGVINPSYGIT
DYNQNFKDRVTITVDTSASTAYMELSSLRSEDTAVYYCARQLRLPGYFDVWGQGTTVTVSS
SEQ ID NO: 49
>h1718-VH3 
EVQLVQSGAEVKKPGASVKVSCKASGYTITDYNMNWVRQAPGQRLEWIGVINPSYGIT
DYNQNFKDRVTITVDTSASTAYMELSSLRSEDTAVYYCAIQLRLPGYFDVWGQGTTVTVSS
SEQ ID NO: 50
>h1718-VH4

EVQLVQSGAEVKKPGASVKVSCKASGYTITDYNMNWVKQAPGQRLEWIGVINPSYGIT
DYNQNFKDKVTITVDTSASTAYMELSSLRSEDTAVYYCAIQLRLPGYFDVWGQGTTVTVSS

SEQ ID NO：51

(5) Hotspot mutation of h1718-VL:
The NN in CDR1 of h1718 series humanized VL (ie, SEQ ID NO: 19, KASQDINNYLN) was mutated to NQ, NT, NL to improve the stability of the antibodies. The general formula of h1718 VL CDR1 sequence is KASQDINX₁YLN (SEQ ID NO: 52), wherein X₁ is selected from: N, T, L and Q. Specific h1718VL CDR1 mutants include KASQDINQYLN (SEQ ID NO: 108), KASQDINTYLN (SEQ ID NO: 109) and KASQDINLYLN (SEQ ID NO: 110).
h1718-VL1 can be mutated to the following light chain variable region sequences: 
>h1718. VL1a（SEQ ID NO：53）
DIQMTQSPSSLSASVGDRVTITCKASQDINQYLNWFQQKPGKAPKSLIYRANRLV
DGVPSRFSGSGSGTDFTLTISSLQPEDFATYYCLKYDEFPYTFGGGTKVEIK
 
>h1718. VL1b（SEQ ID NO：54）
DIQMTQSPSSLSASVGDRVTITCKASQDINTYLNWFQQKPGKAPKSLIYRANRLVD
GVPSRFSGSGSGTDFTLTISSLQPEDFATYYCLKYDEFPYTFGGGTKVEIK
 
>h1718. VL1c（SEQ ID NO：55）
DIQMTQSPSSLSASVGDRVTITCKASQDINLYLNWFQQKPGKAPKSLIYRANRLVD
GVPSRFSGSGSGTDFTLTISSLQPEDFATYYCLKYDEFPYTFGGGTKVEIK
h1718-VL2 can be mutated to the following light chain variable region sequences:

>h1718. VL2a（SEQ ID NO：56）
DIQMTQSPSSLSASVGDRVTITCKASQDINQYLNWFQQKPGKAPKTLIYRANRLV
DGVPSRFSGSGSGQDYTLTISSLQPEDFATYYCLKYDEFPYTFGGGTKVEIK

>h1718. VL2b（SEQ ID NO：57）
DIQMTQSPSSLSASVGDRVTITCKASQDINTYLNWFQQKPGKAPKTLIYRANRLVD
GVPSRFSGSGSGQDYTLTISSLQPEDFATYYCLKYDEFPYTFGGGTKVEIK

>h1718. VL2c（SEQ ID NO：58）
DIQMTQSPSSLSASVGDRVTITCKASQDINLYLNWFQQKPGKAPKTLIYRANRLVD
GVPSRFSGSGSGQDYTLTISSLQPEDFATYYCLKYDEFPYTFGGGTKVEIK

The combinations of the variable region sequences of h1718 series humanized antibodies are as follows:

**Table 4. Combinations of variable region sequences of h1718 series humanized antibodies**

| | | | | |
|---|---|---|---|---|
| | h1718-VH3 | h1718-VH4 | h1718-VH1 | h1718-VH2 |
| h1718-VL1a | h1718-009 | h1718-015 | h1718-021 | h1718-027 |
| h1718-VL1b | h1718-010 | h1718-016 | h1718-022 | h1718-028 |
| h1718-VL1c | h1718-011 | h1718-017 | h1718-023 | h1718-029 |
| h1718-VL2a | h1718-012 | h1718-018 | h1718-024 | h1718-030 |
| h1718-VL2b | h1718-013 | h1718-019 | h1718-025 | h1718-031 |
| h1718-VL2c | h1718-014 | h1718-020 | h1718-026 | h1718-032 |

### 4.2 Humanization of hybridoma clone m1719

(1) Selection of humanized framework for m1719
For murine antibody m1719, the template of humanized light chain is IGKV1-12^{∗}01 and hjk4.1, and the template of humanized heavy chain is IGHV2-26^{∗}01 and hjh6.1. After CDR crafting, humanized antibody h1719-001 was obtained and its sequences of humanized variable region are as follows:
h1719-001 VH-CDR graft 
EVTLKESGPVLVKPTETLTLTCTVSGFSLSSYGVSWIRQPPGKALEWLAVIWGDGNTNY
HSVLISRLTISKDTSKSQVVLTMTNMDPVDTATYYCARNNYYGSRYGYPMDYWGQGTTVTVS
S
SEQ ID NO: 59
h1719-001 VL-CDR graft

DIQMTQSPSSVSASVGDRVTITCKATQDVGTAVAWYQQKPGKAPKLLIYWASTRHTGVP
SRFSGSGSGTDFTLTISSLQPEDFATYYCQQYGSSVLTFGGGTKVEIK
SEQ ID NO: 60

Note: The order of various regions is FR1-CDR1-FR2-CDR2-FR3-CDR3-FR4, the italic part is FR sequence, and underlined part is CDR sequence.
(2) The design of h1719-001 back mutations are as follows:

**Table 5. Back mutations of h1719 antibody**

| h1719**-VL** | | h1719-**VH** | |
|---|---|---|---|
| h1719-VL1 | Grafted | h1719-VH1 | Grafted |
| h1719-VL2 | A43S, S60E, Y87F | h1719-VH2 | I37V, R97K |
| | | h1719-VH3 | I37V, A44G, A49G, M82L, R97K |

Note: For example, A43S is numbered according to the natural sequence of the amino acid sequence, A at position 43 is mutated back to S. 'Grafted' represents that the murine antibody CDR sequence is inserted into the human germline FR region.
(3) The combinations of variable region sequences of h1719 series humanized antibodies are as follows:

**Table 6. Combinations of variable region sequences of h1719 series humanized antibodies**

| | h1719-VH1 | h1719-VH2 | h1719-VH3 |
|---|---|---|---|
| h1719-VL1 | h1719-001 | h1719-002 | h1719-003 |
| h1719-VL2 | h1719-004 | h1719-005 | h1719-006 |

Note: This table shows the sequences obtained from various combinations of mutations. As shown by h1719-005, the humanized antibody h1719-005 contains a light chain variable region h1719-VL2 and a heavy chain variable region h1719-VH2, and so on.
(4) The specific sequences of the variable regions of h1719 series humanized antibodies are as follows:
>h1719-VL1 (The same as h1719-001 VL-CDR graft) 
DIQMTQSPSSVSASVGDRVTITCKATQDVGTAVAWYQQKPGKAPKLLIYWASTRHTGVP
SRFSGSGSGTDFTLTISSLQPEDFATYYCQQYGSSVLTFGGGTKVEIK
SEQ ID NO: 60
>h1719-VL2 
DIQMTQSPSSVSASVGDRVTITCKATQDVGTAVAWYQQKPGKSPKLLIYWASTRHTGVP
ERFSGSGSGTDFTLTISSLQPEDFATYFCQQYGSSVLTFGGGTKVEIK
SEQ ID NO：61
 >h 1719-VH1 (The same as h1719-001 VH-CDR graft) 
EVTLKESGPVLVKPTETLTLTCTVSGFSLSSYGVSWIRQPPGKALEWLAVIWGDGNTNY
HSVLISRLTISKDTSKSQVVLTMTNMDPVDTATYYCARNNYYGSRYGYPMDYWGQGTTVTVS
 
S
SEQ ID NO：59
 >h1719-VH2 
EVTLKESGPVLVKPTETLTLTCTVSGFSLSSYGVSWVRQPPGKALEWLAVIWGDGNTNY
HSVLISRLTISKDTSKSQVVLTMTNMDPVDTATYYCAKNNYYGSRYGYPMDYWGQGTTVTVS
S
SEQ ID NO: 62
 >h1719-VH3

EVTLKESGPVLVKPTETLTLTCTVSGFSLSSYGVSWVRQPPGKGLEWLGVIWGDGNTN
YHSVLISRLTISKDTSKSQVVLTLTNMDPVDTATYYCAKNNYYGSRYGYPMDYWGQGTTVTV
SS
SEQ ID NO: 63

(5) Hotspot mutation of h1719-VH:
The NN in CDR3 of h1719 VH (ie, SEQ ID NO: 19, KASQDINNYLN) was mutated to QN, NY, NQ, and M was mutated to L or I to improve the stability of the antibodies. The general formula of h1719VH CDR3 sequence is X₂X₃YYGSRYGYPX₄DY (SEQ ID NO: 64), wherein X₂ is selected from: N and Q, X₃ is selected from: N, Y and Q, X₄ is selected from: M, L and I. Specific h1719 VH CDR3 mutants may include, but are not limited to QNYYGSRYGYPLDY (SEQ ID NO: 111), NYYYGSRYGYPLDY (SEQ ID NO: 112), NQYYGSRYGYPLDY (SEQ ID NO: 113), QNYYGSRYGYPIDY (SEQ ID NO: 114), NYYYGSRYGYPIDY (SEQ ID NO: 115), NQYYGSRYGYPIDY (SEQ ID NO: 116).
h1719-VH3 can be mutated to the following heavy chain variable region sequences: 
>h1719.VH3a（SEQ ID NO：65）
EVTLKESGPVLVKPTETLTLTCTVSGFSLSSYGVSWVRQPPGKGLEWLGVIWGDG
NTNYHSVLISRLTISKDTSKSQVVLTLTNMDPVDTATYYCAKQNYYGSRYGYPLDYWG
QGTTVTVSS
 
>h1719. VH3b（SEQ ID NO：66）
EVTLKESGPVLVKPTETLTLTCTVSGFSLSSYGVSWVRQPPGKGLEWLGVIWGDG
NTNYHSVLISRLTISKDTSKSQVVLTLTNMDPVDTATYYCAKNYYYGSRYGYPLDYWG
 QGTTVTVSS 
>h1719. VH3c（SEQ ID NO：67）
EVTLKESGPVLVKPTETLTLTCTVSGFSLSSYGVSWVRQPPGKGLEWLGVIWGDG
NTNYHSVLISRLTISKDTSKSQVVLTLTNMDPVDTATYYCAKNQYYGSRYGYPLDYWG
QGTTVTVSS
 
>h1719. VH3d（SEQ ID NO：68）
EVTLKESGPVLVKPTETLTLTCTVSGFSLSSYGVSWVRQPPGKGLEWLGVIWGDG
NTNYHSVLISRLTISKDTSKSQVVLTLTNMDPVDTATYYCAKQNYYGSRYGYPIDYWG
QGTTVTVSS
 
>h1719. VH3e（SEQ ID NO：69）
EVTLKESGPVLVKPTETLTLTCTVSGFSLSSYGVSWVRQPPGKGLEWLGVIWGDG
NTNYHSVLISRLTISKDTSKSQVVLTLTNMDPVDTATYYCAKNYYYGSRYGYPIDYWG
QGTTVTVSS
 
>h1719. VH3f（SEQ ID NO：70）
EVTLKESGPVLVKPTETLTLTCTVSGFSLSSYGVSWVRQPPGKGLEWLGVIWGDG
NTNYHSVLISRLTISKDTSKSQVVLTLTNMDPVDTATYYCAKNQYYGSRYGYPIDYWG
QGTTVTVSS
The combinations of variable region sequences of h1719 series humanized antibodies are as follows:

**Table 7. Combinations of variable region sequences of h1719 series humanized antibodies**

| | h1719-VH3a | h1719-VH3b | h1719-VH3c | h1719-VH3d | h1719-VH3e | h1719-VH3f |
|---|---|---|---|---|---|---|
| h1719-VL1 | h1719-007 | h1719-008 | h1719-009 | h1719-010 | h1719-011 | h1719-012 |
| h1719-VL2 | h1719-013 | h1719-014 | h1719-015 | h1719-016 | h1719-017 | h1719-018 |

### 4.3 Humanization of hybridoma clone m1721

(1) Selection of humanized framework for m1721
For mouse antibody m1721, the template of humanized light chain is IGKV1-39^{∗}01 and hjk4.1, and the template of humanized heavy chain is IGHV2-26^{∗}01 and hjh6.1. After CDR crafting, humanized antibody h1721-001 was obtained and its sequences of humanized variable region are as follows:
h1721-001 VH-CDR graft 
EVTLKESGPVLVKPTETLTLTCTVSGFSLSSYGVNWIRQPPGKALEWLAVIWGDGSTNY
HSALISRLTISKDTSKSQVVLTMTNMDPVDTATYYCARNYFYGSRYGYAMDSWGQGTTVTVS
S
SEQ ID NO: 71
h1721-001 VL-CDR graft

DIQMTQSPSSLSASVGDRVTITCRASENIYSSLAWYQQKPGKAPKLLIYNAKTLIEGVPSR
FSGSGSGTDFTLTISSLQPEDFATYYCQHHYGTPYTFGGGTKVEIK
SEQ ID NO: 72

Note: The order of various regions is FR1-CDR1-FR2-CDR2-FR3-CDR3-FR4, the italic part is FR sequence, and underlined part is CDR sequence.
(2) The design of h1721-001 back mutations are as follows:

**Table 8. Back mutations of h1721 antibody**

| h1721-**VL** | | h1721-**VH** | |
|---|---|---|---|
| VL1 | Grafted | VH1 | Grafted |
| VL2 | A48S, F71Y | VH2 | A49G, R97K |
| VL3 | A43S, I48V, F71Y | VH3 | A49G, M82L, R97K |
| | | VH4 | I37V, A44G, A49G, M82L, R97K |

Note: For example, A48S is numbered according to the natural sequence of the amino acid sequence, A at position 48 is mutated back to S. 'Grafted' represents that the human antibody CDR sequence is inserted into the human germline FR region.
(3) The combinations of variable region sequences of h1721 series humanized antibodies are as follows:

**Table 9. combinations of variable region sequences of h1721 series humanized antibodies**

| | h1721-VH1 | hl721-VH2 | h1721-VH3 | h1721-VH4 |
|---|---|---|---|---|
| h1721-VL1 | h1721-001 | h1721-002 | h1721-003 | h1721-004 |
| h1721-VL2 | h1721-005 | h1721-006 | h1721-007 | h1721-008 |
| h1721-VL3 | h1721-009 | h1721-010 | h1721-011 | h1721-0012 |

Note: This table shows the sequences obtained from various combinations of mutations. As shown by h1721-006, the humanized antibody h1721-006 contains a light chain variable region h1721-VL2 and a heavy chain variable region h1721-VH2, and so on.
(4) The specific sequences of the variable regions of h1721 series humanized antibodies are as follows:
>h1721V-L1 (The same as h1721-001 VL-CDR graft) 
DIQMTQSPSSLSASVGDRVTITCRASENIYSSLAWYQQKPGKAPKLLIYNAKTLIEGVPSR
FSGSGSGTDFTLTISSLQPEDFATYYCQHHYGTPYTFGGGTKVEIK
SEQ ID NO: 72
>h1721-VL2 
DIQMTQSPSSLSASVGDRVTITCRASENIYSSLAWYQQKPGKAPKLLVYNAKTLIEGVPS
RFSGSGSGTDYTLTISSLQPEDFATYYCQHHYGTPYTFGGGTKVEIK
SEQ ID NO: 73
>h1721-VL3 
DIQMTQSPSSLSASVGDRVTITCRASENIYSSLAWYQQKPGKSPKLLVYNAKTLIEGVPSR
FSGSGSGTDYTLTISSLQPEDFATYYCQHHYGTPYTFGGGTKVEIK
SEQ ID NO: 74
> h1721-VH1 (The same as h1721-001 VH-CDR graft) 
EVTLKESGPVLVKPTETLTLTCTVSGFSLSSYGVNWIRQPPGKALEWLAVIWGDGSTNY
HSALISRLTISKDTSKSQVVLTMTNMDPVDTATYYCARNYFYGSRYGYAMDSWGQGTTVTVS
S
SEQ ID NO: 71
>h1721-VH2 
EVTLKESGPVLVKPTETLTLTCTVSGFSLSSYGVNWIRQPPGKALEWLGVIWGDGSTNY
HSALISRLTISKDTSKSQVVLTMTNMDPVDTATYYCAKNYFYGSRYGYAMDSWGQGTTVTVS
S
SEQ ID NO: 75
>h1721-VH3 
EVTLKESGPVLVKPTETLTLTCTVSGFSLSSYGVNWIRQPPGKALEWLGVIWGDGSTNY
HSALISRLTISKDTSKSQVVLTLTNMDPVDTATYYCAKNYFYGSRYGYAMDSWGQGTTVTVSS
SEQ ID NO：76
>h1721-VH4

EVTLKESGPVLVKPTETLTLTCTVSGFSLSSYGVNWVRQPPGKGLEWLGVIWGDGSTN
YHSALISRLTISKDTSKSQVVLTLTNMDPVDTATYYCAKNYFYGSRYGYAMDSWGQGTTVTVS
S
SEQ ID NO：77

### 4.4 Humanization of hybridoma clone m1722

(1) Selection of humanized framework for m1722
For murine antibody m1722, the template of humanized light chain is IGKV1-39^{∗}01 and hjk2.1, and the template of humanized heavy chain is IGHV1-69^{∗}02 and hjh4.1. After CDR crafting, humanized antibody h1722-001 was obtained and its sequences of humanized variable region are as follows:
h1722-001 VH-CDR graft 
EVQLVQSGAEVKKPGSSVKVSCKASGGTFSDYWMHWVRQAPGQGLEWMGMLHPNS
GTTSFNEKFKIRVTITADKSTSTAYMELSSLRSEDTAVYYCARDYSGPFAYWGQGTLVTVSS
SEQ ID NO: 78
h1722-001 VL-CDR graft

DIQMTQSPSSLSASVGDRVTITCSASSSVNYIHWYQQKPGKAPKLLIYDTSKLASGVPSRF
SGSGSGTDFTLTISSLQPEDFATYYCQQWRSNPLTFGQGTKLEIK
SEQ ID NO: 79

Note: The order of various regions is FR1-CDR1-FR2-CDR2-FR3-CDR3-FR4, the italic part is FR sequence, and underlined part is CDR sequence.
(2) The design of h1722-001 back mutations are as follows:

**Table 10. Back mutations of h1722 antibody**

| h1722-**VL** | | hl722-**VH** | |
|---|---|---|---|
| h1722-VL1 | Grafted | M722-VH1 | Grafted |
| h1722-VL2 | L45R, L46W | h1722-VH2 | M48I, R98S |
| h1722-VL3 | M4L, L45R, L46W, Y48F, F70Y | h1722-VH3 | R38K, M48I, A72I, R98S |
| | | h1722-VH4 | G27N, R38K, M48I, V68T, A72I, R98S |

Note: For example, L45R is numbered according to the natural sequence of the amino acid sequence, L at position 45 is mutated back to R. 'Grafted' represents that the murine antibody CDR sequence is inserted into the human germline FR region.
(3) The combinations of variable region sequences of h1722 series humanized antibodies are as follows:

**Table 11. combinations of variable region sequences of h1722 series humanized antibodies**

| | h1722-VH1 | h1722-VH2 | h1722-VH3 | h1722-VH4 |
|---|---|---|---|---|
| h1722-VL1 | h1722-001 | h1722-002 | h1722-003 | h1722-004 |
| h1722-VL2 | h1722-005 | h1722-006 | h1722-007 | h1722-008 |
| h1722-VL3 | h1722-009 | h1722-010 | h1722-011 | h1722-012 |

Note: This table shows the sequences obtained from various combinations of mutations. As shown by h1722-006, the humanized antibody h1722-006 contains a light chain variable region h1722-VL2 and a heavy chain variable region h1722-VH2, and so on.
(4) The specific sequences of the variable regions of h1722 series humanized antibodies are as follows:
>h1722-VL1 (The same as h1722-001 VL-CDR graft) 
DIQMTQSPSSLSASVGDRVTITCSASSSVNYIHWYQQKPGKAPKLLIYDTSKLASGVPSRF
SGSGSGTDFTLTISSLQPEDFATYYCQQWRSNPLTFGQGTKLEIK
SEQ ID NO: 79
>h1722-VL2 
DIQMTQSPSSLSASVGDRVTITCSASSSVNYIHWYQQKPGKAPKRWIYDTSKLASGVPSR
FSGSGSGTDFTLTISSLQPEDFATYYCQQWRSNPLTFGQGTKLEIK
SEQ ID NO：80
>h1722-VL3 
DIQLTQSPSSLSASVGDRVTITCSASSSVNYIHWYQQKPGKAPKRWIFDTSKLASGVPSR
FSGSGSGTDYTLTISSLQPEDFATYYCQQWRSNPLTFGQGTKLEIK
SEQ ID NO: 81
> h1722-VH1 (The same as h1722-001 VH-CDR graft) EVQLVQSGAEVKKPGSSVKVSCKASGGTFSDYWMHWVRQAPGQGLEWMGMLHPNS 
GTTSFNEKFKIRVTITADKSTSTAYMELSSLRSEDTAVYYCARDYSGPFAYWGQGTLVTVSS
SEQ ID NO: 78
>h1722-VH2 
EVQLVQSGAEVKKPGSSVKVSCKASGGTFSDYWMHWVRQAPGQGLEWIGMLHPNSG
TTSFNEKFKIRVTITADKSTSTAYMELSSLRSEDTAVYYCASDYSGPFAYWGQGTLVTVSS
SEQ ID NO: 82
>h1722-VH3 
EVQLVQSGAEVKKPGSSVKVSCKASGGTFSDYWMHWVKQAPGQGLEWIGMLHPNSG
TTSFNEKFKIRVTITIDKSTSTAYMELSSLRSEDTAVYYCASDYSGPFAYWGQGTLVTVSS
SEQ ID NO: 83
>h1722-VH4

EVQLVQSGAEVKKPGSSVKVSCKASGNTFSDYWMHWVKQAPGQGLEWIGMLHPNSG
TTSFNEKFKIRTTITIDKSTSTAYMELSSLRSEDTAVYYCASDYSGPFAYWGQGTLVTVSS
SEQ ID NO: 84

(5) hotspot mutation of h1722-VH:
Because there is an acetylated high-risk site NS, we mutated NS to KS or QS to improve the chemical stability of the antibody. The general formula of h1722-VH CDR2 sequence is MLHPX₅SGTTSFNEKFKI (SEQ ID NO: 119), wherein X₅ is selected from: N, K and Q. Specific h1722 VH CDR2 mutants may include, but are not limited to MLHPKSGTTSFNEKFKI (SEQ ID NO: 120) or MLHPQSGTTSFNEKFKI (SEQ ID NO: 121).
h1722-VH2 can be mutated to the following heavy chain variable region sequences:
> h1722-VH2a (N54K) 
EVQLVQSGAEVKKPGSSVKVSCKASGGTFSDYWMHWVRQAPGQGLEWIGMLHPKSG
TTSFNEKFKIRVTITADKSTSTAYMELSSLRSEDTAVYYCASDYSGPFAYWGQGTLVTVSS
SEQ ID NO: 122
> h1722-VH2b (N54Q)

EVQLVQSGAEVKKPGSSVKVSCKASGGTFSDYWMHWVRQAPGQGLEWIGMLHPQSG
TTSFNEKFKIRVTITADKSTSTAYMELSSLRSEDTAVYYCASDYSGPFAYWGQGTLVTVSS

SEQ ID NO：123

The combinations of variable region sequences of h1722 series humanized antibodies are as follows:

**Table 12. Combinations of variable region sequences of h1722 series humanized antibodies**

| | h1722-VH2a | h1722-VH2b |
|---|---|---|
| h1722-VL1 | h1722-013 | h1722-014 |
| h1722-VL2 | h1722-015 | h1722-016 |
| h1722-VL3 | h1722-017 | h1722-018 |

The heavy chain variable regions described above can be recombinantly expressed with the heavy chain constant region sequence such as SEQ ID NO: 117 to obtain the final complete heavy chain sequence. The light chain variable regions described above can be recombinantly expressed with the light chain constant region sequence such as SEQ ID NO: 118 to obtain the final complete light chain sequence. 
ASTKGPSVFPLAPCSRSTSESTAALGCLVKDYFPEPVTVSWNSGALTSGVHTFPAV
LQSSGLYSLSSVVTVPSSSLGTKTYTCNVDHKPSNTKVDKRVESKYGPPCPPCPAPEFL
GGPSVFLFPPKPKDTLMISRTPEVTCVVVDVSQEDPEVQFNWYVDGVEVHNAKTKPR
EEQFNSTYRVVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLDSDGSFFLYSR
LTVDKSRWQEGNVFSCSVMHEALHNHYTQKSLSLSLGK
SEQ ID NO: 117
 light chain constant region:



SEQ ID NO：118

Using conventional technical methods in the art, the light chain variable regions and heavy chain variable regions described above can be recombinantly expressed with other optional humanized constant regions and functionally modified humanized constant regions.

Monkey CD96 antigen for following assays: Monkey CD96-3×Flag (SEQ ID NO: 95)

MEFGLSWLFLVAILKGVQCVWGKPFNTEENIYATLGSDVNLTCQTQAKGFLVQMQ
WSKVTDKADLIALYHPQYGFHCAYGSPCESLVTFTQTPENGSKWTLHLRNMSSSVSGR

YECMLTLYPEGMQTKIYNLLIQTHVTPDEWKSNHTIEIEINQTLEIPCFQNSSSEISSEFTY
AWLVEDNGTQQTLISQDHLISSSTLLKDRVKVGIDYRLHLSPVQIFDDGRKFSCHIRVGP
DKILRSSTTIKVFAKPEIPMIVENNSTDVLVERTFTCLLKNVFPKANIIWFIDGSFLHDEK
EGIYITNEERKGKDGFLELKSVLTRVHSDKPAQSDNLTIWCMALSPVPGNKVWNISSEK
ITFLLGSEMSTTDLPPSVTESTLDTQPSPASSVSPTRYPATSSVTLADVSALRPNTTPQSSS
SSVTTQDFNYPWTSSGTDAKKSFSQIPSETYSSSPSGAGSTLHDNVFTSTTRALSEVPTT
ANGSTKTNHVHITGIVVSKPKDGM DYKDHDGDYKDHDIDYKDDDDK

The underlined part is the signal peptide and the italic part is 3×Flag-tag. For the preparation method, see item 1 of Embodiment 2.

**The performance and beneficial effects of the present disclosure were verified by following biochemical test methods.**

### Test example 1: ELISA for CD96 antibody binding to human CD96 protein

The binding capacity of the anti-CD96 antibody was tested by ELISA to determine the binding of the antibody to human CD96 protein. The His-tagged CD96 fusion protein was used to coat the microtiter plate, and the intensity of the signal after adding with the antibody was used for evaluating the binding activity of the antibody and CD96. The specific experimental method is as follows.

CD96-His with the sequence of SEQ ID NO: 4 prepared in Embodiment 1 was diluted to a concentration of 2 µg/ml with PBS buffer (Shanghai Yuanpei Biotechnology Co., Ltd. Cat No. B320), pH 7.4, added to a 96-well microtiter plate (Corning, Cat No. CLS3590-100EA) at a volume of 50µl/well, and placed in an incubator at 37°C for 2 hours. After discarding the liquid, 250 µl/well of 5% skim milk (BD, 232100) blocking solution diluted with PBS was added, and the plate was incubated at 37°C for 2.5 hours or at 4°C overnight (16-18 hours) for blocking. After blocking, the blocking solution was discarded, and the plate was washed 4 times with PBST buffer (pH 7.4 PBS containing 0.05% tween-20). 50 µl/well of different concentrations of the antibodies to be tested (hybridoma purification antibody or humanized antibody) diluted with sample dilution was added, and the plate was incubated in an incubator at 37°C for 1 hour. After incubation, the plate was washed for 4 times with PBST, and 50 µl/well of HRP-labeled goat anti-mouse secondary antibody (Jackson Immuno Research, Cat No. 115-035-003) or goat anti-human secondary antibody (Jackson Immuno Research, Cat No. 109-035-003) diluted with sample dilution was added, followed by incubating at 37°C for 1 hour. After washing the plate 4 times with PBST, 50µl/well of TMB chromogenic substrate (KPL, Cat No. 52-00-03) was added, and the plate was incubated at room temperature for 5-15min, followed by adding 50µl/well of 1M H₂SO₄ to stop the reaction. The absorption value was read at 450nm with a NOVOStar microplate reader, and the EC50 value of the binding of CD96 antibody to human CD96 was calculated (see Table 13).

**Table 13. Affinity ELISA of CD96 antibodies to human CD96**

| **Candidate antibody** | **EC50 (nM)** | **Candidate antibody** | **EC50 (nM)** |
|---|---|---|---|
| ch1718 | 0.084 | h1718-012 | 0.095 |
| ch1719 | 0.065 | h1719-003 | 0.085 |
| ch1720 | 0.074 | h1719-006 | 0.083 |
| ch1721 | 0.075 | h1719-014 | 0.091 |
| ch1722 | 0.094 | h1721-003 | 0.07 |
| h1722-005 | 0.072 | h1722-006 | 0.077 |
| h1722-010 | 0.073 | h1722-017 | 0.113 |
| h1722-018 | 0.091 | | |

The results showed that anti-CD96 chimeric antibodies and humanized antibodies are able to specifically bind to human CD96.

### Test Example 2: ELISA of CD96 antibody binding to cynomolgus CD96 protein

The cross-reactive binding capacity of the anti-CD96 antibody to monkey CD96 was tested by ELISA to determine the binding of the antibody to cynomolgus CD96 protein. The 3 ×FLAG -tagged CD96 fusion protein was used to coat the microtiter plate, and the intensity of the signal after adding with the antibody was used for evaluating the binding activity of the antibody and cynomolgus CD96. The specific experimental method is as follows.

cyno-CD96-3 ×FLAG with the amino acid sequence of SEQ ID NO: 95 prepared in Embodiment 4 was diluted to a concentration of 2 µg/ml with PBS buffer (Shanghai Yuanpei Biotechnology Co., Ltd. Cat No. B320), pH 7.4, added to a 96-well microtiter plate (Corning, Cat No. CLS3590-100EA) at a volume of 50µl/well, and placed in an incubator at 37°C for 2 hours. After discarding the liquid, 250 µl/well of 5% skim milk (BD, 232100) blocking solution diluted with PBS was added, and the plate was incubated in an incubator at 37°C for 2.5 hours or at 4°C overnight (16-18 hours) for blocking. After blocking, the blocking solution was discarded, and the plate was washed 4 times with PBST buffer (pH 7.4 PBS containing 0.05% tween-20). 50 µl/well of different concentrations of the antibodies to be tested (hybridoma purification antibody or humanized antibody) diluted with sample dilution was added, and the plate was incubated in an incubator at 37°C for 1 hour. After incubation, the plate was washed for 4 times with PBST, and 50 µl/well of HRP-labeled goat anti-mouse secondary antibody (Jackson Immuno Research, Cat No. 115-035-003) or goat anti-human secondary antibody (Jackson Immuno Research, Cat No. 109-035-003) diluted with sample dilution was added, followed by incubating the plate at 37°C for 1 hour. After washing the plate 4 times with PBST, 50µl/well of TMB chromogenic substrate (KPL, Cat No. 52-00-03) was added, and the plate was incubated at room temperature for 5-15min, followed by adding 50µl/well of 1M H₂SO₄ to stop the reaction. The absorption value was read at 450nm with a NOVOStar microplate reader, and the EC50 value of the binding of CD96 antibody to monkey CD96 was calculated (see Table 14).

**Table 14 ELISA of CD96 antibodies binding to cynomolgus CD96**

| **Antibody** | **EC50 (nM)** | **Antibody** | **EC50 (nM)** |
|---|---|---|---|
| ch1718 | 0.068 | h1718-012 | 0.075 |
| ch1719 | 0.047 | h1719-014 | 0.065 |
| ch1720 | 0.096 | h1721-003 | 0.037 |
| ch1721 | 0.046 | h1722-010 | 0.047 |
| ch1722 | 0.050 | h1722-017 | 0.047 |
| h1722-018 | 0.051 | | |

The results showed that CD96 chimeric antibodies and humanized antibodies are able to cross-react with monkey CD96.

### Test Example 3: Binding assay of CD96 antibody to human CD96 over-expressing CHO-S cells

The binding capacity of the anti-CD96 antibody was tested by binding experiments of the antibody to human CD96 protein over-expressing CHO-S cells. The full-length CD96 plasmid was transfected into CHO-S cells by electrotransfection, and then screened for two weeks under pressure to detect the expression of CD96. After the over-expressing cells were fixed at the bottom of the 96-well plate, the density of the signal after adding the antibody was used to judge the binding activity of the antibody to CD96 over-expressing CHO-S cells. The specific experimental method is as follows.

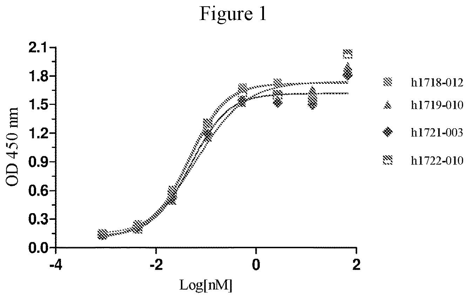

The cells were seeded at a density of 9×10⁴/100µL/well in a 96-well plate and cultured overnight. After aspirating the supernatant, the plate was washed once with PBS, subsequently, 100 µl/well immunostaining fixation solution (Biyuntian, P0098) was added and fixed for one hour at room temperature, and the plate was washed three times with PBS. Once the liquid was discarded, 250 µl/well of 5% skim milk (BD, 232100) blocking solution diluted with PBS was added, and the plate was blocked in an incubator at 37°C for 2.5 hours. After blocking, the blocking solution was discarded, the plate was washed 4 times with PBST buffer (pH 7.4 PBS containing 0.05% tween-20), followed by diluting 50 µl/well of different concentrations of the antibodies (hybridoma purification antibody or humanized antibody) to be tested the plate was incubated in an incubator at 37°C for 2 hours. After incubation, the plate was wash 4 times with PBST. Then 50 µl/well of HRP-labeled goat anti-mouse secondary antibody (Jackson Immuno Research, Cat No. 115-035-003) or goat anti-human secondary antibody (Jackson Immuno Research, Cat No. 109-035-003) diluted with sample dilution was added, thus incubating at 37°C for 1 hour. After washing the plate 4 times with PBST, 50µl/well of TMB chromogenic substrate (KPL, Cat No. 52-00-03) was added, the plate was incubated at room temperature for 10-25min, followed by adding 50µl/well of 1M H₂SO₄ to stop the reaction. The absorption value was read at 450nm with a NOVOStar microplate reader, and the EC50 value of the CD96 antibodies to CD96 overexpressing CHO-S cells was calculated (see Figure 1 and Table 15).

**Table 15 Affinity ELISA of CD96 antibodies to cells overexpressing human CD96-CHOs**

| **Candidate antibody** | **EC50 (nM)** | **Candidate antibody** | **EC50 (nM)** |
|---|---|---|---|
| ch1718 | 0.026 | h1718-012 | 0.031 |
| ch1719 | 0.012 | h1719-003 | 0.028 |
| ch1720 | 0.036 | h1719-006 | 0.016 |
| ch1721 | 0.036 | h1719-014 | 0.02 |
| ch1722 | 0.043 | h1721-003 | 0.074 |
| h1722-005 | 0.045 | h1722-006 | 0.043 |
| h1722-010 | 0.047 | h1722-017 | 0.053 |
| h1722-018 | 0.044 | | |

The results showed that CD96 chimeric antibodies and humanized antibodies are able to specifically bind to CD96 overexpressing CHO-S cells.

### Test Example 4: Anti-CD96 antibody blocks the binding of CD96 antigen and CD155-CHO cells

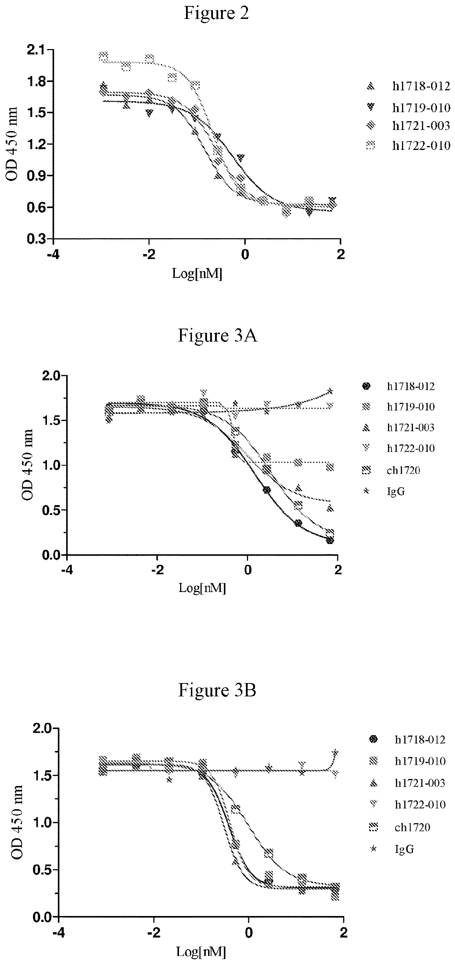

CD155-CHO cells (constructed by Shanghai Hengrui) were seeded in 96-well culture plates at a density of 9×10⁴/100µL/well and cultured overnight. After aspirating the supernatant, the plate was washed once with PBS, 100 µl/well of immunostaining fixation solution (Biyuntian, P0098) was added and fixed for one hour at room temperature, the plate was washed three times with PBS. After discarding the liquid, 250 µl/well of 5% skim milk (BD, 232100) blocking solution diluted with PBS was added, and the plate was blocked in an incubator at 37°C for 2.5 hours. After blocking, the blocking solution was discarded, subsequently, the plate was washed 4 times with PBST buffer (pH 7.4 PBS containing 0.05% tween-20). 50 µl/well of antigen-antibody pre-incubation solution (gradient concentrations of the hybridoma antibody to be tested and CD96-Fc at a final concentration of 0.5 µg/mL were premixed at 37°C for 30 minutes, or gradient concentrations of humanized antibody samples and Bio-CD96-His at a final concentration of 0.5 µg/mL were premixed at 37°C for 30 minutes) (Biotin-labeled kit, Dongren Chemical, Cat No. LK03) was added, and the plate was incubated in an incubator at 37°C for 2 hours. After incubation, the reaction solution in the microtiter plate was discarded, the plate was washed 4 times with PBST, and 50 µl/well of HRP-labeled goat anti-human secondary antibody (Jackson Immuno Research, Cat No. 109-035-003) diluted with sample dilution or HRP-labeled streptavidin diluted with sample dilution (Sigma, Cat No. S2438) was added, followed by incubating at 37°C for 1 hour. After washing the plate 4 times with PBST, 50µl/well of TMB chromogenic substrate (KPL, Cat No. 52-00-03) was added, the plate was incubated at room temperature for 10-20min, followed by adding 50µl/well of 1M H₂SO₄ to stop the reaction. The absorption value was read at 450nm with a NOVOStar microplate reader, and the EC50 value of the effect of CD96 antibody on blocking the binding between the antigens and CD155-CHO cells was calculated (see Figure 2 and Table 16).

**Table 16 CD96 antibodies blocks the binding of CD96 and human CD155-CHO expressing cells**

| **Candidate antibody** | **IC50 (nM)** | **Candidate antibody** | **IC50 (nM)** |
|---|---|---|---|
| ch1718 | 0.096 | h1718-012 | 0.133 |
| ch1719 | 0.137 | h1719-003 | 0.096 |
| ch1720 | 0.149 | h1719-006 | 0.064 |
| ch1721 | 0.214 | h1719-014 | 0.046 |
| ch1722 | 0.159 | h1721-003 | 0.155 |
| h1722-006 | 0.398 | h1722-010 | 0.207 |
| h1722-017 | 0.135 | h1722-018 | 0.134 |

The results showed that CD96 chimeric antibodies and humanized antibodies are able to block the binding of CD96 and CD155-CHO cells.

### Test Example 5: Affinity test of anti-CD96 antibodies by BIAcore

### 1. Test method of human Fc antibody affinity:

Protein A biosensor chip was used to capture antibodies to be tested, then CD96 antigen was flowed on the surface of the chip, and real time reaction signal was detected by Biacore to obtain the binding and dissociation curve. After the dissociation of each experimental cycle was completed, the biosensor chip was washed and regenerated with a glycine-hydrochloric acid regeneration solution (pH 1.5).

### 2. Test method of murine Fc antibody affinity:

CM5 biosensor chip conjugated with anti-murine Fc antibodies was used to capture antibodies to be tested, then CD96 antigen was flowed on the surface of the chip, and real time reaction signal was detected by Biacore to obtain the binding and dissociation curve. After the dissociation of each experimental cycle was completed, the biosensor chip was washed and regenerated with a regeneration solution of mouse antibody capture kit.

The mobile phases were all CD96- ECD-His.

**Table 17 Affinity determination of candidate antibodies by BIACORE**

| **Antibody** | **Affinity(M)** | **Antibody** | **Affinity(M)** |
|---|---|---|---|
| ch1718 | 3.32E-9 | h1721-001 | 2.73E-9 |
| ch1719 | 9.74E-9 | h1721-002 | 2.20E-9 |
| ch1720 | 1.35E-9 | h1721-003 | 1.61E-9 |
| ch1721 | 2.28E-9 | h1721-004 | 2.00E-9 |
| ch1722 | 1.05E-9 | h1721-005 | 2.25E-9 |
| h1718-007 | 3.88E-9 | h1721-006 | 2.29E-9 |
| h1718-008 | 3.37E-9 | h1721-007 | 1.87E-9 |
| h1718-012 | 3.98E-9 | h1721-008 | 2.22E-9 |
| h1718-013 | 4.08E-9 | h1721-009 | 2.01E-9 |
| h1718-014 | 4.31E-9 | h1721-010 | 2.04E-9 |
| h1718-018 | 3.82E-9 | h1721-011 | 2.12E-9 |
| h1718-019 | 3.70E-9 | h1721-012 | 2.14E-9 |
| h1718-020 | 3.41E-9 | h1722-005 | 9.87E-9 |
| h1719-006 | 1.31E-8 | h1722-006 | 1.19E-9 |
| h1719-007 | 2.55E-8 | h1722-007 | 1.31E-9 |
| h1719-008 | 1.76E-8 | h1722-008 | 1.00E-9 |
| h1719-010 | 4.65E-7 | h1722-009 | 4.55E-9 |
| h1719-013 | 1.98E-8 | h1722-010 | 8.60E-10 |
| h1719-014 | 1.49E-8 | h1722-011 | 9.48E-10 |
| h1719-016 | 9.07E-8 | h1722-012 | 7.81E-10 |

| | | | |
|---|---|---|---|
| * For the value of affinity, 3.32E-9M represents 3.32 × 10⁻⁹M. | | | |

All anti-CD96 chimeric antibodies ch1718, ch1720, ch1721, ch1719, ch1722 have strong affinity with human CD96, as do exemplary humanized antibodies.

### Epitope determination of anti-human CD96 monoclonal antibodies

### Test Example 6: Competitive assay of anti-CD96 antibodies

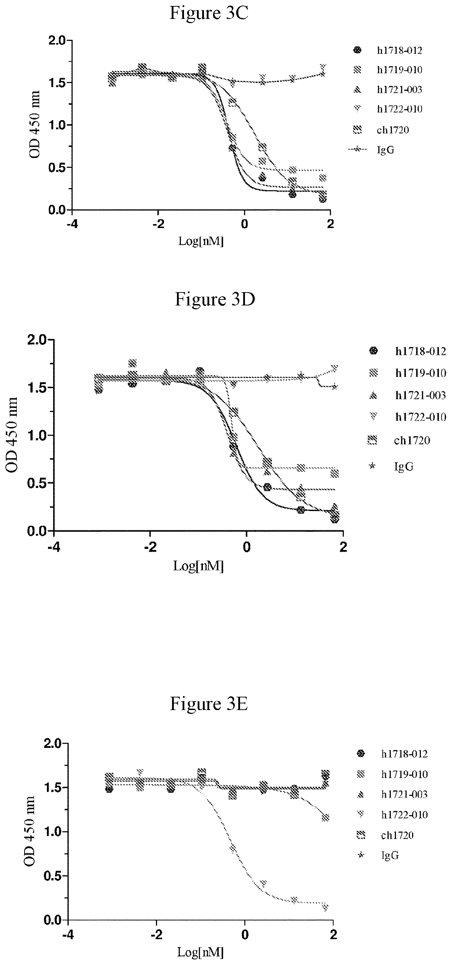

Competitive assay of different antibodies binding to CD96 protein were used to classify binding epitopes of the antibodies. The specific experimental methods are as follows:
Antibody A was diluted to a concentration of 2 µg/ml with PBS buffer (Shanghai Yuanpei Biotechnology Co., Ltd. Cat No. B320), pH 7.4, added to a 96-well microtiter plate (Corning, Cat No. CLS3590-100EA) at a volume of 50µl/well, and coated at 4°C overnight. After discarding the liquid, 250 µl/well of 5% skim milk (BD skim milk, Cat No.232100) blocking solution diluted with PBS was added, and the plate was incubated at 37°C for 3 hours. After blocking, the blocking solution was discarded, and the plate was washed 5 times with PBST buffer (pH 7.4 PBS containing 0.05% tween-20) and stored for later use. Antibody B was diluted to 40 µg/ml and 8 µg/ml with 1% BSA, and Biotinylated CD96 antigen was simultaneously diluted to 4 µg/mL. Subsequently, antibody B and antigen were mixed in a 1: 1 volume ratio, pre-incubated at 37°C for 45 minutes, added into the enzyme plate coated with antibody A, thus incubating the plate in an incubator at 37°C for 1.5 hours. After incubation, the plate was washed for 5 times with PBST, and 50 µl/well of HRP-labeled streptavidin (Sigma, Cat No. S2438) diluted with sample dilution was added, followed by incubating the plate at 37°C for 1 hour. After washing the plate 5 times with PBST, 50µl/well of TMB chromogenic substrate (KPL, Cat No. 52-00-03) was added for color development, followed by adding 50µl/well of 1M H₂SO₄ to stop the reaction. The absorption value was read at 450nm with a NOVOStar microplate reader, and the OD value was used to determine whether A and B CD96 antibodies compete with each other. Antibodies that compete with each other fall into the same class of antibodies, otherwise they are of different classes of antibodies (see Figure 3 and Table 18).

**Table 18 Detection of competition between different groups of antibodies**

| | The coated antibody (antibody A) | | | | |
|---|---|---|---|---|---|
| Competitive antibody (antibody B) | h1718-012 | ch1720 | h1721-003 | h1719-014 | h1722-010 |
| h1718-012 | 100.0% | 102.9% | 102.6% | 94.8% | -11.1% |
| ch1720 | 94.4% | 100.0% | 98.7% | 92.3% | -2.4% |
| h1721-003 | 73.6% | 92.6% | 100.0% | 96.0% | -0.3% |
| h1719-014 | 44.3% | 67.7% | 84.6% | 100.0% | 28.6% |
| h1722-010 | -3.5% | -11.5% | -8.5% | 8.5% | 100.0% |

The results of competitive assay showed that there was a competition between h1721-003, h1719-014, h1718-012 and ch1720 antibodies, all of which did not compete with h1722-010 antibody.

**Test Example 7: ELISA of anti-CD96 antibody binding to different peptide fragments of CD96 protein**

The epitopes recognized by anti-CD96 antibody were detected by the assay of binding the antibody to different polypeptide fragments of CD96 protein. The fragments of different polypeptides of the CD96 antigen were coated in the microtiter plate. The signal density after the antibody was added was used to judge the binding activity of the antibody and the polypeptide. The specific experimental method is as follows:
The polypeptides were diluted to a concentration of 20 µg/ml with PBS buffer (Shanghai Yuanpei Biotechnology Co., Ltd. Cat No. B320), pH 7.4, added to a 96-well microtiter plate (Corning, Cat No. CLS3590-100EA) at a volume of 50µl/well, and coated at 4°C overnight. After discarding the liquid, 250 µl/well of 5% skim milk (BD skim milk, Cat No.232100) blocking solution diluted with PBS was added, and the plate was incubated in an incubator at 37°C for 3 hours. After blocking, the blocking solution was discarded, and the plate was washed 5 times with PBST buffer (pH 7.4 PBS containing 0.05% tween-20). 50 µl/well of different concentrations of the antibodies to be tested (hybridoma purification antibody or humanized antibody) diluted with sample dilution was added, and the plate was incubated in an incubator at 37°C for 2 hours. After incubation, the plate was washed for 5 times with PBST, and 50 µl/well of HRP-labeled goat anti-mouse secondary antibody (Jackson Immuno Research, Cat No. 115-035-003) or goat anti-human secondary antibody (Jackson Immuno Research, Cat No. 109-035-003) or anti-Rat-IgG HRP (Jackson Immuno Research, Cat No. 112-035-003) diluted with sample dilution was added, followed by incubating the plate at 37°C for 1 hour. After washing the plate 5 times with PBST, 50µl/well of TMB chromogenic substrate (KPL, Cat No. 52-00-03) was added, the plate was incubated at room temperature for 5-15min, followed by adding 50µl/well of 1M H₂SO₄ to stop the reaction. The absorption value was read at 450nm with a microplate reader (Thermo scientific MuLtiskan MK3), and the epitopes that CD96 antibody may recognize were analyzed.

**Table 19 List of epitope peptides**

| No. (#) | Polypeptide sequence (position in the extracellular region of CD96, sequence number) | ch1718 | ch1720 | ch1721 | ch1719 |
|---|---|---|---|---|---|
| 1 | GSDVNLTCQTQTVGFFVQMQ (17-36, SEQ ID NO: 85) | +++ | - | + | - |
| 2 | FVQMQWSKVTNKIDLIAVYH (32-51, SEQ ID NO: 86) | +++ | - | +/- | - |
| 3 | IAVYHPQYGFYCAYGRPCES (47-66, SEQ ID NO: 87) | ++++ | +++ | ++++ | +++ |
| 4 | RPCESLVTFTETPENGSKWT (62-81, SEQ ID NO: 88) | +++ | - | - | - |
| 5 | GSKWTLHLRNMSCSVSGRYE (77-86, SEQ ID NO: 89) | +++ | - | + | - |
| 6 | SGRYECMLVLYPEGIQTKIY (92-111, SEQ ID NO: 90) | +++ | - | +/- | - |
| 7 | QTKIYNLLIQTHVTADEWNS (107-126, SEQ ID NO: 91) | +++ | - | +/- | - |
| 8 | DEWNSNHTIEIEINQTLEIP (122-141, SEQ ID NO: 92) | +++ | - | +/- | - |
| 9 | TLEIPCFQNSSSKISSEFTY (137-156, SEQ ID NO: 93) | +++ | - | - | - |
| 10 | KISSEFTYAWSVEDNGTQETLI (149-170, SEQ ID NO: 94) | ++ | - | - | - |
| 11 | QETLISQNHLISNSTLLKDR (166-185, SEQ ID NO: 96) | +++ | - | +/- | - |
| 12 | LLKDRVKLGTDYRLHLSPVQ (181-200, SEQ ID NO: 97) | +++ | - | +/- | - |
| 13 | LSPVQIFDDGRKFSCHIRVG (196-215, SEQ ID NO: 98) | +++ | - | +/- | - |
| 14 | HIRVGPNKILRSSTTVKVFA (211-230, SEQ ID NO: 99) | +++ | - | + | - |
| 15 | LRSSTTVKVFAKPEIPVIVE (220-239, SEQ ID NO: 100) | +++ | - | +/- | - |
| 16 | PVIVENNSTDVLVERRFTCL (235-254, SEQ ID NO: 101) | +++ | - | +/- | - |
| 17 | RFTCLLKNVFPKANITWFID (250-269, SEQ ID NO: 102) | ++++ | - | + | - |
| 18 | TWFIDGSFLHDEKEGIYITN (265-284, SEQ ID NO: 103) | +++ | - | - | - |
| 19 | IYITNEERKGKDGFLELKSV (280-299, SEQ ID NO: 104) | +++ | - | - | - |
| 20 | ELKSVLTRVHSNKPAQSDNL (295-314, SEQ ID NO: 105) | ++ | - | +/- | - |
| 21 | QSDNLTIWCMALSPVPGNKV (310-329, SEQ ID NO: 106) | ++ | - | - | - |
| 22 | PGNKVWNISSEKITFLLGSE (325-344, SEQ ID NO: 107) | ++ | - | +/- | - |

| | | | | | |
|---|---|---|---|---|---|
| Note: "+" indicates the binding intensity. The more "+", the stronger the binding activity; "-" indicates no binding. | | | | | |

According to the above results, antibodies ch1718, ch1720, ch1721 and ch1719 bind strongly to the 3# antigen fragment (SEQ ID NO: 87). The 3# antigen fragment contains epitopes of antibodies ch1718, ch1720, ch1721 and ch1719.

**Test Example 8: Detection of Binding of CD96 antibody to different peptides of CD96 by FACS**

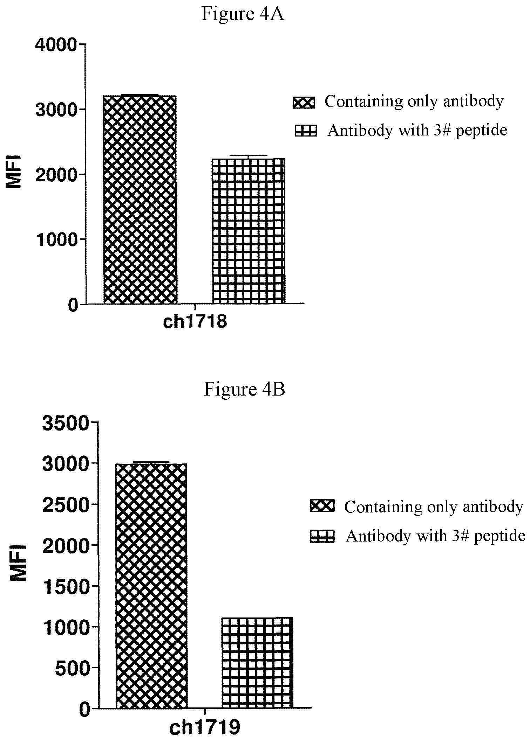

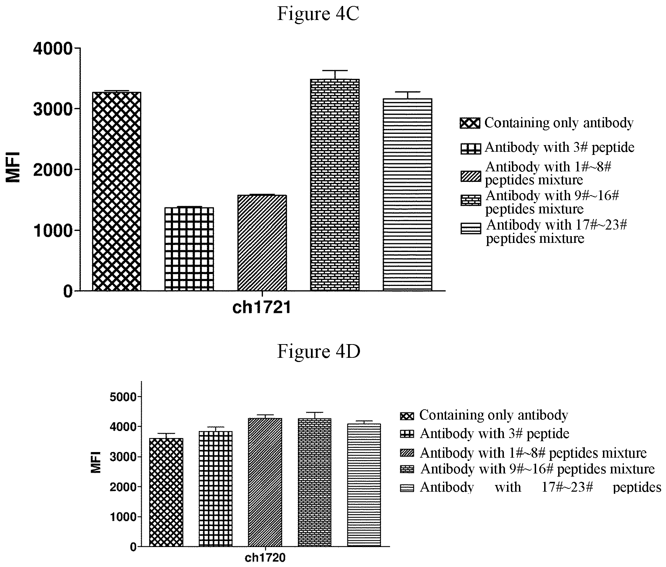

In order to verify that the binding of the antibody to CD96 protein polypeptide fragment is specific, we designed the following FACS for verification. While polypeptide fragments were incubated and bound to an antibody, epitopes of the antibody binding to CD96 protein were occupied, and then the signal of the antibody binding to CD96 protein on the cell surface decreased. The specific experimental methods are as follows:
The antibody samples were diluted to 1.2 µg/mL with 1% BSA, and the peptides were diluted to 40 µg/mL synchronously. The peptides and antibody sample were mixed at a volume ratio of 1: 1 and pre-incubated at 37°C for 90 minutes. CD96-CHOs cells were collected and washed once in PBS, then dispensed according to 0.5×10⁶/test. The cells with 100 µL of peptide-antibody mixture were resuspended, incubated at 4°C for 60 minutes, followed by washing 3 times with 1% BSA. Cells were resuspended with 100 µL dilution (1:400, 1:40, 1:100) of Alexa Fluor 488 anti-human IgG Fc (thermofisher, A-11013), PE goat anti-mouse IgG (Biolegend, 405307), or PE goat anti-Rat IgG (Biolegend, 405406), incubated at 4°C for 40 minutes, followed by washing 3 times with 1% BSA. The cells were resuspended with 200 µL of 1% BSA, MFI of each sample was read on a flow cytometer BD FACSCanto II, and the data were analyzed with GraphPad Prism 5 to make a histogram. MFI difference between the sample without polypetides incubation and the one incubated with polypeptides was used to determine the antigenic epitope recognized by the CD96 antibody. The test results are shown in Figure 4.

The test results showed that the chimeric antibodies ch1721, ch1719 and ch1718 can bind the epitope segment of SEQ ID NO: 87 in the extracellular region of human CD96.

### Test example 9: NK cell-mediated cell killing capability assay

In order to study the effect of CD96 antibody on the killing capability of NK cells, human peripheral blood mononuclear cells (PBMC) were collected and purified to extracted natural killer cells (NK). After 4h of co-cultured with human colorectal cancer cell line WiDr (Cat No. ATCC-CCL-218), the secretion level of lactate dehydrogenase (LDH) was detected.

The experiment process is briefly described as follows:
The human colorectal cancer cell line WiDr was cultured in MEM medium supplemented with 10% (v/v) fetal bovine serum (FBS) at 37°C and 5% CO₂. Fresh blood was used to obtain PBMC by Ficoll-Hypaque density gradient centrifugation (Stem Cell Technologies). Human primary NK cells were extracted from freshly isolated PBMC (Miltenyi, CAT# 130-092-657) and cultured in RPMI 1640 medium supplemented with 10% (v/v) FBS and cultured at 37°C and 5% CO₂. Human primary NK cells were seeded into a 6-well cell culture plate with a cell density of about 2 × 10⁶/ml. After adding 100 U/mL human IL-2 (Peprotech, 200-02-100) and culturing overnight, phenol red-free RPMI 1640 medium was used to wash the cells, the cells were resuspended and seeded into a 96-well U-bottom plate with a cell density of about 3 × 10⁵/well. Synchronously, gradient diluted antibody samples (diluted with PBS) or the equivalent amount of isotype IgG were used as blank control. Wells added with IL-2, NK cells and cancer cells were used as blank controls. After incubating at 37°C for 1 hour in an incubator at 5% CO₂, the target cells WiDr were co-cultured with human primary NK cells at a ratio of 1: 1. After incubating at 37°C for 4 hours in an incubator in 5% CO₂, the cell culture supernatant was collected. CytoTox 96® Non-Radioactive Cytotoxicity Assay (Promega, CAT# G1780) was used to detect the LDH secretion level in the cell culture supernatant. For specific operations, please refer to the reagent instructions. The percentage of specific cell lysis is determined by the following formula: %lysis=100×(ER-SR1-SR2)/(MR-SR1), wherein ER, SR (1&2) and MR represent experiments, spontaneous (1 indicates the target cell, 2 indicates human primary NK cells) and maximum LDH release. Spontaneous release is LDH released by target cells or human primary NK cells cultured in the culture medium alone, and the maximum release is LDH measured when all target cells are lysed using lysate.

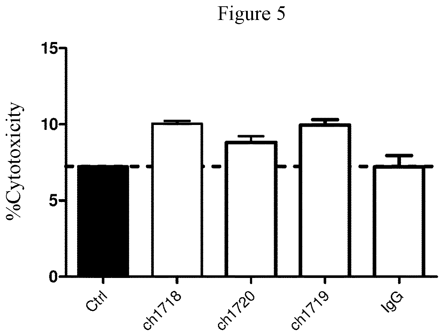

The experimental results are shown in Figure 5. The results show that CD96 antibodies ch1718, ch1720 and ch1719 can enhance the killing of tumor cells by NK cells to varying degrees.

Although the specific embodiments of the present invention have been described above, those skilled in the art will understand that these are merely illustrative. Many changes or modifications may be made to these embodiments without departing from the principle and essence of the invention. Therefore, the scope of the invention is defined by the appended claims.

序列表

<110>  江苏恒瑞医药股份有限公司、上海恒瑞医药有限公司

<120>  CD96 抗体、其抗原结合片段及医药用途

<130>  P184016530C

<150>  CN201711107331.5
<151>  2017-11-10

<160>  123

<170>  SIPOSequenceListing 1.0

<210>  1
<211>  511
<212>  PRT
<213>  人工序列(Artificial Sequence)

<220>
<221>  PEPTIDE
<223>  带 Flag 标签的 CD96 胞外区序列

<400>  1
Met Glu Lys Lys Trp Lys Tyr Cys Ala Val Tyr Tyr Ile Ile Gln Ile
1               5                   10                  15
His Phe Val Lys Gly Val Trp Glu Lys Thr Val Asn Thr Glu Glu Asn
            20                  25                  30
Val Tyr Ala Thr Leu Gly Ser Asp Val Asn Leu Thr Cys Gln Thr Gln
        35                  40                  45
Thr Val Gly Phe Phe Val Gln Met Gln Trp Ser Lys Val Thr Asn Lys
    50                  55                  60
Ile Asp Leu Ile Ala Val Tyr His Pro Gln Tyr Gly Phe Tyr Cys Ala
65                  70                  75                  80
Tyr Gly Arg Pro Cys Glu Ser Leu Val Thr Phe Thr Glu Thr Pro Glu
                85                  90                  95
Asn Gly Ser Lys Trp Thr Leu His Leu Arg Asn Met Ser Cys Ser Val
            100                 105                 110
Ser Gly Arg Tyr Glu Cys Met Leu Val Leu Tyr Pro Glu Gly Ile Gln
        115                 120                 125
Thr Lys Ile Tyr Asn Leu Leu Ile Gln Thr His Val Thr Ala Asp Glu
    130                 135                 140
Trp Asn Ser Asn His Thr Ile Glu Ile Glu Ile Asn Gln Thr Leu Glu
145                 150                 155                 160
Ile Pro Cys Phe Gln Asn Ser Ser Ser Lys Ile Ser Ser Glu Phe Thr
                165                 170                 175
Tyr Ala Trp Ser Val Glu Asp Asn Gly Thr Gln Glu Thr Leu Ile Ser
            180                 185                 190
Gln Asn His Leu Ile Ser Asn Ser Thr Leu Leu Lys Asp Arg Val Lys
        195                 200                 205
Leu Gly Thr Asp Tyr Arg Leu His Leu Ser Pro Val Gln Ile Phe Asp
    210                 215                 220
Asp Gly Arg Lys Phe Ser Cys His Ile Arg Val Gly Pro Asn Lys Ile
225                 230                 235                 240

Leu Arg Ser Ser Thr Thr Val Lys Val Phe Ala Lys Pro Glu Ile Pro
                245                 250                 255
Val Ile Val Glu Asn Asn Ser Thr Asp Val Leu Val Glu Arg Arg Phe
            260                 265                 270
Thr Cys Leu Leu Lys Asn Val Phe Pro Lys Ala Asn Ile Thr Trp Phe
        275                 280                 285
Ile Asp Gly Ser Phe Leu His Asp Glu Lys Glu Gly Ile Tyr Ile Thr
    290                 295                 300
Asn Glu Glu Arg Lys Gly Lys Asp Gly Phe Leu Glu Leu Lys Ser Val
305                 310                 315                 320
Leu Thr Arg Val His Ser Asn Lys Pro Ala Gln Ser Asp Asn Leu Thr
                325                 330                 335
Ile Trp Cys Met Ala Leu Ser Pro Val Pro Gly Asn Lys Val Trp Asn
            340                 345                 350
Ile Ser Ser Glu Lys Ile Thr Phe Leu Leu Gly Ser Glu Ile Ser Ser
        355                 360                 365
Thr Asp Pro Pro Leu Ser Val Thr Glu Ser Thr Leu Asp Thr Gln Pro
    370                 375                 380
Ser Pro Ala Ser Ser Val Ser Pro Ala Arg Tyr Pro Ala Thr Ser Ser
385                 390                 395                 400
Val Thr Leu Val Asp Val Ser Ala Leu Arg Pro Asn Thr Thr Pro Gln
                405                 410                 415
Pro Ser Asn Ser Ser Met Thr Thr Arg Gly Phe Asn Tyr Pro Trp Thr
            420                 425                 430
Ser Ser Gly Thr Asp Thr Lys Lys Ser Val Ser Arg Ile Pro Ser Glu
        435                 440                 445
Thr Tyr Ser Ser Ser Pro Ser Gly Ala Gly Ser Thr Leu His Asp Asn
    450                 455                 460
Val Phe Thr Ser Thr Ala Arg Ala Phe Ser Glu Val Pro Thr Thr Ala
465                 470                 475                 480
Asn Gly Ser Thr Lys Thr Asn His Val His Ile Thr Gly Ile Val Val
                485                 490                 495
Asn Lys Pro Lys Asp Gly Met Asp Tyr Lys Asp Asp Asp Asp Lys
            500                 505                 510

<210>  2
<211>  734
<212>  PRT
<213>  人工序列(Artificial Sequence)

<220>
<221>  PEPTIDE
<223>  CD96胞外区和mIgG2a Fc的融合蛋白序列

<400>  2
Met Glu Phe Gly Leu Ser Trp Leu Phe Leu Val Ala Ile Leu Lys Gly
1               5                   10                  15
Val Gln Cys Val Trp Glu Lys Thr Val Asn Thr Glu Glu Asn Val Tyr
            20                  25                  30
Ala Thr Leu Gly Ser Asp Val Asn Leu Thr Cys Gln Thr Gln Thr Val
        35                  40                  45
Gly Phe Phe Val Gln Met Gln Trp Ser Lys Val Thr Asn Lys Ile Asp
    50                  55                  60
Leu Ile Ala Val Tyr His Pro Gln Tyr Gly Phe Tyr Cys Ala Tyr Gly
65                  70                  75                  80
Arg Pro Cys Glu Ser Leu Val Thr Phe Thr Glu Thr Pro Glu Asn Gly
                85                  90                  95

Ser Lys Trp Thr Leu His Leu Arg Asn Met Ser Cys Ser Val Ser Gly
            100                 105                 110
Arg Tyr Glu Cys Met Leu Val Leu Tyr Pro Glu Gly Ile Gln Thr Lys
        115                 120                 125
Ile Tyr Asn Leu Leu Ile Gln Thr His Val Thr Ala Asp Glu Trp Asn
    130                 135                 140
Ser Asn His Thr Ile Glu Ile Glu Ile Asn Gln Thr Leu Glu Ile Pro
145                 150                 155                 160
Cys Phe Gln Asn Ser Ser Ser Lys Ile Ser Ser Glu Phe Thr Tyr Ala
                165                 170                 175
Trp Ser Val Glu Asp Asn Gly Thr Gln Glu Thr Leu Ile Ser Gln Asn
            180                 185                 190
His Leu Ile Ser Asn Ser Thr Leu Leu Lys Asp Arg Val Lys Leu Gly
        195                 200                 205
Thr Asp Tyr Arg Leu His Leu Ser Pro Val Gln Ile Phe Asp Asp Gly
    210                 215                 220
Arg Lys Phe Ser Cys His Ile Arg Val Gly Pro Asn Lys Ile Leu Arg
225                 230                 235                 240
Ser Ser Thr Thr Val Lys Val Phe Ala Lys Pro Glu Ile Pro Val Ile
                245                 250                 255
Val Glu Asn Asn Ser Thr Asp Val Leu Val Glu Arg Arg Phe Thr Cys
            260                 265                 270
Leu Leu Lys Asn Val Phe Pro Lys Ala Asn Ile Thr Trp Phe Ile Asp
        275                 280                 285
Gly Ser Phe Leu His Asp Glu Lys Glu Gly Ile Tyr Ile Thr Asn Glu
    290                 295                 300
Glu Arg Lys Gly Lys Asp Gly Phe Leu Glu Leu Lys Ser Val Leu Thr
305                 310                 315                 320
Arg Val His Ser Asn Lys Pro Ala Gln Ser Asp Asn Leu Thr Ile Trp
                325                 330                 335
Cys Met Ala Leu Ser Pro Val Pro Gly Asn Lys Val Trp Asn Ile Ser
            340                 345                 350
Ser Glu Lys Ile Thr Phe Leu Leu Gly Ser Glu Ile Ser Ser Thr Asp
        355                 360                 365
Pro Pro Leu Ser Val Thr Glu Ser Thr Leu Asp Thr Gln Pro Ser Pro
    370                 375                 380
Ala Ser Ser Val Ser Pro Ala Arg Tyr Pro Ala Thr Ser Ser Val Thr
385                 390                 395                 400
Leu Val Asp Val Ser Ala Leu Arg Pro Asn Thr Thr Pro Gln Pro Ser
                405                 410                 415
Asn Ser Ser Met Thr Thr Arg Gly Phe Asn Tyr Pro Trp Thr Ser Ser
            420                 425                 430
Gly Thr Asp Thr Lys Lys Ser Val Ser Arg Ile Pro Ser Glu Thr Tyr
        435                 440                 445
Ser Ser Ser Pro Ser Gly Ala Gly Ser Thr Leu His Asp Asn Val Phe
    450                 455                 460
Thr Ser Thr Ala Arg Ala Phe Ser Glu Val Pro Thr Thr Ala Asn Gly
465                 470                 475                 480
Ser Thr Lys Thr Asn His Val His Ile Thr Gly Ile Val Val Asn Lys
                485                 490                 495
Pro Lys Asp Gly Met Glu Pro Arg Gly Pro Thr Ile Lys Pro Cys Pro
            500                 505                 510
Pro Cys Lys Cys Pro Ala Pro Asn Leu Leu Gly Gly Pro Ser Val Phe
        515                 520                 525
Ile Phe Pro Pro Lys Ile Lys Asp Val Leu Met Ile Ser Leu Ser Pro
    530                 535                 540
Ile Val Thr Cys Val Val Val Asp Val Ser Glu Asp Asp Pro Asp Val
545                 550                 555                 560

Gln Ile Ser Trp Phe Val Asn Asn Val Glu Val His Thr Ala Gln Thr
                565                 570                 575
Gln Thr His Arg Glu Asp Tyr Asn Ser Thr Leu Arg Val Val Ser Ala
            580                 585                 590
Leu Pro Ile Gln His Gln Asp Trp Met Ser Gly Lys Glu Phe Lys Cys
        595                 600                 605
Lys Val Asn Asn Lys Asp Leu Pro Ala Pro Ile Glu Arg Thr Ile Ser
    610                 615                 620
Lys Pro Lys Gly Ser Val Arg Ala Pro Gln Val Tyr Val Leu Pro Pro
625                 630                 635                 640
Pro Glu Glu Glu Met Thr Lys Lys Gln Val Thr Leu Thr Cys Met Val
                645                 650                 655
Thr Asp Phe Met Pro Glu Asp Ile Tyr Val Glu Trp Thr Asn Asn Gly
            660                 665                 670
Lys Thr Glu Leu Asn Tyr Lys Asn Thr Glu Pro Val Leu Asp Ser Asp
        675                 680                 685
Gly Ser Tyr Phe Met Tyr Ser Lys Leu Arg Val Glu Lys Lys Asn Trp
    690                 695                 700
Val Glu Arg Asn Ser Tyr Ser Cys Ser Val Val His Glu Gly Leu His
705                 710                 715                 720
Asn His His Thr Thr Lys Ser Phe Ser Arg Thr Pro Gly Lys
                725                 730

<210>  3
<211>  569
<212>  PRT
<213>  智人(Homo sapiens)

<220>
<221>  PEPTIDE
<223>  全长CD96序列

<400>  3
Met Glu Lys Lys Trp Lys Tyr Cys Ala Val Tyr Tyr Ile Ile Gln Ile
1               5                   10                  15
His Phe Val Lys Gly Val Trp Glu Lys Thr Val Asn Thr Glu Glu Asn
            20                  25                  30
Val Tyr Ala Thr Leu Gly Ser Asp Val Asn Leu Thr Cys Gln Thr Gln
        35                  40                  45
Thr Val Gly Phe Phe Val Gln Met Gln Trp Ser Lys Val Thr Asn Lys
    50                  55                  60
Ile Asp Leu Ile Ala Val Tyr His Pro Gln Tyr Gly Phe Tyr Cys Ala
65                  70                  75                  80
Tyr Gly Arg Pro Cys Glu Ser Leu Val Thr Phe Thr Glu Thr Pro Glu
                85                  90                  95
Asn Gly Ser Lys Trp Thr Leu His Leu Arg Asn Met Ser Cys Ser Val
            100                 105                 110
Ser Gly Arg Tyr Glu Cys Met Leu Val Leu Tyr Pro Glu Gly Ile Gln
        115                 120                 125
Thr Lys Ile Tyr Asn Leu Leu Ile Gln Thr His Val Thr Ala Asp Glu
    130                 135                 140
Trp Asn Ser Asn His Thr Ile Glu Ile Glu Ile Asn Gln Thr Leu Glu
145                 150                 155                 160
Ile Pro Cys Phe Gln Asn Ser Ser Ser Lys Ile Ser Ser Glu Phe Thr
                165                 170                 175
Tyr Ala Trp Ser Val Glu Asp Asn Gly Thr Gln Glu Thr Leu Ile Ser

180                 185                 190
Gln Asn His Leu Ile Ser Asn Ser Thr Leu Leu Lys Asp Arg Val Lys
        195                 200                 205
Leu Gly Thr Asp Tyr Arg Leu His Leu Ser Pro Val Gln Ile Phe Asp
    210                 215                 220
Asp Gly Arg Lys Phe Ser Cys His Ile Arg Val Gly Pro Asn Lys Ile
225                 230                 235                 240
Leu Arg Ser Ser Thr Thr Val Lys Val Phe Ala Lys Pro Glu Ile Pro
                245                 250                 255
Val Ile Val Glu Asn Asn Ser Thr Asp Val Leu Val Glu Arg Arg Phe
            260                 265                 270
Thr Cys Leu Leu Lys Asn Val Phe Pro Lys Ala Asn Ile Thr Trp Phe
        275                 280                 285
Ile Asp Gly Ser Phe Leu His Asp Glu Lys Glu Gly Ile Tyr Ile Thr
    290                 295                 300
Asn Glu Glu Arg Lys Gly Lys Asp Gly Phe Leu Glu Leu Lys Ser Val
305                 310                 315                 320
Leu Thr Arg Val His Ser Asn Lys Pro Ala Gln Ser Asp Asn Leu Thr
                325                 330                 335
Ile Trp Cys Met Ala Leu Ser Pro Val Pro Gly Asn Lys Val Trp Asn
            340                 345                 350
Ile Ser Ser Glu Lys Ile Thr Phe Leu Leu Gly Ser Glu Ile Ser Ser
        355                 360                 365
Thr Asp Pro Pro Leu Ser Val Thr Glu Ser Thr Leu Asp Thr Gln Pro
    370                 375                 380
Ser Pro Ala Ser Ser Val Ser Pro Ala Arg Tyr Pro Ala Thr Ser Ser
385                 390                 395                 400
Val Thr Leu Val Asp Val Ser Ala Leu Arg Pro Asn Thr Thr Pro Gln
                405                 410                 415
Pro Ser Asn Ser Ser Met Thr Thr Arg Gly Phe Asn Tyr Pro Trp Thr
            420                 425                 430
Ser Ser Gly Thr Asp Thr Lys Lys Ser Val Ser Arg Ile Pro Ser Glu
        435                 440                 445
Thr Tyr Ser Ser Ser Pro Ser Gly Ala Gly Ser Thr Leu His Asp Asn
    450                 455                 460
Val Phe Thr Ser Thr Ala Arg Ala Phe Ser Glu Val Pro Thr Thr Ala
465                 470                 475                 480
Asn Gly Ser Thr Lys Thr Asn His Val His Ile Thr Gly Ile Val Val
                485                 490                 495
Asn Lys Pro Lys Asp Gly Met Ser Trp Pro Val Ile Val Ala Ala Leu
            500                 505                 510
Leu Phe Cys Cys Met Ile Leu Phe Gly Leu Gly Val Arg Lys Trp Cys
        515                 520                 525
Gln Tyr Gln Lys Glu Ile Met Glu Arg Pro Pro Pro Phe Lys Pro Pro
    530                 535                 540
Pro Pro Pro Ile Lys Tyr Thr Cys Ile Gln Glu Pro Asn Glu Ser Asp
545                 550                 555                 560
Leu Pro Tyr His Glu Met Glu Thr Leu
                565

<210>  4
<211>  509
<212>  PRT
<213>  人工序列(Artificial Sequence)

<220>
<221>  PEPTIDE

<223>  带 His 标签的 CD96 胞外区

<400>  4
Met Glu Lys Lys Trp Lys Tyr Cys Ala Val Tyr Tyr Ile Ile Gln Ile
1               5                   10                  15
His Phe Val Lys Gly Val Trp Glu Lys Thr Val Asn Thr Glu Glu Asn
            20                  25                  30
Val Tyr Ala Thr Leu Gly Ser Asp Val Asn Leu Thr Cys Gln Thr Gln
        35                  40                  45
Thr Val Gly Phe Phe Val Gln Met Gln Trp Ser Lys Val Thr Asn Lys
    50                  55                  60
Ile Asp Leu Ile Ala Val Tyr His Pro Gln Tyr Gly Phe Tyr Cys Ala
65                  70                  75                  80
Tyr Gly Arg Pro Cys Glu Ser Leu Val Thr Phe Thr Glu Thr Pro Glu
                85                  90                  95
Asn Gly Ser Lys Trp Thr Leu His Leu Arg Asn Met Ser Cys Ser Val
            100                 105                 110
Ser Gly Arg Tyr Glu Cys Met Leu Val Leu Tyr Pro Glu Gly Ile Gln
        115                 120                 125
Thr Lys Ile Tyr Asn Leu Leu Ile Gln Thr His Val Thr Ala Asp Glu
    130                 135                 140
Trp Asn Ser Asn His Thr Ile Glu Ile Glu Ile Asn Gln Thr Leu Glu
145                 150                 155                 160
Ile Pro Cys Phe Gln Asn Ser Ser Ser Lys Ile Ser Ser Glu Phe Thr
                165                 170                 175
Tyr Ala Trp Ser Val Glu Asp Asn Gly Thr Gln Glu Thr Leu Ile Ser
            180                 185                 190
Gln Asn His Leu Ile Ser Asn Ser Thr Leu Leu Lys Asp Arg Val Lys
        195                 200                 205
Leu Gly Thr Asp Tyr Arg Leu His Leu Ser Pro Val Gln Ile Phe Asp
    210                 215                 220
Asp Gly Arg Lys Phe Ser Cys His Ile Arg Val Gly Pro Asn Lys Ile
225                 230                 235                 240
Leu Arg Ser Ser Thr Thr Val Lys Val Phe Ala Lys Pro Glu Ile Pro
                245                 250                 255
Val Ile Val Glu Asn Asn Ser Thr Asp Val Leu Val Glu Arg Arg Phe
            260                 265                 270
Thr Cys Leu Leu Lys Asn Val Phe Pro Lys Ala Asn Ile Thr Trp Phe
        275                 280                 285
Ile Asp Gly Ser Phe Leu His Asp Glu Lys Glu Gly Ile Tyr Ile Thr
    290                 295                 300
Asn Glu Glu Arg Lys Gly Lys Asp Gly Phe Leu Glu Leu Lys Ser Val
305                 310                 315                 320
Leu Thr Arg Val His Ser Asn Lys Pro Ala Gln Ser Asp Asn Leu Thr
                325                 330                 335
Ile Trp Cys Met Ala Leu Ser Pro Val Pro Gly Asn Lys Val Trp Asn
            340                 345                 350
Ile Ser Ser Glu Lys Ile Thr Phe Leu Leu Gly Ser Glu Ile Ser Ser
        355                 360                 365
Thr Asp Pro Pro Leu Ser Val Thr Glu Ser Thr Leu Asp Thr Gln Pro
    370                 375                 380
Ser Pro Ala Ser Ser Val Ser Pro Ala Arg Tyr Pro Ala Thr Ser Ser
385                 390                 395                 400
Val Thr Leu Val Asp Val Ser Ala Leu Arg Pro Asn Thr Thr Pro Gln
                405                 410                 415
Pro Ser Asn Ser Ser Met Thr Thr Arg Gly Phe Asn Tyr Pro Trp Thr
            420                 425                 430

Ser Ser Gly Thr Asp Thr Lys Lys Ser Val Ser Arg Ile Pro Ser Glu
        435                 440                 445
Thr Tyr Ser Ser Ser Pro Ser Gly Ala Gly Ser Thr Leu His Asp Asn
    450                 455                 460
Val Phe Thr Ser Thr Ala Arg Ala Phe Ser Glu Val Pro Thr Thr Ala
465                 470                 475                 480
Asn Gly Ser Thr Lys Thr Asn His Val His Ile Thr Gly Ile Val Val
                485                 490                 495
Asn Lys Pro Lys Asp Gly Met His His His His His His
            500                 505

<210>  5
<211>  733
<212>  PRT
<213>  人工序列(Artificial Sequence)

<220>
<221>  PEPTIDE
<223>  CD96胞外区和hIgG1Fc的融合蛋白

<400>  5
Met Glu Phe Gly Leu Ser Trp Leu Phe Leu Val Ala Ile Leu Lys Gly
1               5                   10                  15
Val Gln Cys Val Trp Glu Lys Thr Val Asn Thr Glu Glu Asn Val Tyr
            20                  25                  30
Ala Thr Leu Gly Ser Asp Val Asn Leu Thr Cys Gln Thr Gln Thr Val
        35                  40                  45
Gly Phe Phe Val Gln Met Gln Trp Ser Lys Val Thr Asn Lys Ile Asp
    50                  55                  60
Leu Ile Ala Val Tyr His Pro Gln Tyr Gly Phe Tyr Cys Ala Tyr Gly
65                  70                  75                  80
Arg Pro Cys Glu Ser Leu Val Thr Phe Thr Glu Thr Pro Glu Asn Gly
                85                  90                  95
Ser Lys Trp Thr Leu His Leu Arg Asn Met Ser Cys Ser Val Ser Gly
            100                 105                 110
Arg Tyr Glu Cys Met Leu Val Leu Tyr Pro Glu Gly Ile Gln Thr Lys
        115                 120                 125
Ile Tyr Asn Leu Leu Ile Gln Thr His Val Thr Ala Asp Glu Trp Asn
    130                 135                 140
Ser Asn His Thr Ile Glu Ile Glu Ile Asn Gln Thr Leu Glu Ile Pro
145                 150                 155                 160
Cys Phe Gln Asn Ser Ser Ser Lys Ile Ser Ser Glu Phe Thr Tyr Ala
                165                 170                 175
Trp Ser Val Glu Asp Asn Gly Thr Gln Glu Thr Leu Ile Ser Gln Asn
            180                 185                 190
His Leu Ile Ser Asn Ser Thr Leu Leu Lys Asp Arg Val Lys Leu Gly
        195                 200                 205
Thr Asp Tyr Arg Leu His Leu Ser Pro Val Gln Ile Phe Asp Asp Gly
    210                 215                 220
Arg Lys Phe Ser Cys His Ile Arg Val Gly Pro Asn Lys Ile Leu Arg
225                 230                 235                 240
Ser Ser Thr Thr Val Lys Val Phe Ala Lys Pro Glu Ile Pro Val Ile
                245                 250                 255
Val Glu Asn Asn Ser Thr Asp Val Leu Val Glu Arg Arg Phe Thr Cys
            260                 265                 270
Leu Leu Lys Asn Val Phe Pro Lys Ala Asn Ile Thr Trp Phe Ile Asp
        275                 280                 285

Gly Ser Phe Leu His Asp Glu Lys Glu Gly Ile Tyr Ile Thr Asn Glu
    290                 295                 300
Glu Arg Lys Gly Lys Asp Gly Phe Leu Glu Leu Lys Ser Val Leu Thr
305                 310                 315                 320
Arg Val His Ser Asn Lys Pro Ala Gln Ser Asp Asn Leu Thr Ile Trp
                325                 330                 335
Cys Met Ala Leu Ser Pro Val Pro Gly Asn Lys Val Trp Asn Ile Ser
            340                 345                 350
Ser Glu Lys Ile Thr Phe Leu Leu Gly Ser Glu Ile Ser Ser Thr Asp
        355                 360                 365
Pro Pro Leu Ser Val Thr Glu Ser Thr Leu Asp Thr Gln Pro Ser Pro
    370                 375                 380
Ala Ser Ser Val Ser Pro Ala Arg Tyr Pro Ala Thr Ser Ser Val Thr
385                 390                 395                 400
Leu Val Asp Val Ser Ala Leu Arg Pro Asn Thr Thr Pro Gln Pro Ser
                405                 410                 415
Asn Ser Ser Met Thr Thr Arg Gly Phe Asn Tyr Pro Trp Thr Ser Ser
            420                 425                 430
Gly Thr Asp Thr Lys Lys Ser Val Ser Arg Ile Pro Ser Glu Thr Tyr
        435                 440                 445
Ser Ser Ser Pro Ser Gly Ala Gly Ser Thr Leu His Asp Asn Val Phe
    450                 455                 460
Thr Ser Thr Ala Arg Ala Phe Ser Glu Val Pro Thr Thr Ala Asn Gly
465                 470                 475                 480
Ser Thr Lys Thr Asn His Val His Ile Thr Gly Ile Val Val Asn Lys
                485                 490                 495
Pro Lys Asp Gly Met Glu Pro Lys Ser Ser Asp Lys Thr His Thr Cys
            500                 505                 510
Pro Pro Cys Pro Ala Pro Glu Leu Leu Gly Gly Pro Ser Val Phe Leu
        515                 520                 525
Phe Pro Pro Lys Pro Lys Asp Thr Leu Met Ile Ser Arg Thr Pro Glu
    530                 535                 540
Val Thr Cys Val Val Val Asp Val Ser His Glu Asp Pro Glu Val Lys
545                 550                 555                 560
Phe Asn Trp Tyr Val Asp Gly Val Glu Val His Asn Ala Lys Thr Lys
                565                 570                 575
Pro Arg Glu Glu Gln Tyr Asn Ser Thr Tyr Arg Val Val Ser Val Leu
            580                 585                 590
Thr Val Leu His Gln Asp Trp Leu Asn Gly Lys Glu Tyr Lys Cys Lys
        595                 600                 605
Val Ser Asn Lys Ala Leu Pro Ala Pro Ile Glu Lys Thr Ile Ser Lys
    610                 615                 620
Ala Lys Gly Gln Pro Arg Glu Pro Gln Val Tyr Thr Leu Pro Pro Ser
625                 630                 635                 640
Arg Asp Glu Leu Thr Lys Asn Gln Val Ser Leu Thr Cys Leu Val Lys
                645                 650                 655
Gly Phe Tyr Pro Ser Asp Ile Ala Val Glu Trp Glu Ser Asn Gly Gln
            660                 665                 670
Pro Glu Asn Asn Tyr Lys Thr Thr Pro Pro Val Leu Asp Ser Asp Gly
        675                 680                 685
Ser Phe Phe Leu Tyr Ser Lys Leu Thr Val Asp Lys Ser Arg Trp Gln
    690                 695                 700
Gln Gly Asn Val Phe Ser Cys Ser Val Met His Glu Ala Leu His Asn
705                 710                 715                 720
His Tyr Thr Gln Lys Ser Leu Ser Leu Ser Pro Gly Lys
                725                 730

<210>  6

<211>  119
<212>  PRT
<213>  小鼠(Mus musculus)

<220>
<221>  DOMAIN
<223>  m1718 VH

<400>  6
Glu Phe Gln Leu Gln Gln Ser Gly Pro Glu Leu Val Lys Pro Gly Ala
1               5                   10                  15
Ser Val Lys Ile Ser Cys Lys Ala Ser Ala Tyr Ser Ile Thr Asp Tyr
            20                  25                  30
Asn Met Asn Trp Val Lys Gln Ser Asn Gly Lys Ser Leu Glu Trp Ile
        35                  40                  45
Gly Val Ile Asn Pro Ser Tyr Gly Ile Thr Asp Tyr Asn Gln Asn Phe
    50                  55                  60
Lys Asp Lys Ala Thr Leu Thr Val Asp Gln Ser Ser Ser Thr Ala Tyr
65                  70                  75                  80
Met Gln Leu Asn Ser Leu Thr Ser Glu Asp Ser Ala Val Tyr Tyr Cys
                85                  90                  95
Ala Ile Gln Leu Arg Leu Pro Gly Tyr Phe Asp Val Trp Gly Thr Gly
            100                 105                 110
Thr Thr Val Ile Val Ser Ser
        115

<210>  7
<211>  107
<212>  PRT
<213>  小鼠(Mus musculus)

<220>
<221>  DOMAIN
<223>  m1718 VL

<400>  7
Asp Ile Lys Met Thr Gln Ser Pro Ser Ser Met Tyr Ala Ser Leu Gly
1               5                   10                  15
Glu Arg Val Thr Ile Thr Cys Lys Ala Ser Gln Asp Ile Asn Asn Tyr
            20                  25                  30
Leu Asn Trp Phe Gln Gln Lys Pro Gly Lys Ser Pro Lys Thr Leu Ile
        35                  40                  45
Tyr Arg Ala Asn Arg Leu Val Asp Gly Val Pro Ser Arg Phe Ser Gly
    50                  55                  60
Ser Gly Ser Gly Gln Asp Tyr Ser Leu Thr Ile Ser Ser Leu Glu Tyr
65                  70                  75                  80
Glu Asp Met Gly Ile Tyr Tyr Cys Leu Lys Tyr Asp Glu Phe Pro Tyr
                85                  90                  95
Thr Phe Gly Gly Gly Thr Lys Val Glu Ile Lys
            100                 105

<210>  8
<211>  122
<212>  PRT
<213>  小鼠(Mus musculus)

<220>
<221>  DOMAIN
<223>  m1719VH

<400>  8
Gln Val Gln Leu Lys Glu Ser Gly Pro Gly Leu Val Ala Pro Ser Gln
1               5                   10                  15
Ser Leu Ser Ile Thr Cys Thr Val Ser Gly Phe Ser Leu Ile Ser Tyr
            20                  25                  30
Gly Val Ser Trp Val Arg Gln Pro Pro Gly Lys Gly Leu Glu Trp Leu
        35                  40                  45
Gly Val Ile Trp Gly Asp Gly Asn Thr Asn Tyr His Ser Val Leu Ile
    50                  55                  60
Ser Arg Leu Ser Ile Arg Lys Asp Asp Ser Lys Ser Gln Val Phe Leu
65                  70                  75                  80
Lys Leu Asn Ser Leu Gln Thr Asp Asp Thr Ala Thr Tyr Tyr Cys Ala
                85                  90                  95
Lys Asn Asn Tyr Tyr Gly Ser Arg Tyr Gly Tyr Pro Met Asp Tyr Trp
            100                 105                 110
Gly Gln Gly Thr Ser Val Thr Val Ser Ser
        115                 120

<210>  9
<211>  107
<212>  PRT
<213>  小鼠(Mus musculus)

<220>
<221>  DOMAIN
<223>  m1719VL

<400>  9
Asp Ile Val Met Thr Gln Ser His Lys Phe Met Ser Thr Ser Val Gly
1               5                   10                  15
Asp Arg Val Ser Ile Thr Cys Lys Ala Thr Gln Asp Val Gly Thr Ala
            20                  25                  30
Val Ala Trp Tyr Gln Gln Lys Pro Gly His Ser Pro Lys Leu Leu Ile
        35                  40                  45
Tyr Trp Ala Ser Thr Arg His Thr Gly Val Pro Glu Arg Phe Thr Gly
    50                  55                  60
Ser Gly Ser Gly Thr Asp Phe Thr Leu Thr Ile Asp Asn Val Gln Ser
65                  70                  75                  80
Glu Asp Leu Ala Asp Tyr Phe Cys Gln Gln Tyr Gly Ser Ser Val Leu
                85                  90                  95
Thr Phe Gly Ala Gly Thr Lys Val Glu Leu Arg
            100                 105

<210>  10
<211>  119
<212>  PRT
<213>  小鼠(Mus musculus)

<220>
<221>  DOMAIN
<223>  m1720VH

<400>  10
Glu Phe Gln Leu Gln Gln Ser Gly Pro Glu Leu Val Lys Pro Gly Ala
1               5                   10                  15
Ser Val Lys Ile Ser Cys Lys Ala Ser Ala Tyr Ser Leu Thr Asp Tyr
            20                  25                  30
Asn Met Asn Trp Val Lys Gln Ser Asn Gly Lys Ser Leu Glu Trp Ile
        35                  40                  45
Gly Val Ile Asn Pro Ser Tyr Gly Ile Ser Ser Tyr Asn Gln Lys Phe
    50                  55                  60
Lys Gly Lys Ala Thr Leu Thr Val Asp Gln Ser Ser Ser Thr Ala Tyr
65                  70                  75                  80
Met His Leu Asn Ser Leu Thr Ser Glu Asp Ser Ala Val Tyr Tyr Cys
                85                  90                  95
Ala Arg Gln Leu Arg Leu Pro Gly Tyr Phe Asp Val Trp Gly Thr Gly
            100                 105                 110
Thr Thr Val Thr Val Ser Ser
        115

<210>  11
<211>  107
<212>  PRT
<213>  小鼠(Mus musculus)

<220>
<221>  DOMAIN
<223>  m1720VL


<400>  11
Asp Ile Lys Met Thr Gln Ser Pro Ser Ser Lys Asn Ala Ser Leu Gly
1               5                   10                  15
Glu Arg Val Thr Ile Thr Cys Lys Ala Ser Gln Asp Ile Asn Ser Tyr
            20                  25                  30
Leu Asn Trp Phe Gln Gln Lys Pro Gly Lys Ser Pro Lys Thr Leu Ile
        35                  40                  45
Tyr Arg Ala Ser Arg Leu Val Asp Gly Val Pro Ser Arg Phe Ser Gly
    50                  55                  60
Ser Gly Ser Gly Gln Asp Tyr Ser Leu Thr Ile Ser Ser Leu Glu Tyr
65                  70                  75                  80
Glu Asp Met Gly Ile Tyr Tyr Cys Leu Lys Tyr Asp Glu Phe Pro Tyr
                85                  90                  95
Thr Phe Gly Gly Gly Thr Lys Leu Glu Ile Lys
            100                 105

<210>  12
<211>  122
<212>  PRT
<213>  小鼠(Mus musculus)

<220>
<221>  DOMAIN
<223>  m1721VH


<400>  12
Gln Val Gln Leu Lys Glu Ser Gly Pro Gly Leu Val Ala Pro Ser Gln
1               5                   10                  15

Ser Leu Ser Ile Thr Cys Thr Val Ser Gly Phe Ser Leu Ile Ser Tyr
            20                  25                  30
Gly Val Asn Trp Val Arg Gln Pro Pro Gly Lys Gly Leu Glu Trp Leu
        35                  40                  45
Gly Val Ile Trp Gly Asp Gly Ser Thr Asn Tyr His Ser Ala Leu Ile
    50                  55                  60
Ser Arg Leu Ser Ile Ser Lys Asp Asp Ser Lys Ser Gln Val Phe Leu
65                  70                  75                  80
Asn Leu Asn Ser Leu Gln Thr Asp Asp Thr Ala Thr Tyr Tyr Cys Ala
                85                  90                  95
Lys Asn Tyr Phe Tyr Gly Ser Arg Tyr Gly Tyr Ala Met Asp Ser Trp
            100                 105                 110
Gly Gln Gly Ile Ser Val Thr Val Ser Ser
        115                 120

<210>  13
<211>  107
<212>  PRT
<213>  小鼠(Mus musculus)

<220>
<221>  DOMAIN
<223>  m1721VL


<400>  13
Asp Ile Gln Met Thr Gln Ser Pro Ala Ser Leu Ser Ala Ala Val Gly
1               5                   10                  15
Glu Thr Val Thr Ile Thr Cys Arg Ala Ser Glu Asn Ile Tyr Ser Ser
            20                  25                  30
Leu Ala Trp Tyr Gln Gln Lys Gln Gly Lys Ser Pro Gln Leu Leu Val
        35                  40                  45
Tyr Asn Ala Lys Thr Leu Ile Glu Thr Val Ala Ser Arg Phe Ser Gly
    50                  55                  60
Ser Gly Ser Gly Thr Gln Tyr Ser Leu Lys Ile Asn Ser Leu Gln Pro
65                  70                  75                  80
Glu Asp Phe Gly Ser Phe Tyr Cys Gln His His Tyr Gly Thr Pro Tyr
                85                  90                  95
Thr Phe Gly Gly Gly Thr Lys Leu Glu Ile Lys
            100                 105

<210>  14
<211>  117
<212>  PRT
<213>  小鼠(Mus musculus)

<220>
<221>  DOMAIN
<223>  m1722VH


<400>  14
Gln Val Gln Leu Gln Gln Pro Gly Ala Glu Leu Val Lys Pro Gly Thr
1               5                   10                  15
Ser Val Lys Leu Ser Cys Lys Ala Ser Gly Asn Thr Phe Ile Asp Tyr
            20                  25                  30
Trp Met His Trp Val Lys Gln Arg Pro Gly Gln Gly Leu Glu Trp Ile
        35                  40                  45

Gly Met Leu His Pro Asn Ser Gly Thr Thr Ser Phe Asn Glu Lys Phe
    50                  55                  60
Lys Ile Lys Thr Thr Leu Thr Ile Asp Lys Ser Ser Ser Thr Ala Tyr
65                  70                  75                  80
Met Gln Leu Ser Ser Leu Thr Ser Glu Asp Ser Ala Ile Tyr Tyr Cys
                85                  90                  95
Ala Ser Asp Tyr Ser Gly Pro Phe Ala Tyr Trp Gly Gln Gly Thr Leu
            100                 105                 110
Val Thr Val Ser Ala
        115

<210>  15
<211>  106
<212>  PRT
<213>  小鼠(Mus musculus)

<220>
<221>  DOMAIN
<223>  m1722VL

<400>  15
Gln Ile Val Leu Thr Gln Ser Pro Gly Ile Met Ser Ala Phe Pro Gly
1               5                   10                  15
Glu Lys Val Thr Met Thr Cys Ser Ala Ser Ser Ser Val Asn Tyr Ile
            20                  25                  30
His Trp Tyr Gln Gln Lys Ser Gly Thr Ser Pro Lys Arg Trp Ile Phe
        35                  40                  45
Asp Thr Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys Gln Gln Trp Arg Ser Asn Pro Leu Thr
                85                  90                  95
Phe Gly Ala Gly Thr Lys Leu Glu Leu Lys
            100                 105

<210>  16
<211>  5
<212>  PRT
<213>  小鼠(Mus musculus)

<220>
<221>  DOMAIN
<223>  m1718 HCDR1

<400>  16
Asp Tyr Asn Met Asn
1               5

<210>  17
<211>  17
<212>  PRT
<213>  小鼠(Mus musculus)

<220>
<221>  DOMAIN

<223>  m1718 HCDR2

<400>  17
Val Ile Asn Pro Ser Tyr Gly Ile Thr Asp Tyr Asn Gln Asn Phe Lys
1               5                   10                  15
Asp

<210>  18
<211>  10
<212>  PRT
<213>  小鼠(Mus musculus)

<220>
<221>  DOMAIN
<223>  m1718 HCDR3

<400>  18
Gln Leu Arg Leu Pro Gly Tyr Phe Asp Val
1               5                   10

<210>  19
<211>  11
<212>  PRT
<213>  小鼠(Mus musculus)

<220>
<221>  DOMAIN
<223>  m1718 LCDR1

<400>  19
Lys Ala Ser Gln Asp Ile Asn Asn Tyr Leu Asn
1               5                   10

<210>  20
<211>  7
<212>  PRT
<213>  小鼠(Mus musculus)

<220>
<221>  DOMAIN
<223>  m1718 LCDR2

<400>  20
Arg Ala Asn Arg Leu Val Asp
1               5

<210>  21
<211>  10
<212>  PRT
<213>  小鼠(Mus musculus)

<220>
<221>  DOMAIN

<223>  m1718 LCDR3

<400>  21
Leu Lys Tyr Asp Glu Phe Pro Tyr Thr Phe
1               5                   10

<210>  22
<211>  5
<212>  PRT
<213>  小鼠(Mus musculus)

<220>
<221>  DOMAIN
<223>  m1719 HCDR1

<400>  22
Ser Tyr Gly Val Ser
1               5

<210>  23
<211>  16
<212>  PRT
<213>  小鼠(Mus musculus)

<220>
<221>  DOMAIN
<223>  m1719 HCDR2

<400>  23
Val Ile Trp Gly Asp Gly Asn Thr Asn Tyr His Ser Val Leu Ile Ser
1               5                   10                  15

<210>  24
<211>  14
<212>  PRT
<213>  小鼠(Mus musculus)

<220>
<221>  DOMAIN
<223>  m1719 HCDR3

<400>  24
Asn Asn Tyr Tyr Gly Ser Arg Tyr Gly Tyr Pro Met Asp Tyr
1               5                   10

<210>  25
<211>  11
<212>  PRT
<213>  小鼠(Mus musculus)

<220>
<221>  DOMAIN
<223>  m1719 LCDR1

<400>  25
Lys Ala Thr Gln Asp Val Gly Thr Ala Val Ala
1               5                   10

<210>  26
<211>  7
<212>  PRT
<213>  小鼠(Mus musculus)

<220>
<221>  DOMAIN
<223>  m1719 LCDR2

<400>  26
Trp Ala Ser Thr Arg His Thr
1               5

<210>  27
<211>  9
<212>  PRT
<213>  小鼠(Mus musculus)

<220>
<221>  DOMAIN
<223>  m1719 LCDR3

<400>  27
Gln Gln Tyr Gly Ser Ser Val Leu Thr
1               5

<210>  28
<211>  5
<212>  PRT
<213>  小鼠(Mus musculus)

<220>
<221>  DOMAIN
<223>  m1720 HCDR1

<400>  28
Asp Tyr Asn Met Asn
1               5

<210>  29
<211>  17
<212>  PRT
<213>  小鼠(Mus musculus)

<220>
<221>  DOMAIN
<223>  m1720 HCDR2

<400>  29

Val Ile Asn Pro Ser Tyr Gly Ile Ser Ser Tyr Asn Gln Lys Phe Lys
1               5                   10                  15
Gly

<210>  30
<211>  10
<212>  PRT
<213>  小鼠(Mus musculus)

<220>
<221>  DOMAIN
<223>  m1720 HCDR3

<400>  30
Gln Leu Arg Leu Pro Gly Tyr Phe Asp Val
1               5                   10

<210>  31
<211>  11
<212>  PRT
<213>  小鼠(Mus musculus)

<220>
<221>  DOMAIN
<223>  m1720 LCDR1

<400>  31
Lys Ala Ser Gln Asp Ile Asn Ser Tyr Leu Asn
1               5                   10

<210>  32
<211>  7
<212>  PRT
<213>  小鼠(Mus musculus)

<220>
<221>  DOMAIN
<223>  m1720 LCDR2

<400>  32
Arg Ala Ser Arg Leu Val Asp
1               5

<210>  33
<211>  10
<212>  PRT
<213>  小鼠(Mus musculus)

<220>
<221>  DOMAIN
<223>  m1720 LCDR3

<400>  33

Leu Lys Tyr Asp Glu Phe Pro Tyr Thr Phe
1               5                   10

<210>  34
<211>  5
<212>  PRT
<213>  小鼠(Mus musculus)

<220>
<221>  DOMAIN
<223>  m1721 HCDR1


<400>  34
Ser Tyr Gly Val Asn
1               5

<210>  35
<211>  16
<212>  PRT
<213>  小鼠(Mus musculus)

<220>
<221>  DOMAIN
<223>  m1721 HCDR2


<400>  35
Val Ile Trp Gly Asp Gly Ser Thr Asn Tyr His Ser Ala Leu Ile Ser
1               5                   10                  15

<210>  36
<211>  14
<212>  PRT
<213>  小鼠(Mus musculus)

<220>
<221>  DOMAIN
<223>  m1721 HCDR3


<400>  36
Asn Tyr Phe Tyr Gly Ser Arg Tyr Gly Tyr Ala Met Asp Ser
1               5                   10

<210>  37
<211>  11
<212>  PRT
<213>  小鼠(Mus musculus)

<220>
<221>  DOMAIN
<223>  m1721 LCDR1


<400>  37
Arg Ala Ser Glu Asn Ile Tyr Ser Ser Leu Ala
1               5                   10

<210>  38
<211>  7
<212>  PRT
<213>  小鼠(Mus musculus)

<220>
<221>  DOMAIN
<223>  m1721 LCDR2


<400>  38
Asn Ala Lys Thr Leu Ile Glu
1               5

<210>  39
<211>  9
<212>  PRT
<213>  小鼠(Mus musculus)

<220>
<221>  DOMAIN
<223>  m1721 LCDR3


<400>  39
Gln His His Tyr Gly Thr Pro Tyr Thr
1               5

<210>  40
<211>  5
<212>  PRT
<213>  小鼠(Mus musculus)

<220>
<221>  DOMAIN
<223>  m1722 HCDR1


<400>  40
Asp Tyr Trp Met His
1               5

<210>  41
<211>  17
<212>  PRT
<213>  小鼠(Mus musculus)

<220>
<221>  DOMAIN
<223>  m1722 HCDR2


<400>  41
Met Leu His Pro Asn Ser Gly Thr Thr Ser Phe Asn Glu Lys Phe Lys
1               5                   10                  15
Ile

<210>  42
<211>  8
<212>  PRT
<213>  小鼠(Mus musculus)

<220>
<221>  DOMAIN
<223>  m1722 HCDR3

<400>  42
Asp Tyr Ser Gly Pro Phe Ala Tyr
1               5

<210>  43
<211>  10
<212>  PRT
<213>  小鼠(Mus musculus)

<220>
<221>  DOMAIN
<223>  m1722 LCDR1

<400>  43
Ser Ala Ser Ser Ser Val Asn Tyr Ile His
1               5                   10

<210>  44
<211>  7
<212>  PRT
<213>  小鼠(Mus musculus)

<220>
<221>  DOMAIN
<223>  m1722 LCDR2

<400>  44
Asp Thr Ser Lys Leu Ala Ser
1               5

<210>  45
<211>  9
<212>  PRT
<213>  小鼠(Mus musculus)

<220>
<221>  DOMAIN
<223>  m1722 LCDR3

<400>  45
Gln Gln Trp Arg Ser Asn Pro Leu Thr
1               5

<210>  46

<211>  119
<212>  PRT
<213>  人工序列(Artificial Sequence)

<220>
<221>  DOMAIN
<223>  h1718-001VH-CDR graft


<400>  46
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Asn Met Asn Trp Val Arg Gln Ala Pro Gly Gln Arg Leu Glu Trp Met
        35                  40                  45
Gly Val Ile Asn Pro Ser Tyr Gly Ile Thr Asp Tyr Asn Gln Asn Phe
    50                  55                  60
Lys Asp Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gln Leu Arg Leu Pro Gly Tyr Phe Asp Val Trp Gly Gln Gly
            100                 105                 110
Thr Thr Val Thr Val Ser Ser
        115

<210>  47
<211>  107
<212>  PRT
<213>  人工序列(Artificial Sequence)

<220>
<221>  DOMAIN
<223>  h1718-001VL-CDR graft


<400>  47
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Asn Asn Tyr
            20                  25                  30
Leu Asn Trp Phe Gln Gln Lys Pro Gly Lys Ala Pro Lys Ser Leu Ile
        35                  40                  45
Tyr Arg Ala Asn Arg Leu Val Asp Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Lys Tyr Asp Glu Phe Pro Tyr
                85                  90                  95
Thr Phe Gly Gly Gly Thr Lys Val Glu Ile Lys
            100                 105

<210>  48
<211>  107
<212>  PRT
<213>  人工序列(Artificial Sequence)

<220>
<221>  DOMAIN
<223>  h1718-VL2

<400>  48
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Asn Asn Tyr
            20                  25                  30
Leu Asn Trp Phe Gln Gln Lys Pro Gly Lys Ala Pro Lys Thr Leu Ile
        35                  40                  45
Tyr Arg Ala Asn Arg Leu Val Asp Gly Val Pro Ser Arg Phe Ser Gly
    50                  55                  60
Ser Gly Ser Gly Gln Asp Tyr Thr Leu Thr Ile Ser Ser Leu Gln Pro
65                  70                  75                  80
Glu Asp Phe Ala Thr Tyr Tyr Cys Leu Lys Tyr Asp Glu Phe Pro Tyr
                85                  90                  95
Thr Phe Gly Gly Gly Thr Lys Val Glu Ile Lys
            100                 105

<210>  49
<211>  119
<212>  PRT
<213>  人工序列(Artificial Sequence)

<220>
<221>  DOMAIN
<223>  h1718-VH2

<400>  49
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Asn Met Asn Trp Val Arg Gln Ala Pro Gly Gln Arg Leu Glu Trp Ile
        35                  40                  45
Gly Val Ile Asn Pro Ser Tyr Gly Ile Thr Asp Tyr Asn Gln Asn Phe
    50                  55                  60
Lys Asp Arg Val Thr Ile Thr Val Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gln Leu Arg Leu Pro Gly Tyr Phe Asp Val Trp Gly Gln Gly
            100                 105                 110
Thr Thr Val Thr Val Ser Ser
        115

<210>  50
<211>  119
<212>  PRT
<213>  人工序列(Artificial Sequence)

<220>
<221>  DOMAIN
<223>  h1718-VH3

<400>  50
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Ile Thr Asp Tyr
            20                  25                  30
Asn Met Asn Trp Val Arg Gln Ala Pro Gly Gln Arg Leu Glu Trp Ile
        35                  40                  45
Gly Val Ile Asn Pro Ser Tyr Gly Ile Thr Asp Tyr Asn Gln Asn Phe
    50                  55                  60
Lys Asp Arg Val Thr Ile Thr Val Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Ile Gln Leu Arg Leu Pro Gly Tyr Phe Asp Val Trp Gly Gln Gly
            100                 105                 110
Thr Thr Val Thr Val Ser Ser
        115

<210>  51
<211>  119
<212>  PRT
<213>  人工序列(Artificial Sequence)

<220>
<221>  DOMAIN
<223>  h1718-VH4


<400>  51
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Ile Thr Asp Tyr
            20                  25                  30
Asn Met Asn Trp Val Lys Gln Ala Pro Gly Gln Arg Leu Glu Trp Ile
        35                  40                  45
Gly Val Ile Asn Pro Ser Tyr Gly Ile Thr Asp Tyr Asn Gln Asn Phe
    50                  55                  60
Lys Asp Lys Val Thr Ile Thr Val Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Ile Gln Leu Arg Leu Pro Gly Tyr Phe Asp Val Trp Gly Gln Gly
            100                 105                 110
Thr Thr Val Thr Val Ser Ser
        115

<210>  52
<211>  11
<212>  PRT
<213>  人工序列(Artificial Sequence)

<220>
<221>  DOMAIN
<223>  h1718-VL CDR1 序列通式


<220>

<221>  UNSURE
<222>  (8)..(8)
<223>  The 'Xaa' at location 8 stands for Asn, Thr, Leu or Gln.

<400>  52
Lys Ala Ser Gln Asp Ile Asn Xaa Tyr Leu Asn
1               5                   10

<210>  53
<211>  107
<212>  PRT
<213>  人工序列(Artificial Sequence)

<220>
<221>  DOMAIN
<223>  h1718.VL1a

<400>  53
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Asn Gln Tyr
            20                  25                  30
Leu Asn Trp Phe Gln Gln Lys Pro Gly Lys Ala Pro Lys Ser Leu Ile
        35                  40                  45
Tyr Arg Ala Asn Arg Leu Val Asp Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Lys Tyr Asp Glu Phe Pro Tyr
                85                  90                  95
Thr Phe Gly Gly Gly Thr Lys Val Glu Ile Lys
            100                 105

<210>  54
<211>  107
<212>  PRT
<213>  人工序列(Artificial Sequence)

<220>
<221>  DOMAIN
<223>  h1718.VL1b

<400>  54
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Asn Thr Tyr
            20                  25                  30
Leu Asn Trp Phe Gln Gln Lys Pro Gly Lys Ala Pro Lys Ser Leu Ile
        35                  40                  45
Tyr Arg Ala Asn Arg Leu Val Asp Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Lys Tyr Asp Glu Phe Pro Tyr
                85                  90                  95

Thr Phe Gly Gly Gly Thr Lys Val Glu Ile Lys
            100                 105

<210>  55
<211>  107
<212>  PRT
<213>  人工序列(Artificial Sequence)

<220>
<221>  DOMAIN
<223>  h1718.VL1c


<400>  55
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Asn Leu Tyr
            20                  25                  30
Leu Asn Trp Phe Gln Gln Lys Pro Gly Lys Ala Pro Lys Ser Leu Ile
        35                  40                  45
Tyr Arg Ala Asn Arg Leu Val Asp Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Lys Tyr Asp Glu Phe Pro Tyr
                85                  90                  95
Thr Phe Gly Gly Gly Thr Lys Val Glu Ile Lys
            100                 105

<210>  56
<211>  107
<212>  PRT
<213>  人工序列(Artificial Sequence)

<220>
<221>  DOMAIN
<223>  h1718.VL2a


<400>  56
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Asn Gln Tyr
            20                  25                  30
Leu Asn Trp Phe Gln Gln Lys Pro Gly Lys Ala Pro Lys Thr Leu Ile
        35                  40                  45
Tyr Arg Ala Asn Arg Leu Val Asp Gly Val Pro Ser Arg Phe Ser Gly
    50                  55                  60
Ser Gly Ser Gly Gln Asp Tyr Thr Leu Thr Ile Ser Ser Leu Gln Pro
65                  70                  75                  80
Glu Asp Phe Ala Thr Tyr Tyr Cys Leu Lys Tyr Asp Glu Phe Pro Tyr
                85                  90                  95
Thr Phe Gly Gly Gly Thr Lys Val Glu Ile Lys
            100                 105

<210>  57
<211>  107
<212>  PRT

<213>  人工序列(Artificial Sequence)

<220>
<221>  DOMAIN
<223>  h1718.VL2b


<400>  57
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Asn Thr Tyr
            20                  25                  30
Leu Asn Trp Phe Gln Gln Lys Pro Gly Lys Ala Pro Lys Thr Leu Ile
        35                  40                  45
Tyr Arg Ala Asn Arg Leu Val Asp Gly Val Pro Ser Arg Phe Ser Gly
    50                  55                  60
Ser Gly Ser Gly Gln Asp Tyr Thr Leu Thr Ile Ser Ser Leu Gln Pro
65                  70                  75                  80
Glu Asp Phe Ala Thr Tyr Tyr Cys Leu Lys Tyr Asp Glu Phe Pro Tyr
                85                  90                  95
Thr Phe Gly Gly Gly Thr Lys Val Glu Ile Lys
            100                 105

<210>  58
<211>  107
<212>  PRT
<213>  人工序列(Artificial Sequence)

<220>
<221>  DOMAIN
<223>  h1718.VL2c


<400>  58
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Ile Asn Leu Tyr
            20                  25                  30
Leu Asn Trp Phe Gln Gln Lys Pro Gly Lys Ala Pro Lys Thr Leu Ile
        35                  40                  45
Tyr Arg Ala Asn Arg Leu Val Asp Gly Val Pro Ser Arg Phe Ser Gly
    50                  55                  60
Ser Gly Ser Gly Gln Asp Tyr Thr Leu Thr Ile Ser Ser Leu Gln Pro
65                  70                  75                  80
Glu Asp Phe Ala Thr Tyr Tyr Cys Leu Lys Tyr Asp Glu Phe Pro Tyr
                85                  90                  95
Thr Phe Gly Gly Gly Thr Lys Val Glu Ile Lys
            100                 105

<210>  59
<211>  122
<212>  PRT
<213>  人工序列(Artificial Sequence)

<220>
<221>  DOMAIN
<223>  HRP00721VH-CDR graft

<400>  59
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Ser Trp Ile Arg Gln Pro Pro Gly Lys Ala Leu Glu Trp Leu
        35                  40                  45
Ala Val Ile Trp Gly Asp Gly Asn Thr Asn Tyr His Ser Val Leu Ile
    50                  55                  60
Ser Arg Leu Thr Ile Ser Lys Asp Thr Ser Lys Ser Gln Val Val Leu
65                  70                  75                  80
Thr Met Thr Asn Met Asp Pro Val Asp Thr Ala Thr Tyr Tyr Cys Ala
                85                  90                  95
Arg Asn Asn Tyr Tyr Gly Ser Arg Tyr Gly Tyr Pro Met Asp Tyr Trp
            100                 105                 110
Gly Gln Gly Thr Thr Val Thr Val Ser Ser
        115                 120

<210>  60
<211>  107
<212>  PRT
<213>  人工序列(Artificial Sequence)

<220>
<221>  DOMAIN
<223>  HRP00721VL-CDR graft


<400>  60
Asp Ile Gln Met Thr Gln Ser Pro Ser Ser Val Ser Ala Ser Val Gly
1               5                   10                  15
Asp Arg Val Thr Ile Thr Cys Lys Ala Thr Gln Asp Val Gly Thr Ala
            20                  25                  30
Val Ala Trp Tyr Gln Gln Lys Pro Gly Lys Ala Pro Lys Leu Leu Ile
        35                  40                  45
Tyr Trp Ala Ser Thr Arg His Thr Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Gly Ser Ser Val Leu
                85                  90                  95
Thr Phe Gly Gly Gly Thr Lys Val Glu Ile Lys
            100                 105

<210>  61
<211>  107
<212>  PRT
<213>  人工序列(Artificial Sequence)

<220>
<221>  DOMAIN
<223>  h1719-VL2


<400>  61
Asp Ile Gln Met Thr Gln Ser Pro Ser Ser Val Ser Ala Ser Val Gly
1               5                   10                  15

Asp Arg Val Thr Ile Thr Cys Lys Ala Thr Gln Asp Val Gly Thr Ala
            20                  25                  30
Val Ala Trp Tyr Gln Gln Lys Pro Gly Lys Ser Pro Lys Leu Leu Ile
        35                  40                  45
Tyr Trp Ala Ser Thr Arg His Thr Gly Val Pro Glu Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Phe Cys Gln Gln Tyr Gly Ser Ser Val Leu
                85                  90                  95
Thr Phe Gly Gly Gly Thr Lys Val Glu Ile Lys
            100                 105

<210> 62
<211> 122
<212> PRT
<213> 人工序列(Artificial Sequence)

<220>
<221> DOMAIN
<223> h1719-VH2

<400> 62
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Ser Trp Val Arg Gln Pro Pro Gly Lys Ala Leu Glu Trp Leu
        35                  40                  45
Ala Val Ile Trp Gly Asp Gly Asn Thr Asn Tyr His Ser Val Leu Ile
    50                  55                  60
Ser Arg Leu Thr Ile Ser Lys Asp Thr Ser Lys Ser Gln Val Val Leu
65                  70                  75                  80
Thr Met Thr Asn Met Asp Pro Val Asp Thr Ala Thr Tyr Tyr Cys Ala
                85                  90                  95
Lys Asn Asn Tyr Tyr Gly Ser Arg Tyr Gly Tyr Pro Met Asp Tyr Trp
            100                 105                 110
Gly Gln Gly Thr Thr Val Thr Val Ser Ser
        115                 120

<210> 63
<211> 122
<212> PRT
<213> 人工序列(Artificial Sequence)

<220>
<221> DOMAIN
<223> h1719-VH3

<400> 63
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Ser Trp Val Arg Gln Pro Pro Gly Lys Gly Leu Glu Trp Leu
        35                  40                  45

Gly Val Ile Trp Gly Asp Gly Asn Thr Asn Tyr His Ser Val Leu Ile
    50                  55                  60
Ser Arg Leu Thr Ile Ser Lys Asp Thr Ser Lys Ser Gln Val Val Leu
65                  70                  75                  80
Thr Leu Thr Asn Met Asp Pro Val Asp Thr Ala Thr Tyr Tyr Cys Ala
                85                  90                  95
Lys Asn Asn Tyr Tyr Gly Ser Arg Tyr Gly Tyr Pro Met Asp Tyr Trp
            100                 105                 110
Gly Gln Gly Thr Thr Val Thr Val Ser Ser
        115                 120

<210>  64
<211>  14
<212>  PRT
<213>  人工序列(Artificial Sequence)

<220>
<221>  DOMAIN
<223>  h1719-VH CDR3 通式


<220>
<221>  UNSURE
<222>  (1)..(1)
<223>  The 'Xaa' at location 1 stands for Gln or Asn.


<220>
<221>  UNSURE
<222>  (2)..(2)
<223>  The 'Xaa' at location 2 stands for Gln, Asn, or Tyr.


<220>
<221>  UNSURE
<222>  (12)..(12)
<223>  The 'Xaa' at location 12 stands for Met, Leu, or Ile.


<400>  64
Xaa Xaa Tyr Tyr Gly Ser Arg Tyr Gly Tyr Pro Xaa Asp Tyr
1               5                   10

<210>  65
<211>  122
<212>  PRT
<213>  人工序列(Artificial Sequence)

<220>
<221>  DOMAIN
<223>  h1719.VH3a


<400>  65
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30

Gly Val Ser Trp Val Arg Gln Pro Pro Gly Lys Gly Leu Glu Trp Leu
        35                  40                  45
Gly Val Ile Trp Gly Asp Gly Asn Thr Asn Tyr His Ser Val Leu Ile
    50                  55                  60
Ser Arg Leu Thr Ile Ser Lys Asp Thr Ser Lys Ser Gln Val Val Leu
65                  70                  75                  80
Thr Leu Thr Asn Met Asp Pro Val Asp Thr Ala Thr Tyr Tyr Cys Ala
                85                  90                  95
Lys Gln Asn Tyr Tyr Gly Ser Arg Tyr Gly Tyr Pro Leu Asp Tyr Trp
            100                 105                 110
Gly Gln Gly Thr Thr Val Thr Val Ser Ser
        115                 120

<210>  66
<211>  122
<212>  PRT
<213>  人工序列(Artificial Sequence)

<220>
<221>  DOMAIN
<223>  h1719.VH3b


<400>  66
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Ser Trp Val Arg Gln Pro Pro Gly Lys Gly Leu Glu Trp Leu
        35                  40                  45
Gly Val Ile Trp Gly Asp Gly Asn Thr Asn Tyr His Ser Val Leu Ile
    50                  55                  60
Ser Arg Leu Thr Ile Ser Lys Asp Thr Ser Lys Ser Gln Val Val Leu
65                  70                  75                  80
Thr Leu Thr Asn Met Asp Pro Val Asp Thr Ala Thr Tyr Tyr Cys Ala
                85                  90                  95
Lys Asn Tyr Tyr Tyr Gly Ser Arg Tyr Gly Tyr Pro Leu Asp Tyr Trp
            100                 105                 110
Gly Gln Gly Thr Thr Val Thr Val Ser Ser
        115                 120

<210>  67
<211>  122
<212>  PRT
<213>  人工序列(Artificial Sequence)

<220>
<221>  DOMAIN
<223>  h1719.VH3c


<400>  67
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Ser Trp Val Arg Gln Pro Pro Gly Lys Gly Leu Glu Trp Leu
        35                  40                  45

Gly Val Ile Trp Gly Asp Gly Asn Thr Asn Tyr His Ser Val Leu Ile
    50                  55                  60
Ser Arg Leu Thr Ile Ser Lys Asp Thr Ser Lys Ser Gln Val Val Leu
65                  70                  75                  80
Thr Leu Thr Asn Met Asp Pro Val Asp Thr Ala Thr Tyr Tyr Cys Ala
                85                  90                  95
Lys Asn Gln Tyr Tyr Gly Ser Arg Tyr Gly Tyr Pro Leu Asp Tyr Trp
            100                 105                 110
Gly Gln Gly Thr Thr Val Thr Val Ser Ser
        115                 120

<210>  68
<211>  122
<212>  PRT
<213>  人工序列(Artificial Sequence)

<220>
<221>  DOMAIN
<223>  h1719.VH3d


<400>  68
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Ser Trp Val Arg Gln Pro Pro Gly Lys Gly Leu Glu Trp Leu
        35                  40                  45
Gly Val Ile Trp Gly Asp Gly Asn Thr Asn Tyr His Ser Val Leu Ile
    50                  55                  60
Ser Arg Leu Thr Ile Ser Lys Asp Thr Ser Lys Ser Gln Val Val Leu
65                  70                  75                  80
Thr Leu Thr Asn Met Asp Pro Val Asp Thr Ala Thr Tyr Tyr Cys Ala
                85                  90                  95
Lys Gln Asn Tyr Tyr Gly Ser Arg Tyr Gly Tyr Pro Ile Asp Tyr Trp
            100                 105                 110
Gly Gln Gly Thr Thr Val Thr Val Ser Ser
        115                 120

<210>  69
<211>  122
<212>  PRT
<213>  人工序列(Artificial Sequence)

<220>
<221>  DOMAIN
<223>  h1719.VH3e


<400>  69
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Ser Trp Val Arg Gln Pro Pro Gly Lys Gly Leu Glu Trp Leu
        35                  40                  45
Gly Val Ile Trp Gly Asp Gly Asn Thr Asn Tyr His Ser Val Leu Ile
    50                  55                  60

Ser Arg Leu Thr Ile Ser Lys Asp Thr Ser Lys Ser Gln Val Val Leu
65                  70                  75                  80
Thr Leu Thr Asn Met Asp Pro Val Asp Thr Ala Thr Tyr Tyr Cys Ala
                85                  90                  95
Lys Asn Tyr Tyr Tyr Gly Ser Arg Tyr Gly Tyr Pro Ile Asp Tyr Trp
            100                 105                 110
Gly Gln Gly Thr Thr Val Thr Val Ser Ser
        115                 120

<210>  70
<211>  122
<212>  PRT
<213>  人工序列(Artificial Sequence)

<220>
<221>  DOMAIN
<223>  h1719.VH3f


<400>  70
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Ser Trp Val Arg Gln Pro Pro Gly Lys Gly Leu Glu Trp Leu
        35                  40                  45
Gly Val Ile Trp Gly Asp Gly Asn Thr Asn Tyr His Ser Val Leu Ile
    50                  55                  60
Ser Arg Leu Thr Ile Ser Lys Asp Thr Ser Lys Ser Gln Val Val Leu
65                  70                  75                  80
Thr Leu Thr Asn Met Asp Pro Val Asp Thr Ala Thr Tyr Tyr Cys Ala
                85                  90                  95
Lys Asn Gln Tyr Tyr Gly Ser Arg Tyr Gly Tyr Pro Ile Asp Tyr Trp
            100                 105                 110
Gly Gln Gly Thr Thr Val Thr Val Ser Ser
        115                 120

<210>  71
<211>  122
<212>  PRT
<213>  人工序列(Artificial Sequence)

<220>
<221>  DOMAIN
<223>  h1721-001 VH-CDR graft


<400>  71
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Asn Trp Ile Arg Gln Pro Pro Gly Lys Ala Leu Glu Trp Leu
        35                  40                  45
Ala Val Ile Trp Gly Asp Gly Ser Thr Asn Tyr His Ser Ala Leu Ile
    50                  55                  60
Ser Arg Leu Thr Ile Ser Lys Asp Thr Ser Lys Ser Gln Val Val Leu
65                  70                  75                  80

Thr Met Thr Asn Met Asp Pro Val Asp Thr Ala Thr Tyr Tyr Cys Ala
                85                  90                  95
Arg Asn Tyr Phe Tyr Gly Ser Arg Tyr Gly Tyr Ala Met Asp Ser Trp
            100                 105                 110
Gly Gln Gly Thr Thr Val Thr Val Ser Ser
        115                 120

<210>  72
<211>  107
<212>  PRT
<213>  人工序列(Artificial Sequence)

<220>
<221>  DOMAIN
<223>  h1721-001 VL-CDR graft

<400>  72
Asp Ile Gln Met Thr Gln Ser Pro Ser Ser Leu Ser Ala Ser Val Gly
1               5                   10                  15
Asp Arg Val Thr Ile Thr Cys Arg Ala Ser Glu Asn Ile Tyr Ser Ser
            20                  25                  30
Leu Ala Trp Tyr Gln Gln Lys Pro Gly Lys Ala Pro Lys Leu Leu Ile
        35                  40                  45
Tyr Asn Ala Lys Thr Leu Ile Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln His His Tyr Gly Thr Pro Tyr
                85                  90                  95
Thr Phe Gly Gly Gly Thr Lys Val Glu Ile Lys
            100                 105

<210>  73
<211>  107
<212>  PRT
<213>  人工序列(Artificial Sequence)

<220>
<221>  DOMAIN
<223>  h1721-VL2

<400>  73
Asp Ile Gln Met Thr Gln Ser Pro Ser Ser Leu Ser Ala Ser Val Gly
1               5                   10                  15
Asp Arg Val Thr Ile Thr Cys Arg Ala Ser Glu Asn Ile Tyr Ser Ser
            20                  25                  30
Leu Ala Trp Tyr Gln Gln Lys Pro Gly Lys Ala Pro Lys Leu Leu Val
        35                  40                  45
Tyr Asn Ala Lys Thr Leu Ile Glu Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln His His Tyr Gly Thr Pro Tyr
                85                  90                  95
Thr Phe Gly Gly Gly Thr Lys Val Glu Ile Lys
            100                 105

<210>  74
<211>  107
<212>  PRT
<213>  人工序列(Artificial Sequence)

<220>
<221>  DOMAIN
<223>  h1721-VL3


<400>  74
Asp Ile Gln Met Thr Gln Ser Pro Ser Ser Leu Ser Ala Ser Val Gly
1               5                   10                  15
Asp Arg Val Thr Ile Thr Cys Arg Ala Ser Glu Asn Ile Tyr Ser Ser
            20                  25                  30
Leu Ala Trp Tyr Gln Gln Lys Pro Gly Lys Ser Pro Lys Leu Leu Val
        35                  40                  45
Tyr Asn Ala Lys Thr Leu Ile Glu Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln His His Tyr Gly Thr Pro Tyr
                85                  90                  95
Thr Phe Gly Gly Gly Thr Lys Val Glu Ile Lys
            100                 105

<210>  75
<211>  122
<212>  PRT
<213>  人工序列(Artificial Sequence)

<220>
<221>  DOMAIN
<223>  h1721-VH2


<400>  75
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Asn Trp Ile Arg Gln Pro Pro Gly Lys Ala Leu Glu Trp Leu
        35                  40                  45
Gly Val Ile Trp Gly Asp Gly Ser Thr Asn Tyr His Ser Ala Leu Ile
    50                  55                  60
Ser Arg Leu Thr Ile Ser Lys Asp Thr Ser Lys Ser Gln Val Val Leu
65                  70                  75                  80
Thr Met Thr Asn Met Asp Pro Val Asp Thr Ala Thr Tyr Tyr Cys Ala
                85                  90                  95
Lys Asn Tyr Phe Tyr Gly Ser Arg Tyr Gly Tyr Ala Met Asp Ser Trp
            100                 105                 110
Gly Gln Gly Thr Thr Val Thr Val Ser Ser
        115                 120

<210>  76
<211>  122
<212>  PRT

<213>  人工序列(Artificial Sequence)

<220>
<221>  DOMAIN
<223>  h1721-VH3


<400>  76
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Asn Trp Ile Arg Gln Pro Pro Gly Lys Ala Leu Glu Trp Leu
        35                  40                  45
Gly Val Ile Trp Gly Asp Gly Ser Thr Asn Tyr His Ser Ala Leu Ile
    50                  55                  60
Ser Arg Leu Thr Ile Ser Lys Asp Thr Ser Lys Ser Gln Val Val Leu
65                  70                  75                  80
Thr Leu Thr Asn Met Asp Pro Val Asp Thr Ala Thr Tyr Tyr Cys Ala
                85                  90                  95
Lys Asn Tyr Phe Tyr Gly Ser Arg Tyr Gly Tyr Ala Met Asp Ser Trp
            100                 105                 110
Gly Gln Gly Thr Thr Val Thr Val Ser Ser
        115                 120

<210>  77
<211>  122
<212>  PRT
<213>  人工序列(Artificial Sequence)

<220>
<221>  DOMAIN
<223>  h1721-VH4


<400>  77
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Val Asn Trp Val Arg Gln Pro Pro Gly Lys Gly Leu Glu Trp Leu
        35                  40                  45
Gly Val Ile Trp Gly Asp Gly Ser Thr Asn Tyr His Ser Ala Leu Ile
    50                  55                  60
Ser Arg Leu Thr Ile Ser Lys Asp Thr Ser Lys Ser Gln Val Val Leu
65                  70                  75                  80
Thr Leu Thr Asn Met Asp Pro Val Asp Thr Ala Thr Tyr Tyr Cys Ala
                85                  90                  95
Lys Asn Tyr Phe Tyr Gly Ser Arg Tyr Gly Tyr Ala Met Asp Ser Trp
            100                 105                 110
Gly Gln Gly Thr Thr Val Thr Val Ser Ser
        115                 120

<210>  78
<211>  117
<212>  PRT
<213>  人工序列(Artificial Sequence)

<220>
<221>  DOMAIN
<223>  h1722-001 VH-CDR graft

<400>  78
Glu Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Asp Tyr
            20                  25                  30
Trp Met His Trp Val Arg Gln Ala Pro Gly Gln Gly Leu Glu Trp Met
        35                  40                  45
Gly Met Leu His Pro Asn Ser Gly Thr Thr Ser Phe Asn Glu Lys Phe
    50                  55                  60
Lys Ile Arg Val Thr Ile Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Asp Tyr Ser Gly Pro Phe Ala Tyr Trp Gly Gln Gly Thr Leu
            100                 105                 110
Val Thr Val Ser Ser
        115

<210>  79
<211>  106
<212>  PRT
<213>  人工序列(Artificial Sequence)

<220>
<221>  DOMAIN
<223>  h1722-001 VL-CDR graft

<400>  79
Asp Ile Gln Met Thr Gln Ser Pro Ser Ser Leu Ser Ala Ser Val Gly
1               5                   10                  15
Asp Arg Val Thr Ile Thr Cys Ser Ala Ser Ser Ser Val Asn Tyr Ile
            20                  25                  30
His Trp Tyr Gln Gln Lys Pro Gly Lys Ala Pro Lys Leu Leu Ile Tyr
        35                  40                  45
Asp Thr Ser Lys Leu Ala Ser Gly Val Pro Ser Arg Phe Ser Gly Ser
    50                  55                  60
Gly Ser Gly Thr Asp Phe Thr Leu Thr Ile Ser Ser Leu Gln Pro Glu
65                  70                  75                  80
Asp Phe Ala Thr Tyr Tyr Cys Gln Gln Trp Arg Ser Asn Pro Leu Thr
                85                  90                  95
Phe Gly Gln Gly Thr Lys Leu Glu Ile Lys
            100                 105

<210>  80
<211>  106
<212>  PRT
<213>  人工序列(Artificial Sequence)

<220>
<221>  DOMAIN
<223>  h1722-VL2

<400>  80
Asp Ile Gln Met Thr Gln Ser Pro Ser Ser Leu Ser Ala Ser Val Gly
1               5                   10                  15
Asp Arg Val Thr Ile Thr Cys Ser Ala Ser Ser Ser Val Asn Tyr Ile
            20                  25                  30
His Trp Tyr Gln Gln Lys Pro Gly Lys Ala Pro Lys Arg Trp Ile Tyr
        35                  40                  45
Asp Thr Ser Lys Leu Ala Ser Gly Val Pro Ser Arg Phe Ser Gly Ser
    50                  55                  60
Gly Ser Gly Thr Asp Phe Thr Leu Thr Ile Ser Ser Leu Gln Pro Glu
65                  70                  75                  80
Asp Phe Ala Thr Tyr Tyr Cys Gln Gln Trp Arg Ser Asn Pro Leu Thr
                85                  90                  95
Phe Gly Gln Gly Thr Lys Leu Glu Ile Lys
            100                 105

<210>  81
<211>  106
<212>  PRT
<213>  人工序列(Artificial Sequence)

<220>
<221>  DOMAIN
<223>  h1722-VL3


<400>  81
Asp Ile Gln Leu Thr Gln Ser Pro Ser Ser Leu Ser Ala Ser Val Gly
1               5                   10                  15
Asp Arg Val Thr Ile Thr Cys Ser Ala Ser Ser Ser Val Asn Tyr Ile
            20                  25                  30
His Trp Tyr Gln Gln Lys Pro Gly Lys Ala Pro Lys Arg Trp Ile Phe
        35                  40                  45
Asp Thr Ser Lys Leu Ala Ser Gly Val Pro Ser Arg Phe Ser Gly Ser
    50                  55                  60
Gly Ser Gly Thr Asp Tyr Thr Leu Thr Ile Ser Ser Leu Gln Pro Glu
65                  70                  75                  80
Asp Phe Ala Thr Tyr Tyr Cys Gln Gln Trp Arg Ser Asn Pro Leu Thr
                85                  90                  95
Phe Gly Gln Gly Thr Lys Leu Glu Ile Lys
            100                 105

<210>  82
<211>  117
<212>  PRT
<213>  人工序列(Artificial Sequence)

<220>
<221>  DOMAIN
<223>  h1722-VH2


<400>  82
Glu Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Asp Tyr
            20                  25                  30

Trp Met His Trp Val Arg Gln Ala Pro Gly Gln Gly Leu Glu Trp Ile
        35                  40                  45
Gly Met Leu His Pro Asn Ser Gly Thr Thr Ser Phe Asn Glu Lys Phe
    50                  55                  60
Lys Ile Arg Val Thr Ile Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Ser Asp Tyr Ser Gly Pro Phe Ala Tyr Trp Gly Gln Gly Thr Leu
            100                 105                 110
Val Thr Val Ser Ser
        115

<210> 83
<211> 117
<212> PRT
<213> 人工序列(Artificial Sequence)

<220>
<221> DOMAIN
<223> h1722-VH3

<400> 83
Glu Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Asp Tyr
            20                  25                  30
Trp Met His Trp Val Lys Gln Ala Pro Gly Gln Gly Leu Glu Trp Ile
        35                  40                  45
Gly Met Leu His Pro Asn Ser Gly Thr Thr Ser Phe Asn Glu Lys Phe
    50                  55                  60
Lys Ile Arg Val Thr Ile Thr Ile Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Ser Asp Tyr Ser Gly Pro Phe Ala Tyr Trp Gly Gln Gly Thr Leu
            100                 105                 110
Val Thr Val Ser Ser
        115

<210> 84
<211> 117
<212> PRT
<213> 人工序列(Artificial Sequence)

<220>
<221> DOMAIN
<223> h1722-VH4

<400> 84
Glu Val Gln Leu Val Gln Ser Gly Ala Glu Val Lys Lys Pro Gly Ser
1               5                   10                  15
Ser Val Lys Val Ser Cys Lys Ala Ser Gly Asn Thr Phe Ser Asp Tyr
            20                  25                  30
Trp Met His Trp Val Lys Gln Ala Pro Gly Gln Gly Leu Glu Trp Ile
        35                  40                  45

Gly Met Leu His Pro Asn Ser Gly Thr Thr Ser Phe Asn Glu Lys Phe
    50                  55                  60
Lys Ile Arg Thr Thr Ile Thr Ile Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Ser Asp Tyr Ser Gly Pro Phe Ala Tyr Trp Gly Gln Gly Thr Leu
            100                 105                 110
Val Thr Val Ser Ser
        115

<210>  85
<211>  20
<212>  PRT
<213>  人工序列(Artificial Sequence)

<220>
<221>  PEPTIDE
<223>  CD96 胞外区 17-36 位多肽序列

<400>  85
Gly Ser Asp Val Asn Leu Thr Cys Gln Thr Gln Thr Val Gly Phe Phe
1               5                   10                  15
Val Gln Met Gln
            20

<210>  86
<211>  20
<212>  PRT
<213>  人工序列(Artificial Sequence)

<220>
<221>  PEPTIDE
<223>  CD96 胞外区 32-51 位多肽序列

<400>  86
Phe Val Gln Met Gln Trp Ser Lys Val Thr Asn Lys Ile Asp Leu Ile
1               5                   10                  15
Ala Val Tyr His
            20

<210>  87
<211>  20
<212>  PRT
<213>  人工序列(Artificial Sequence)

<220>
<221>  PEPTIDE
<223>  CD96 胞外区 47-66 位多肽序列

<400>  87
Ile Ala Val Tyr His Pro Gln Tyr Gly Phe Tyr Cys Ala Tyr Gly Arg
1               5                   10                  15

Pro Cys Glu Ser
            20

<210>  88
<211>  20
<212>  PRT
<213>  人工序列(Artificial Sequence)

<220>
<221>  PEPTIDE
<223>  CD96 胞外区 62-81 位多肽序列

<400>  88
Arg Pro Cys Glu Ser Leu Val Thr Phe Thr Glu Thr Pro Glu Asn Gly
1               5                   10                  15
Ser Lys Trp Thr
            20

<210>  89
<211>  20
<212>  PRT
<213>  人工序列(Artificial Sequence)

<220>
<221>  PEPTIDE
<223>  CD96 胞外区 77-86 位多肽序列

<400>  89
Gly Ser Lys Trp Thr Leu His Leu Arg Asn Met Ser Cys Ser Val Ser
1               5                   10                  15
Gly Arg Tyr Glu
            20

<210>  90
<211>  20
<212>  PRT
<213>  人工序列(Artificial Sequence)

<220>
<221>  PEPTIDE
<223>  CD96 胞外区 92-111 位多肽序列

<400>  90
Ser Gly Arg Tyr Glu Cys Met Leu Val Leu Tyr Pro Glu Gly Ile Gln
1               5                   10                  15
Thr Lys Ile Tyr
            20

<210>  91
<211>  20
<212>  PRT
<213>  人工序列(Artificial Sequence)

<220>
<221>  PEPTIDE
<223>  CD96 胞外区 107-126 位多肽序列

<400>  91
Gln Thr Lys Ile Tyr Asn Leu Leu Ile Gln Thr His Val Thr Ala Asp
1               5                   10                  15
Glu Trp Asn Ser
            20

<210>  92
<211>  20
<212>  PRT
<213>  人工序列(Artificial Sequence)

<220>
<221>  PEPTIDE
<223>  CD96 胞外区 122-141 位多肽序列

<400>  92
Asp Glu Trp Asn Ser Asn His Thr Ile Glu Ile Glu Ile Asn Gln Thr
1               5                   10                  15
Leu Glu Ile Pro
            20

<210>  93
<211>  20
<212>  PRT
<213>  人工序列(Artificial Sequence)

<220>
<221>  PEPTIDE
<223>  CD96 胞外区 137-156 位多肽序列

<400>  93
Thr Leu Glu Ile Pro Cys Phe Gln Asn Ser Ser Ser Lys Ile Ser Ser
1               5                   10                  15
Glu Phe Thr Tyr
            20

<210>  94
<211>  22
<212>  PRT
<213>  人工序列(Artificial Sequence)

<220>
<221>  PEPTIDE
<223>  CD96 胞外区 149-170 位多肽序列

<400>  94
Lys Ile Ser Ser Glu Phe Thr Tyr Ala Trp Ser Val Glu Asp Asn Gly
1               5                   10                  15

Thr Gln Glu Thr Leu Ile
                20

<210>  95
<211>  523
<212>  PRT
<213>  人工序列(Artificial Sequence)

<220>
<221>  PEPTIDE

<400>  95
Met Glu Phe Gly Leu Ser Trp Leu Phe Leu Val Ala Ile Leu Lys Gly
1               5                   10                  15
Val Gln Cys Val Trp Gly Lys Pro Phe Asn Thr Glu Glu Asn Ile Tyr
            20                  25                  30
Ala Thr Leu Gly Ser Asp Val Asn Leu Thr Cys Gln Thr Gln Ala Lys
        35                  40                  45
Gly Phe Leu Val Gln Met Gln Trp Ser Lys Val Thr Asp Lys Ala Asp
    50                  55                  60
Leu Ile Ala Leu Tyr His Pro Gln Tyr Gly Phe His Cys Ala Tyr Gly
65                  70                  75                  80
Ser Pro Cys Glu Ser Leu Val Thr Phe Thr Gln Thr Pro Glu Asn Gly
                85                  90                  95
Ser Lys Trp Thr Leu His Leu Arg Asn Met Ser Ser Ser Val Ser Gly
            100                 105                 110
Arg Tyr Glu Cys Met Leu Thr Leu Tyr Pro Glu Gly Met Gln Thr Lys
        115                 120                 125
Ile Tyr Asn Leu Leu Ile Gln Thr His Val Thr Pro Asp Glu Trp Lys
    130                 135                 140
Ser Asn His Thr Ile Glu Ile Glu Ile Asn Gln Thr Leu Glu Ile Pro
145                 150                 155                 160
Cys Phe Gln Asn Ser Ser Ser Glu Ile Ser Ser Glu Phe Thr Tyr Ala
                165                 170                 175
Trp Leu Val Glu Asp Asn Gly Thr Gln Gln Thr Leu Ile Ser Gln Asp
            180                 185                 190
His Leu Ile Ser Ser Ser Thr Leu Leu Lys Asp Arg Val Lys Val Gly
        195                 200                 205
Ile Asp Tyr Arg Leu His Leu Ser Pro Val Gln Ile Phe Asp Asp Gly
    210                 215                 220
Arg Lys Phe Ser Cys His Ile Arg Val Gly Pro Asp Lys Ile Leu Arg
225                 230                 235                 240
Ser Ser Thr Thr Ile Lys Val Phe Ala Lys Pro Glu Ile Pro Met Ile
                245                 250                 255
Val Glu Asn Asn Ser Thr Asp Val Leu Val Glu Arg Thr Phe Thr Cys
            260                 265                 270
Leu Leu Lys Asn Val Phe Pro Lys Ala Asn Ile Ile Trp Phe Ile Asp
        275                 280                 285
Gly Ser Phe Leu His Asp Glu Lys Glu Gly Ile Tyr Ile Thr Asn Glu
    290                 295                 300
Glu Arg Lys Gly Lys Asp Gly Phe Leu Glu Leu Lys Ser Val Leu Thr
305                 310                 315                 320
Arg Val His Ser Asp Lys Pro Ala Gln Ser Asp Asn Leu Thr Ile Trp
                325                 330                 335
Cys Met Ala Leu Ser Pro Val Pro Gly Asn Lys Val Trp Asn Ile Ser
            340                 345                 350
Ser Glu Lys Ile Thr Phe Leu Leu Gly Ser Glu Met Ser Thr Thr Asp
        355                 360                 365

Leu Pro Pro Ser Val Thr Glu Ser Thr Leu Asp Thr Gln Pro Ser Pro
    370                 375                 380
Ala Ser Ser Val Ser Pro Thr Arg Tyr Pro Ala Thr Ser Ser Val Thr
385                 390                 395                 400
Leu Ala Asp Val Ser Ala Leu Arg Pro Asn Thr Thr Pro Gln Ser Ser
                405                 410                 415
Ser Ser Ser Val Thr Thr Gln Asp Phe Asn Tyr Pro Trp Thr Ser Ser
            420                 425                 430
Gly Thr Asp Ala Lys Lys Ser Phe Ser Gln Ile Pro Ser Glu Thr Tyr
        435                 440                 445
Ser Ser Ser Pro Ser Gly Ala Gly Ser Thr Leu His Asp Asn Val Phe
    450                 455                 460
Thr Ser Thr Thr Arg Ala Leu Ser Glu Val Pro Thr Thr Ala Asn Gly
465                 470                 475                 480
Ser Thr Lys Thr Asn His Val His Ile Thr Gly Ile Val Val Ser Lys
                485                 490                 495
Pro Lys Asp Gly Met Asp Tyr Lys Asp His Asp Gly Asp Tyr Lys Asp
            500                 505                 510
His Asp Ile Asp Tyr Lys Asp Asp Asp Asp Lys
        515                 520

<210> 96
<211> 20
<212> PRT
<213> 人工序列(Artificial Sequence)

<220>
<221> PEPTIDE
<223> CD96 胞外区 166-185 位多肽序列

<400> 96
Gln Glu Thr Leu Ile Ser Gln Asn His Leu Ile Ser Asn Ser Thr Leu
1               5                   10                  15
Leu Lys Asp Arg
            20

<210> 97
<211> 20
<212> PRT
<213> 人工序列(Artificial Sequence)

<220>
<221> PEPTIDE
<223> CD96 胞外区 181-200 位多肽序列

<400> 97
Leu Leu Lys Asp Arg Val Lys Leu Gly Thr Asp Tyr Arg Leu His Leu
1               5                   10                  15
Ser Pro Val Gln
            20

<210> 98
<211> 20
<212> PRT
<213> 人工序列(Artificial Sequence)

<220>
<221>  PEPTIDE
<223>  CD96 胞外区 196-215 位多肽序列

<400>  98
Leu Ser Pro Val Gln Ile Phe Asp Asp Gly Arg Lys Phe Ser Cys His
1               5                   10                  15
Ile Arg Val Gly
            20

<210>  99
<211>  20
<212>  PRT
<213>  人工序列(Artificial Sequence)

<220>
<221>  PEPTIDE
<223>  CD96 胞外区 211-230 位多肽序列

<400>  99
His Ile Arg Val Gly Pro Asn Lys Ile Leu Arg Ser Ser Thr Thr Val
1               5                   10                  15
Lys Val Phe Ala
            20

<210>  100
<211>  20
<212>  PRT
<213>  人工序列(Artificial Sequence)

<220>
<221>  PEPTIDE
<223>  CD96 胞外区 220-239 位多肽序列

<400>  100
Leu Arg Ser Ser Thr Thr Val Lys Val Phe Ala Lys Pro Glu Ile Pro
1               5                   10                  15
Val Ile Val Glu
            20

<210>  101
<211>  20
<212>  PRT
<213>  人工序列(Artificial Sequence)

<220>
<221>  PEPTIDE
<223>  CD96 胞外区 235-254 位多肽序列

<400>  101
Pro Val Ile Val Glu Asn Asn Ser Thr Asp Val Leu Val Glu Arg Arg

1                   5                   10                  15
Phe Thr Cys Leu
            20

<210>  102
<211>  20
<212>  PRT
<213>  人工序列(Artificial Sequence)

<220>
<221>  PEPTIDE
<223>  CD96 胞外区 250-269 位多肽序列

<400>  102
Arg Phe Thr Cys Leu Leu Lys Asn Val Phe Pro Lys Ala Asn Ile Thr
1                   5                   10                  15
Trp Phe Ile Asp
            20

<210>  103
<211>  20
<212>  PRT
<213>  人工序列(Artificial Sequence)

<220>
<221>  PEPTIDE
<223>  CD96 胞外区 265-284 位多肽序列

<400>  103
Thr Trp Phe Ile Asp Gly Ser Phe Leu His Asp Glu Lys Glu Gly Ile
1                   5                   10                  15
Tyr Ile Thr Asn
            20

<210>  104
<211>  20
<212>  PRT
<213>  人工序列(Artificial Sequence)

<220>
<221>  PEPTIDE
<223>  CD96 胞外区 280-299 位多肽序列

<400>  104
Ile Tyr Ile Thr Asn Glu Glu Arg Lys Gly Lys Asp Gly Phe Leu Glu
1                   5                   10                  15
Leu Lys Ser Val
            20

<210>  105
<211>  20
<212>  PRT
<213>  人工序列(Artificial Sequence)

<220>
<221>  PEPTIDE
<223>  CD96 胞外区 295-314 位多肽序列

<400>  105
Glu Leu Lys Ser Val Leu Thr Arg Val His Ser Asn Lys Pro Ala Gln
1               5                   10                  15
Ser Asp Asn Leu
            20

<210>  106
<211>  20
<212>  PRT
<213>  人工序列(Artificial Sequence)

<220>
<221>  PEPTIDE
<223>  CD96 胞外区 310-329 位多肽序列

<400>  106
Gln Ser Asp Asn Leu Thr Ile Trp Cys Met Ala Leu Ser Pro Val Pro
1               5                   10                  15
Gly Asn Lys Val
            20

<210>  107
<211>  20
<212>  PRT
<213>  人工序列(Artificial Sequence)

<220>
<221>  PEPTIDE
<223>  CD96 胞外区 325-344 位多肽序列

<400>  107
Pro Gly Asn Lys Val Trp Asn Ile Ser Ser Glu Lys Ile Thr Phe Leu
1               5                   10                  15
Leu Gly Ser Glu
            20

<210>  108
<211>  11
<212>  PRT
<213>  人工序列(Artificial Sequence)

<220>
<221>  DOMAIN
<223>  h1718-VL CDR1 突变体

<400>  108
Lys Ala Ser Gln Asp Ile Asn Gln Tyr Leu Asn

1                   5                        10

<210>  109
<211>  11
<212>  PRT
<213>  人工序列(Artificial Sequence)

<220>
<221>  DOMAIN
<223>  h1718-VL CDR1 突变体

<400>  109
Lys Ala Ser Gln Asp Ile Asn Thr Tyr Leu Asn
1                   5                        10

<210>  110
<211>  11
<212>  PRT
<213>  人工序列(Artificial Sequence)

<220>
<221>  DOMAIN
<223>  h1718-VL CDR1 突变体

<400>  110
Lys Ala Ser Gln Asp Ile Asn Leu Tyr Leu Asn
1                   5                        10

<210>  111
<211>  14
<212>  PRT
<213>  人工序列(Artificial Sequence)

<220>
<221>  DOMAIN
<223>  h1719-VH CDR3 突变体

<400>  111
Gln Asn Tyr Tyr Gly Ser Arg Tyr Gly Tyr Pro Leu Asp Tyr
1                   5                        10

<210>  112
<211>  14
<212>  PRT
<213>  人工序列(Artificial Sequence)

<220>
<221>  DOMAIN
<223>  h1719-VH CDR3 突变体

<400>  112

Asn Tyr Tyr Tyr Gly Ser Arg Tyr Gly Tyr Pro Leu Asp Tyr
1               5                   10

<210>  113
<211>  14
<212>  PRT
<213>  人工序列(Artificial Sequence)

<220>
<221>  DOMAIN
<223>  h1719-VH CDR3 突变体

<400>  113
Asn Gln Tyr Tyr Gly Ser Arg Tyr Gly Tyr Pro Leu Asp Tyr
1               5                   10

<210>  114
<211>  14
<212>  PRT
<213>  人工序列(Artificial Sequence)

<220>
<221>  DOMAIN
<223>  h1719-VH CDR3 突变体

<400>  114
Gln Asn Tyr Tyr Gly Ser Arg Tyr Gly Tyr Pro Ile Asp Tyr
1               5                   10

<210>  115
<211>  14
<212>  PRT
<213>  人工序列(Artificial Sequence)

<220>
<221>  DOMAIN
<223>  h1719-VH CDR3 突变体

<400>  115
Asn Tyr Tyr Tyr Gly Ser Arg Tyr Gly Tyr Pro Ile Asp Tyr
1               5                   10

<210>  116
<211>  14
<212>  PRT
<213>  人工序列(Artificial Sequence)

<220>
<221>  DOMAIN
<223>  h1719-VH CDR3 突变体

<400>  116
Asn Gln Tyr Tyr Gly Ser Arg Tyr Gly Tyr Pro Ile Asp Tyr
1               5                   10

<210>  117
<211>  327
<212>  PRT
<213>  人工序列(Artificial Sequence)

<220>
<221>  DOMAIN
<223>  重链恒定区

<400>  117
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325

<210>  118
<211>  107
<212>  PRT
<213>  人工序列(Artificial Sequence)

<220>
<221>  DOMAIN
<223>  轻链恒定区

<400>  118

<210>  119
<211>  17
<212>  PRT
<213>  人工序列(Artificial Sequence)

<220>
<221>  DOMAIN
<223>  h1722-VH CDR2 的序列通式

<220>
<221>  DOMAIN
<222>  (5)..(5)
<223>  Xaa 选自 Asn,Lys or Gln.

<400>  119
Met Leu His Pro Xaa Ser Gly Thr Thr Ser Phe Asn Glu Lys Phe Lys
1               5                   10                  15
Ile

<210>  120
<211>  17
<212>  PRT
<213>  人工序列(Artificial Sequence)

<220>
<221>  DOMAIN

<223>  h1722-VH CDR2 的突变体

<400>  120
Met Leu His Pro Lys Ser Gly Thr Thr Ser Phe Asn Glu Lys Phe Lys
1               5                   10                  15
Ile

<210>  121
<211>  17
<212>  PRT
<213>  人工序列(Artificial Sequence)

<220>
<221>  DOMAIN
<223>  h1722-VH CDR2 的突变体

<400>  121
Met Leu His Pro Gln Ser Gly Thr Thr Ser Phe Asn Glu Lys Phe Lys
1               5                   10                  15
Ile

<210>  122
<211>  117
<212>  PRT
<213>  人工序列(Artificial Sequence)

<220>
<221>  DOMAIN
<223>  h1722-VH2a (N54K)

<400>  122
Glu Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Asp Tyr
            20                  25                  30
Trp Met His Trp Val Arg Gln Ala Pro Gly Gln Gly Leu Glu Trp Ile
        35                  40                  45
Gly Met Leu His Pro Lys Ser Gly Thr Thr Ser Phe Asn Glu Lys Phe
    50                  55                  60
Lys Ile Arg Val Thr Ile Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Ser Asp Tyr Ser Gly Pro Phe Ala Tyr Trp Gly Gln Gly Thr Leu
            100                 105                 110
Val Thr Val Ser Ser
        115

<210>  123
<211>  117
<212>  PRT
<213>  人工序列(Artificial Sequence)

<220>
<221>  DOMAIN
<223>   h1722-VH2b (N54Q)


<400>  123
Glu Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Asp Tyr
            20                  25                  30
Trp Met His Trp Val Arg Gln Ala Pro Gly Gln Gly Leu Glu Trp Ile
        35                  40                  45
Gly Met Leu His Pro Gln Ser Gly Thr Thr Ser Phe Asn Glu Lys Phe
    50                  55                  60
Lys Ile Arg Val Thr Ile Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Ser Asp Tyr Ser Gly Pro Phe Ala Tyr Trp Gly Gln Gly Thr Leu
            100                 105                 110
Val Thr Val Ser Ser
        115

SEQUENCE LISTING

<110>  JIANGSU HENGRUI MEDICINE CO., LTD.
       SHANGHAI HENGRUI PHARMACEUTICAL CO., LTD

<120>  CD96 antibody, antigen-binding fragment and pharmaceutical use
       thereof

<130>  P20412054US

<140>  PCT/CN2018/114797
<141>  2018-11-09

<150>  CN201711107331.5
<151>  2017-11-10

<160>  123

<170>  PatentIn version 3.5

<210>  1
<211>  511
<212>  PRT
<213>  Artificial Sequence

<220>
<223>  CD96 extracellular region with Flag tag

<400>  1

Met Glu Lys Lys Trp Lys Tyr Cys Ala Val Tyr Tyr Ile Ile Gln Ile
1               5                   10                  15

His Phe Val Lys Gly Val Trp Glu Lys Thr Val Asn Thr Glu Glu Asn
            20                  25                  30

Val Tyr Ala Thr Leu Gly Ser Asp Val Asn Leu Thr Cys Gln Thr Gln
        35                  40                  45

Thr Val Gly Phe Phe Val Gln Met Gln Trp Ser Lys Val Thr Asn Lys
    50                  55                  60

Ile Asp Leu Ile Ala Val Tyr His Pro Gln Tyr Gly Phe Tyr Cys Ala
65                  70                  75                  80

Tyr Gly Arg Pro Cys Glu Ser Leu Val Thr Phe Thr Glu Thr Pro Glu
                85                  90                  95

Asn Gly Ser Lys Trp Thr Leu His Leu Arg Asn Met Ser Cys Ser Val
            100                 105                 110

Ser Gly Arg Tyr Glu Cys Met Leu Val Leu Tyr Pro Glu Gly Ile Gln
        115                 120                 125

Thr Lys Ile Tyr Asn Leu Leu Ile Gln Thr His Val Thr Ala Asp Glu

130                 135                 140

Trp Asn Ser Asn His Thr Ile Glu Ile Glu Ile Asn Gln Thr Leu Glu
145                 150                 155                 160

Ile Pro Cys Phe Gln Asn Ser Ser Ser Lys Ile Ser Ser Glu Phe Thr
                165                 170                 175

Tyr Ala Trp Ser Val Glu Asp Asn Gly Thr Gln Glu Thr Leu Ile Ser
            180                 185                 190

Gln Asn His Leu Ile Ser Asn Ser Thr Leu Leu Lys Asp Arg Val Lys
        195                 200                 205

Leu Gly Thr Asp Tyr Arg Leu His Leu Ser Pro Val Gln Ile Phe Asp
    210                 215                 220

Asp Gly Arg Lys Phe Ser Cys His Ile Arg Val Gly Pro Asn Lys Ile
225                 230                 235                 240

Leu Arg Ser Ser Thr Thr Val Lys Val Phe Ala Lys Pro Glu Ile Pro
                245                 250                 255

Val Ile Val Glu Asn Asn Ser Thr Asp Val Leu Val Glu Arg Arg Phe
            260                 265                 270

Thr Cys Leu Leu Lys Asn Val Phe Pro Lys Ala Asn Ile Thr Trp Phe
        275                 280                 285

Ile Asp Gly Ser Phe Leu His Asp Glu Lys Glu Gly Ile Tyr Ile Thr
    290                 295                 300

Asn Glu Glu Arg Lys Gly Lys Asp Gly Phe Leu Glu Leu Lys Ser Val
305                 310                 315                 320

Leu Thr Arg Val His Ser Asn Lys Pro Ala Gln Ser Asp Asn Leu Thr
                325                 330                 335

Ile Trp Cys Met Ala Leu Ser Pro Val Pro Gly Asn Lys Val Trp Asn
            340                 345                 350

Ile Ser Ser Glu Lys Ile Thr Phe Leu Leu Gly Ser Glu Ile Ser Ser
        355                 360                 365

Thr Asp Pro Pro Leu Ser Val Thr Glu Ser Thr Leu Asp Thr Gln Pro
    370                 375                 380

Ser Pro Ala Ser Ser Val Ser Pro Ala Arg Tyr Pro Ala Thr Ser Ser
385                 390                 395                 400

Val Thr Leu Val Asp Val Ser Ala Leu Arg Pro Asn Thr Thr Pro Gln
                405                 410                 415

Pro Ser Asn Ser Ser Met Thr Thr Arg Gly Phe Asn Tyr Pro Trp Thr
            420                 425                 430

Ser Ser Gly Thr Asp Thr Lys Lys Ser Val Ser Arg Ile Pro Ser Glu
        435                 440                 445

Thr Tyr Ser Ser Ser Pro Ser Gly Ala Gly Ser Thr Leu His Asp Asn
    450                 455                 460

Val Phe Thr Ser Thr Ala Arg Ala Phe Ser Glu Val Pro Thr Thr Ala
465                 470                 475                 480

Asn Gly Ser Thr Lys Thr Asn His Val His Ile Thr Gly Ile Val Val
                485                 490                 495

Asn Lys Pro Lys Asp Gly Met Asp Tyr Lys Asp Asp Asp Asp Lys
            500                 505                 510

<210>  2
<211>  734
<212>  PRT
<213>  Artificial Sequence

<220>
<223>  Fusion protein of CD96 extracellular domain and mIgG2a Fc

<400>  2

Met Glu Phe Gly Leu Ser Trp Leu Phe Leu Val Ala Ile Leu Lys Gly
1               5                   10                  15

Val Gln Cys Val Trp Glu Lys Thr Val Asn Thr Glu Glu Asn Val Tyr
            20                  25                  30

Ala Thr Leu Gly Ser Asp Val Asn Leu Thr Cys Gln Thr Gln Thr Val
        35                  40                  45

Gly Phe Phe Val Gln Met Gln Trp Ser Lys Val Thr Asn Lys Ile Asp
    50                  55                  60

Leu Ile Ala Val Tyr His Pro Gln Tyr Gly Phe Tyr Cys Ala Tyr Gly
65                  70                  75                  80

Arg Pro Cys Glu Ser Leu Val Thr Phe Thr Glu Thr Pro Glu Asn Gly

                85                  90                  95

Ser Lys Trp Thr Leu His Leu Arg Asn Met Ser Cys Ser Val Ser Gly
            100                 105                 110

Arg Tyr Glu Cys Met Leu Val Leu Tyr Pro Glu Gly Ile Gln Thr Lys
        115                 120                 125

Ile Tyr Asn Leu Leu Ile Gln Thr His Val Thr Ala Asp Glu Trp Asn
    130                 135                 140

Ser Asn His Thr Ile Glu Ile Glu Ile Asn Gln Thr Leu Glu Ile Pro
145                 150                 155                 160

Cys Phe Gln Asn Ser Ser Ser Lys Ile Ser Ser Glu Phe Thr Tyr Ala
                165                 170                 175

Trp Ser Val Glu Asp Asn Gly Thr Gln Glu Thr Leu Ile Ser Gln Asn
            180                 185                 190

His Leu Ile Ser Asn Ser Thr Leu Leu Lys Asp Arg Val Lys Leu Gly
        195                 200                 205

Thr Asp Tyr Arg Leu His Leu Ser Pro Val Gln Ile Phe Asp Asp Gly
    210                 215                 220

Arg Lys Phe Ser Cys His Ile Arg Val Gly Pro Asn Lys Ile Leu Arg
225                 230                 235                 240

Ser Ser Thr Thr Val Lys Val Phe Ala Lys Pro Glu Ile Pro Val Ile
                245                 250                 255

Val Glu Asn Asn Ser Thr Asp Val Leu Val Glu Arg Arg Phe Thr Cys
            260                 265                 270

Leu Leu Lys Asn Val Phe Pro Lys Ala Asn Ile Thr Trp Phe Ile Asp
        275                 280                 285

Gly Ser Phe Leu His Asp Glu Lys Glu Gly Ile Tyr Ile Thr Asn Glu
    290                 295                 300

Glu Arg Lys Gly Lys Asp Gly Phe Leu Glu Leu Lys Ser Val Leu Thr
305                 310                 315                 320

Arg Val His Ser Asn Lys Pro Ala Gln Ser Asp Asn Leu Thr Ile Trp
                325                 330                 335

Cys Met Ala Leu Ser Pro Val Pro Gly Asn Lys Val Trp Asn Ile Ser
                340                 345                 350

Ser Glu Lys Ile Thr Phe Leu Leu Gly Ser Glu Ile Ser Ser Thr Asp
        355                 360                 365

Pro Pro Leu Ser Val Thr Glu Ser Thr Leu Asp Thr Gln Pro Ser Pro
    370                 375                 380

Ala Ser Ser Val Ser Pro Ala Arg Tyr Pro Ala Thr Ser Ser Val Thr
385                 390                 395                 400

Leu Val Asp Val Ser Ala Leu Arg Pro Asn Thr Thr Pro Gln Pro Ser
                405                 410                 415

Asn Ser Ser Met Thr Thr Arg Gly Phe Asn Tyr Pro Trp Thr Ser Ser
            420                 425                 430

Gly Thr Asp Thr Lys Lys Ser Val Ser Arg Ile Pro Ser Glu Thr Tyr
        435                 440                 445

Ser Ser Ser Pro Ser Gly Ala Gly Ser Thr Leu His Asp Asn Val Phe
    450                 455                 460

Thr Ser Thr Ala Arg Ala Phe Ser Glu Val Pro Thr Thr Ala Asn Gly
465                 470                 475                 480

Ser Thr Lys Thr Asn His Val His Ile Thr Gly Ile Val Val Asn Lys
                485                 490                 495

Pro Lys Asp Gly Met Glu Pro Arg Gly Pro Thr Ile Lys Pro Cys Pro
            500                 505                 510

Pro Cys Lys Cys Pro Ala Pro Asn Leu Leu Gly Gly Pro Ser Val Phe
        515                 520                 525

Ile Phe Pro Pro Lys Ile Lys Asp Val Leu Met Ile Ser Leu Ser Pro
    530                 535                 540

Ile Val Thr Cys Val Val Val Asp Val Ser Glu Asp Asp Pro Asp Val
545                 550                 555                 560

Gln Ile Ser Trp Phe Val Asn Asn Val Glu Val His Thr Ala Gln Thr
                565                 570                 575

Gln Thr His Arg Glu Asp Tyr Asn Ser Thr Leu Arg Val Val Ser Ala
            580                 585                 590

Leu Pro Ile Gln His Gln Asp Trp Met Ser Gly Lys Glu Phe Lys Cys
        595                 600                 605

Lys Val Asn Asn Lys Asp Leu Pro Ala Pro Ile Glu Arg Thr Ile Ser
    610                 615                 620

Lys Pro Lys Gly Ser Val Arg Ala Pro Gln Val Tyr Val Leu Pro Pro
625                 630                 635                 640

Pro Glu Glu Glu Met Thr Lys Lys Gln Val Thr Leu Thr Cys Met Val
                645                 650                 655

Thr Asp Phe Met Pro Glu Asp Ile Tyr Val Glu Trp Thr Asn Asn Gly
            660                 665                 670

Lys Thr Glu Leu Asn Tyr Lys Asn Thr Glu Pro Val Leu Asp Ser Asp
        675                 680                 685

Gly Ser Tyr Phe Met Tyr Ser Lys Leu Arg Val Glu Lys Lys Asn Trp
    690                 695                 700

Val Glu Arg Asn Ser Tyr Ser Cys Ser Val Val His Glu Gly Leu His
705                 710                 715                 720

Asn His His Thr Thr Lys Ser Phe Ser Arg Thr Pro Gly Lys
                725                 730

<210> 3
<211> 569
<212> PRT
<213> Artificial Sequence

<220>
<223> Full-length CD96

<400> 3

Met Glu Lys Lys Trp Lys Tyr Cys Ala Val Tyr Tyr Ile Ile Gln Ile
1               5                   10                  15

His Phe Val Lys Gly Val Trp Glu Lys Thr Val Asn Thr Glu Glu Asn
            20                  25                  30

Val Tyr Ala Thr Leu Gly Ser Asp Val Asn Leu Thr Cys Gln Thr Gln
        35                  40                  45

Thr Val Gly Phe Phe Val Gln Met Gln Trp Ser Lys Val Thr Asn Lys
    50                  55                  60

Ile Asp Leu Ile Ala Val Tyr His Pro Gln Tyr Gly Phe Tyr Cys Ala
65                  70                  75                  80

Tyr Gly Arg Pro Cys Glu Ser Leu Val Thr Phe Thr Glu Thr Pro Glu
                85                  90                  95

Asn Gly Ser Lys Trp Thr Leu His Leu Arg Asn Met Ser Cys Ser Val
            100                 105                 110

Ser Gly Arg Tyr Glu Cys Met Leu Val Leu Tyr Pro Glu Gly Ile Gln
        115                 120                 125

Thr Lys Ile Tyr Asn Leu Leu Ile Gln Thr His Val Thr Ala Asp Glu
    130                 135                 140

Trp Asn Ser Asn His Thr Ile Glu Ile Glu Ile Asn Gln Thr Leu Glu
145                 150                 155                 160

Ile Pro Cys Phe Gln Asn Ser Ser Ser Lys Ile Ser Ser Glu Phe Thr
                165                 170                 175

Tyr Ala Trp Ser Val Glu Asp Asn Gly Thr Gln Glu Thr Leu Ile Ser
            180                 185                 190

Gln Asn His Leu Ile Ser Asn Ser Thr Leu Leu Lys Asp Arg Val Lys
        195                 200                 205

Leu Gly Thr Asp Tyr Arg Leu His Leu Ser Pro Val Gln Ile Phe Asp
    210                 215                 220

Asp Gly Arg Lys Phe Ser Cys His Ile Arg Val Gly Pro Asn Lys Ile
225                 230                 235                 240

Leu Arg Ser Ser Thr Thr Val Lys Val Phe Ala Lys Pro Glu Ile Pro
                245                 250                 255

Val Ile Val Glu Asn Asn Ser Thr Asp Val Leu Val Glu Arg Arg Phe
            260                 265                 270

Thr Cys Leu Leu Lys Asn Val Phe Pro Lys Ala Asn Ile Thr Trp Phe
        275                 280                 285

Ile Asp Gly Ser Phe Leu His Asp Glu Lys Glu Gly Ile Tyr Ile Thr
    290                 295                 300

Asn Glu Glu Arg Lys Gly Lys Asp Gly Phe Leu Glu Leu Lys Ser Val
305                 310                 315                 320

Leu Thr Arg Val His Ser Asn Lys Pro Ala Gln Ser Asp Asn Leu Thr
                325                 330                 335

Ile Trp Cys Met Ala Leu Ser Pro Val Pro Gly Asn Lys Val Trp Asn
            340                 345                 350

Ile Ser Ser Glu Lys Ile Thr Phe Leu Leu Gly Ser Glu Ile Ser Ser
        355                 360                 365

Thr Asp Pro Pro Leu Ser Val Thr Glu Ser Thr Leu Asp Thr Gln Pro
    370                 375                 380

Ser Pro Ala Ser Ser Val Ser Pro Ala Arg Tyr Pro Ala Thr Ser Ser
385                 390                 395                 400

Val Thr Leu Val Asp Val Ser Ala Leu Arg Pro Asn Thr Thr Pro Gln
                405                 410                 415

Pro Ser Asn Ser Ser Met Thr Thr Arg Gly Phe Asn Tyr Pro Trp Thr
            420                 425                 430

Ser Ser Gly Thr Asp Thr Lys Lys Ser Val Ser Arg Ile Pro Ser Glu
        435                 440                 445

Thr Tyr Ser Ser Ser Pro Ser Gly Ala Gly Ser Thr Leu His Asp Asn
    450                 455                 460

Val Phe Thr Ser Thr Ala Arg Ala Phe Ser Glu Val Pro Thr Thr Ala
465                 470                 475                 480

Asn Gly Ser Thr Lys Thr Asn His Val His Ile Thr Gly Ile Val Val
                485                 490                 495

Asn Lys Pro Lys Asp Gly Met Ser Trp Pro Val Ile Val Ala Ala Leu
            500                 505                 510

Leu Phe Cys Cys Met Ile Leu Phe Gly Leu Gly Val Arg Lys Trp Cys
        515                 520                 525

Gln Tyr Gln Lys Glu Ile Met Glu Arg Pro Pro Pro Phe Lys Pro Pro
    530                 535                 540

Pro Pro Pro Ile Lys Tyr Thr Cys Ile Gln Glu Pro Asn Glu Ser Asp
545                 550                 555                 560

Leu Pro Tyr His Glu Met Glu Thr Leu
                565

<210>  4
<211>  509
<212>  PRT
<213>  Artificial Sequence

<220>
<223>  CD96 extracellular region with His tag

<400>  4

Met Glu Lys Lys Trp Lys Tyr Cys Ala Val Tyr Tyr Ile Ile Gln Ile
1               5                   10                  15

His Phe Val Lys Gly Val Trp Glu Lys Thr Val Asn Thr Glu Glu Asn
            20                  25                  30

Val Tyr Ala Thr Leu Gly Ser Asp Val Asn Leu Thr Cys Gln Thr Gln
        35                  40                  45

Thr Val Gly Phe Phe Val Gln Met Gln Trp Ser Lys Val Thr Asn Lys
    50                  55                  60

Ile Asp Leu Ile Ala Val Tyr His Pro Gln Tyr Gly Phe Tyr Cys Ala
65                  70                  75                  80

Tyr Gly Arg Pro Cys Glu Ser Leu Val Thr Phe Thr Glu Thr Pro Glu
                85                  90                  95

Asn Gly Ser Lys Trp Thr Leu His Leu Arg Asn Met Ser Cys Ser Val
            100                 105                 110

Ser Gly Arg Tyr Glu Cys Met Leu Val Leu Tyr Pro Glu Gly Ile Gln
        115                 120                 125

Thr Lys Ile Tyr Asn Leu Leu Ile Gln Thr His Val Thr Ala Asp Glu
    130                 135                 140

Trp Asn Ser Asn His Thr Ile Glu Ile Glu Ile Asn Gln Thr Leu Glu
145                 150                 155                 160

Ile Pro Cys Phe Gln Asn Ser Ser Ser Lys Ile Ser Ser Glu Phe Thr
                165                 170                 175

Tyr Ala Trp Ser Val Glu Asp Asn Gly Thr Gln Glu Thr Leu Ile Ser
            180                 185                 190

Gln Asn His Leu Ile Ser Asn Ser Thr Leu Leu Lys Asp Arg Val Lys
        195                 200                 205

Leu Gly Thr Asp Tyr Arg Leu His Leu Ser Pro Val Gln Ile Phe Asp
    210                 215                 220

Asp Gly Arg Lys Phe Ser Cys His Ile Arg Val Gly Pro Asn Lys Ile
225                 230                 235                 240

Leu Arg Ser Ser Thr Thr Val Lys Val Phe Ala Lys Pro Glu Ile Pro
                245                 250                 255

Val Ile Val Glu Asn Asn Ser Thr Asp Val Leu Val Glu Arg Arg Phe
            260                 265                 270

Thr Cys Leu Leu Lys Asn Val Phe Pro Lys Ala Asn Ile Thr Trp Phe
        275                 280                 285

Ile Asp Gly Ser Phe Leu His Asp Glu Lys Glu Gly Ile Tyr Ile Thr
    290                 295                 300

Asn Glu Glu Arg Lys Gly Lys Asp Gly Phe Leu Glu Leu Lys Ser Val
305                 310                 315                 320

Leu Thr Arg Val His Ser Asn Lys Pro Ala Gln Ser Asp Asn Leu Thr
                325                 330                 335

Ile Trp Cys Met Ala Leu Ser Pro Val Pro Gly Asn Lys Val Trp Asn
            340                 345                 350

Ile Ser Ser Glu Lys Ile Thr Phe Leu Leu Gly Ser Glu Ile Ser Ser
        355                 360                 365

Thr Asp Pro Pro Leu Ser Val Thr Glu Ser Thr Leu Asp Thr Gln Pro
    370                 375                 380

Ser Pro Ala Ser Ser Val Ser Pro Ala Arg Tyr Pro Ala Thr Ser Ser
385                 390                 395                 400

Val Thr Leu Val Asp Val Ser Ala Leu Arg Pro Asn Thr Thr Pro Gln
                405                 410                 415

Pro Ser Asn Ser Ser Met Thr Thr Arg Gly Phe Asn Tyr Pro Trp Thr
            420                 425                 430

Ser Ser Gly Thr Asp Thr Lys Lys Ser Val Ser Arg Ile Pro Ser Glu
        435                 440                 445

Thr Tyr Ser Ser Ser Pro Ser Gly Ala Gly Ser Thr Leu His Asp Asn
    450                 455                 460

Val Phe Thr Ser Thr Ala Arg Ala Phe Ser Glu Val Pro Thr Thr Ala
465                 470                 475                 480

Asn Gly Ser Thr Lys Thr Asn His Val His Ile Thr Gly Ile Val Val
                485                 490                 495

Asn Lys Pro Lys Asp Gly Met His His His His His His
            500                 505

<210>  5
<211>  733
<212>  PRT
<213>  Artificial Sequence

<220>
<223>  Fusion protein of CD96 extracellular region and hIgG1Fc

<400>  5

Met Glu Phe Gly Leu Ser Trp Leu Phe Leu Val Ala Ile Leu Lys Gly
1               5                   10                  15

Val Gln Cys Val Trp Glu Lys Thr Val Asn Thr Glu Glu Asn Val Tyr
            20                  25                  30

Ala Thr Leu Gly Ser Asp Val Asn Leu Thr Cys Gln Thr Gln Thr Val
        35                  40                  45

Gly Phe Phe Val Gln Met Gln Trp Ser Lys Val Thr Asn Lys Ile Asp
    50                  55                  60

Leu Ile Ala Val Tyr His Pro Gln Tyr Gly Phe Tyr Cys Ala Tyr Gly
65                  70                  75                  80

Arg Pro Cys Glu Ser Leu Val Thr Phe Thr Glu Thr Pro Glu Asn Gly
                85                  90                  95

Ser Lys Trp Thr Leu His Leu Arg Asn Met Ser Cys Ser Val Ser Gly
            100                 105                 110

Arg Tyr Glu Cys Met Leu Val Leu Tyr Pro Glu Gly Ile Gln Thr Lys
        115                 120                 125

Ile Tyr Asn Leu Leu Ile Gln Thr His Val Thr Ala Asp Glu Trp Asn
    130                 135                 140

Ser Asn His Thr Ile Glu Ile Glu Ile Asn Gln Thr Leu Glu Ile Pro
145                 150                 155                 160

Cys Phe Gln Asn Ser Ser Ser Lys Ile Ser Ser Glu Phe Thr Tyr Ala
                165                 170                 175

Trp Ser Val Glu Asp Asn Gly Thr Gln Glu Thr Leu Ile Ser Gln Asn
            180                 185                 190

His Leu Ile Ser Asn Ser Thr Leu Leu Lys Asp Arg Val Lys Leu Gly
        195                 200                 205

Thr Asp Tyr Arg Leu His Leu Ser Pro Val Gln Ile Phe Asp Asp Gly
    210                 215                 220

Arg Lys Phe Ser Cys His Ile Arg Val Gly Pro Asn Lys Ile Leu Arg
225                 230                 235                 240

Ser Ser Thr Thr Val Lys Val Phe Ala Lys Pro Glu Ile Pro Val Ile
                245                 250                 255

Val Glu Asn Asn Ser Thr Asp Val Leu Val Glu Arg Arg Phe Thr Cys
            260                 265                 270

Leu Leu Lys Asn Val Phe Pro Lys Ala Asn Ile Thr Trp Phe Ile Asp
        275                 280                 285

Gly Ser Phe Leu His Asp Glu Lys Glu Gly Ile Tyr Ile Thr Asn Glu
    290                 295                 300

Glu Arg Lys Gly Lys Asp Gly Phe Leu Glu Leu Lys Ser Val Leu Thr
305                 310                 315                 320

Arg Val His Ser Asn Lys Pro Ala Gln Ser Asp Asn Leu Thr Ile Trp
                325                 330                 335

Cys Met Ala Leu Ser Pro Val Pro Gly Asn Lys Val Trp Asn Ile Ser
            340                 345                 350

Ser Glu Lys Ile Thr Phe Leu Leu Gly Ser Glu Ile Ser Ser Thr Asp
        355                 360                 365

Pro Pro Leu Ser Val Thr Glu Ser Thr Leu Asp Thr Gln Pro Ser Pro
    370                 375                 380

Ala Ser Ser Val Ser Pro Ala Arg Tyr Pro Ala Thr Ser Ser Val Thr
385                 390                 395                 400

Leu Val Asp Val Ser Ala Leu Arg Pro Asn Thr Thr Pro Gln Pro Ser
                405                 410                 415

Asn Ser Ser Met Thr Thr Arg Gly Phe Asn Tyr Pro Trp Thr Ser Ser
            420                 425                 430

Gly Thr Asp Thr Lys Lys Ser Val Ser Arg Ile Pro Ser Glu Thr Tyr
        435                 440                 445

Ser Ser Ser Pro Ser Gly Ala Gly Ser Thr Leu His Asp Asn Val Phe
    450                 455                 460

Thr Ser Thr Ala Arg Ala Phe Ser Glu Val Pro Thr Thr Ala Asn Gly
465                 470                 475                 480

Ser Thr Lys Thr Asn His Val His Ile Thr Gly Ile Val Val Asn Lys
                485                 490                 495

Pro Lys Asp Gly Met Glu Pro Lys Ser Ser Asp Lys Thr His Thr Cys
            500                 505                 510

Pro Pro Cys Pro Ala Pro Glu Leu Leu Gly Gly Pro Ser Val Phe Leu
        515                 520                 525

Phe Pro Pro Lys Pro Lys Asp Thr Leu Met Ile Ser Arg Thr Pro Glu
    530                 535                 540

Val Thr Cys Val Val Val Asp Val Ser His Glu Asp Pro Glu Val Lys
545                 550                 555                 560

Phe Asn Trp Tyr Val Asp Gly Val Glu Val His Asn Ala Lys Thr Lys
                565                 570                 575

Pro Arg Glu Glu Gln Tyr Asn Ser Thr Tyr Arg Val Val Ser Val Leu
            580                 585                 590

Thr Val Leu His Gln Asp Trp Leu Asn Gly Lys Glu Tyr Lys Cys Lys
        595                 600                 605

Val Ser Asn Lys Ala Leu Pro Ala Pro Ile Glu Lys Thr Ile Ser Lys
    610                 615                 620

Ala Lys Gly Gln Pro Arg Glu Pro Gln Val Tyr Thr Leu Pro Pro Ser
625                 630                 635                 640

Arg Asp Glu Leu Thr Lys Asn Gln Val Ser Leu Thr Cys Leu Val Lys
                645                 650                 655

Gly Phe Tyr Pro Ser Asp Ile Ala Val Glu Trp Glu Ser Asn Gly Gln

Pro Glu Asn Asn Tyr Lys Thr Thr Pro Pro Val Leu Asp Ser Asp Gly
        675                 680                 685

Ser Phe Phe Leu Tyr Ser Lys Leu Thr Val Asp Lys Ser Arg Trp Gln
    690                 695                 700

Gln Gly Asn Val Phe Ser Cys Ser Val Met His Glu Ala Leu His Asn
705                 710                 715                 720

His Tyr Thr Gln Lys Ser Leu Ser Leu Ser Pro Gly Lys
                725                 730

<210>  6
<211>  119
<212>  PRT
<213>  Artificial Sequence

<220>
<223>  m1718 VH

<400>  6

Glu Phe Gln Leu Gln Gln Ser Gly Pro Glu Leu Val Lys Pro Gly Ala
1               5                   10                  15

Ser Val Lys Ile Ser Cys Lys Ala Ser Ala Tyr Ser Ile Thr Asp Tyr
            20                  25                  30

Asn Met Asn Trp Val Lys Gln Ser Asn Gly Lys Ser Leu Glu Trp Ile
        35                  40                  45

Gly Val Ile Asn Pro Ser Tyr Gly Ile Thr Asp Tyr Asn Gln Asn Phe
    50                  55                  60

Lys Asp Lys Ala Thr Leu Thr Val Asp Gln Ser Ser Ser Thr Ala Tyr
65                  70                  75                  80

Met Gln Leu Asn Ser Leu Thr Ser Glu Asp Ser Ala Val Tyr Tyr Cys
                85                  90                  95

Ala Ile Gln Leu Arg Leu Pro Gly Tyr Phe Asp Val Trp Gly Thr Gly
            100                 105                 110

Thr Thr Val Ile Val Ser Ser
        115

<210>  7
<211>  107

<212>  PRT
<213>  Artificial Sequence

<220>
<223>  m1718 VL

<400>  7

Asp Ile Lys Met Thr Gln Ser Pro Ser Ser Met Tyr Ala Ser Leu Gly
1               5                   10                  15

Glu Arg Val Thr Ile Thr Cys Lys Ala Ser Gln Asp Ile Asn Asn Tyr
            20                  25                  30

Leu Asn Trp Phe Gln Gln Lys Pro Gly Lys Ser Pro Lys Thr Leu Ile
        35                  40                  45

Tyr Arg Ala Asn Arg Leu Val Asp Gly Val Pro Ser Arg Phe Ser Gly
    50                  55                  60

Ser Gly Ser Gly Gln Asp Tyr Ser Leu Thr Ile Ser Ser Leu Glu Tyr
65                  70                  75                  80

Glu Asp Met Gly Ile Tyr Tyr Cys Leu Lys Tyr Asp Glu Phe Pro Tyr
                85                  90                  95

Thr Phe Gly Gly Gly Thr Lys Val Glu Ile Lys
            100                 105


<210>  8
<211>  122
<212>  PRT
<213>  Artificial Sequence

<220>
<223>  m1719VH

<400>  8

Gln Val Gln Leu Lys Glu Ser Gly Pro Gly Leu Val Ala Pro Ser Gln
1               5                   10                  15

Ser Leu Ser Ile Thr Cys Thr Val Ser Gly Phe Ser Leu Ile Ser Tyr
            20                  25                  30

Gly Val Ser Trp Val Arg Gln Pro Pro Gly Lys Gly Leu Glu Trp Leu
        35                  40                  45

Gly Val Ile Trp Gly Asp Gly Asn Thr Asn Tyr His Ser Val Leu Ile
    50                  55                  60

Ser Arg Leu Ser Ile Arg Lys Asp Asp Ser Lys Ser Gln Val Phe Leu

65                  70                  75                  80

Lys Leu Asn Ser Leu Gln Thr Asp Asp Thr Ala Thr Tyr Tyr Cys Ala
                85                  90                  95

Lys Asn Asn Tyr Tyr Gly Ser Arg Tyr Gly Tyr Pro Met Asp Tyr Trp
            100                 105                 110

Gly Gln Gly Thr Ser Val Thr Val Ser Ser
        115                 120

<210>  9
<211>  107
<212>  PRT
<213>  Artificial Sequence

<220>
<223>  m1719VL

<400>  9

Asp Ile Val Met Thr Gln Ser His Lys Phe Met Ser Thr Ser Val Gly
1               5                   10                  15

Asp Arg Val Ser Ile Thr Cys Lys Ala Thr Gln Asp Val Gly Thr Ala
            20                  25                  30

Val Ala Trp Tyr Gln Gln Lys Pro Gly His Ser Pro Lys Leu Leu Ile
        35                  40                  45

Tyr Trp Ala Ser Thr Arg His Thr Gly Val Pro Glu Arg Phe Thr Gly
    50                  55                  60

Ser Gly Ser Gly Thr Asp Phe Thr Leu Thr Ile Asp Asn Val Gln Ser
65                  70                  75                  80

Glu Asp Leu Ala Asp Tyr Phe Cys Gln Gln Tyr Gly Ser Ser Val Leu
                85                  90                  95

Thr Phe Gly Ala Gly Thr Lys Val Glu Leu Arg
            100                 105

<210>  10
<211>  119
<212>  PRT
<213>  Artificial Sequence

<220>
<223>  m1720VH

<400>  10

Glu Phe Gln Leu Gln Gln Ser Gly Pro Glu Leu Val Lys Pro Gly Ala
1               5                   10                  15

Ser Val Lys Ile Ser Cys Lys Ala Ser Ala Tyr Ser Leu Thr Asp Tyr
            20                  25                  30

Asn Met Asn Trp Val Lys Gln Ser Asn Gly Lys Ser Leu Glu Trp Ile
        35                  40                  45

Gly Val Ile Asn Pro Ser Tyr Gly Ile Ser Ser Tyr Asn Gln Lys Phe
    50                  55                  60

Lys Gly Lys Ala Thr Leu Thr Val Asp Gln Ser Ser Ser Thr Ala Tyr
65                  70                  75                  80

Met His Leu Asn Ser Leu Thr Ser Glu Asp Ser Ala Val Tyr Tyr Cys
                85                  90                  95

Ala Arg Gln Leu Arg Leu Pro Gly Tyr Phe Asp Val Trp Gly Thr Gly
            100                 105                 110

Thr Thr Val Thr Val Ser Ser
        115

<210>  11
<211>  107
<212>  PRT
<213>  Artificial Sequence

<220>
<223>  m1720VL

<400>  11

Asp Ile Lys Met Thr Gln Ser Pro Ser Ser Lys Asn Ala Ser Leu Gly
1               5                   10                  15

Glu Arg Val Thr Ile Thr Cys Lys Ala Ser Gln Asp Ile Asn Ser Tyr
            20                  25                  30

Leu Asn Trp Phe Gln Gln Lys Pro Gly Lys Ser Pro Lys Thr Leu Ile
        35                  40                  45

Tyr Arg Ala Ser Arg Leu Val Asp Gly Val Pro Ser Arg Phe Ser Gly
    50                  55                  60

Ser Gly Ser Gly Gln Asp Tyr Ser Leu Thr Ile Ser Ser Leu Glu Tyr
65                  70                  75                  80

Glu Asp Met Gly Ile Tyr Tyr Cys Leu Lys Tyr Asp Glu Phe Pro Tyr

85                  90                  95

Thr Phe Gly Gly Gly Thr Lys Leu Glu Ile Lys
            100                 105

<210>  12
<211>  122
<212>  PRT
<213>  Artificial Sequence

<220>
<223>  m1721VH

<400>  12

Gln Val Gln Leu Lys Glu Ser Gly Pro Gly Leu Val Ala Pro Ser Gln
1               5                   10                  15

Ser Leu Ser Ile Thr Cys Thr Val Ser Gly Phe Ser Leu Ile Ser Tyr
            20                  25                  30

Gly Val Asn Trp Val Arg Gln Pro Pro Gly Lys Gly Leu Glu Trp Leu
        35                  40                  45

Gly Val Ile Trp Gly Asp Gly Ser Thr Asn Tyr His Ser Ala Leu Ile
    50                  55                  60

Ser Arg Leu Ser Ile Ser Lys Asp Asp Ser Lys Ser Gln Val Phe Leu
65                  70                  75                  80

Asn Leu Asn Ser Leu Gln Thr Asp Asp Thr Ala Thr Tyr Tyr Cys Ala
                85                  90                  95

Lys Asn Tyr Phe Tyr Gly Ser Arg Tyr Gly Tyr Ala Met Asp Ser Trp
            100                 105                 110

Gly Gln Gly Ile Ser Val Thr Val Ser Ser
        115                 120

<210>  13
<211>  107
<212>  PRT
<213>  Artificial Sequence

<220>
<223>  m1721VL

<400>  13

Asp Ile Gln Met Thr Gln Ser Pro Ala Ser Leu Ser Ala Ala Val Gly
1               5                   10                  15

Glu Thr Val Thr Ile Thr Cys Arg Ala Ser Glu Asn Ile Tyr Ser Ser
            20                  25                  30

Leu Ala Trp Tyr Gln Gln Lys Gln Gly Lys Ser Pro Gln Leu Leu Val
        35                  40                  45

Tyr Asn Ala Lys Thr Leu Ile Glu Thr Val Ala Ser Arg Phe Ser Gly
    50                  55                  60

Ser Gly Ser Gly Thr Gln Tyr Ser Leu Lys Ile Asn Ser Leu Gln Pro
65                  70                  75                  80

Glu Asp Phe Gly Ser Phe Tyr Cys Gln His His Tyr Gly Thr Pro Tyr
                85                  90                  95

Thr Phe Gly Gly Gly Thr Lys Leu Glu Ile Lys
            100                 105

<210>  14
<211>  117
<212>  PRT
<213>  Artificial Sequence

<220>
<223>  m1722VH

<400>  14

Gln Val Gln Leu Gln Gln Pro Gly Ala Glu Leu Val Lys Pro Gly Thr
1               5                   10                  15

Ser Val Lys Leu Ser Cys Lys Ala Ser Gly Asn Thr Phe Ile Asp Tyr
            20                  25                  30

Trp Met His Trp Val Lys Gln Arg Pro Gly Gln Gly Leu Glu Trp Ile
        35                  40                  45

Gly Met Leu His Pro Asn Ser Gly Thr Thr Ser Phe Asn Glu Lys Phe
    50                  55                  60

Lys Ile Lys Thr Thr Leu Thr Ile Asp Lys Ser Ser Ser Thr Ala Tyr
65                  70                  75                  80

Met Gln Leu Ser Ser Leu Thr Ser Glu Asp Ser Ala Ile Tyr Tyr Cys
                85                  90                  95

Ala Ser Asp Tyr Ser Gly Pro Phe Ala Tyr Trp Gly Gln Gly Thr Leu
            100                 105                 110

Val Thr Val Ser Ala

        115

<210>  15
<211>  106
<212>  PRT
<213>  Artificial Sequence

<220>
<223>  m1722VL

<400>  15

Gln Ile Val Leu Thr Gln Ser Pro Gly Ile Met Ser Ala Phe Pro Gly
1               5                   10                  15

Glu Lys Val Thr Met Thr Cys Ser Ala Ser Ser Ser Val Asn Tyr Ile
            20                  25                  30

His Trp Tyr Gln Gln Lys Ser Gly Thr Ser Pro Lys Arg Trp Ile Phe
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Trp Arg Ser Asn Pro Leu Thr
                85                  90                  95

Phe Gly Ala Gly Thr Lys Leu Glu Leu Lys
            100                 105


<210>  16
<211>  5
<212>  PRT
<213>  Artificial Sequence

<220>
<223>  m1718 HCDR1

<400>  16

Asp Tyr Asn Met Asn
1               5


<210>  17
<211>  17
<212>  PRT
<213>  Artificial Sequence

<220>
<223>  m1718 HCDR2

<400>  17

Val Ile Asn Pro Ser Tyr Gly Ile Thr Asp Tyr Asn Gln Asn Phe Lys
1               5                   10                  15

Asp

<210>  18
<211>  10
<212>  PRT
<213>  Artificial Sequence

<220>
<223>  m1718 HCDR3

<400>  18

Gln Leu Arg Leu Pro Gly Tyr Phe Asp Val
1               5                   10

<210>  19
<211>  11
<212>  PRT
<213>  Artificial Sequence

<220>
<223>  m1718 LCDR1

<400>  19

Lys Ala Ser Gln Asp Ile Asn Asn Tyr Leu Asn
1               5                   10

<210>  20
<211>  7
<212>  PRT
<213>  Artificial Sequence

<220>
<223>  m1718 LCDR2

<400>  20

Arg Ala Asn Arg Leu Val Asp
1               5

<210>  21
<211>  10
<212>  PRT
<213>  Artificial Sequence

<220>
<223>  m1718 LCDR3

<400>  21

Leu Lys Tyr Asp Glu Phe Pro Tyr Thr Phe

1               5                   10


<210>  22
<211>  5
<212>  PRT
<213>  Artificial Sequence

<220>
<223>  m1719 HCDR1

<400>  22

Ser Tyr Gly Val Ser
1               5


<210>  23
<211>  16
<212>  PRT
<213>  Artificial Sequence

<220>
<223>  m1719 HCDR2

<400>  23

Val Ile Trp Gly Asp Gly Asn Thr Asn Tyr His Ser Val Leu Ile Ser
1               5                   10                  15


<210>  24
<211>  14
<212>  PRT
<213>  Artificial Sequence

<220>
<223>  m1719 HCDR3

<400>  24

Asn Asn Tyr Tyr Gly Ser Arg Tyr Gly Tyr Pro Met Asp Tyr
1               5                   10


<210>  25
<211>  11
<212>  PRT
<213>  Artificial Sequence

<220>
<223>  m1719 LCDR1

<400>  25

Lys Ala Thr Gln Asp Val Gly Thr Ala Val Ala
1               5                   10


<210>  26
<211>  7
<212>  PRT
<213>  Artificial Sequence

<220>
<223>  m1719 LCDR2

<400>  26

Trp Ala Ser Thr Arg His Thr
1               5


<210>  27
<211>  9
<212>  PRT
<213>  Artificial Sequence

<220>
<223>  m1719 LCDR3

<400>  27

Gln Gln Tyr Gly Ser Ser Val Leu Thr
1               5


<210>  28
<211>  5
<212>  PRT
<213>  Artificial Sequence

<220>
<223>  m1720 HCDR1

<400>  28

Asp Tyr Asn Met Asn
1               5


<210>  29
<211>  17
<212>  PRT
<213>  Artificial Sequence

<220>
<223>  m1720 HCDR2

<400>  29

Val Ile Asn Pro Ser Tyr Gly Ile Ser Ser Tyr Asn Gln Lys Phe Lys
1               5                   10                  15


Gly


<210>  30
<211>  10
<212>  PRT
<213>  Artificial Sequence

<220>
<223>  m1720 HCDR3

<400>  30

Gln Leu Arg Leu Pro Gly Tyr Phe Asp Val
1               5                   10


<210>  31
<211>  11
<212>  PRT
<213>  Artificial Sequence

<220>
<223>  m1720 LCDR1

<400>  31

Lys Ala Ser Gln Asp Ile Asn Ser Tyr Leu Asn
1               5                   10


<210>  32
<211>  7
<212>  PRT
<213>  Artificial Sequence

<220>
<223>  m1720 LCDR2

<400>  32

Arg Ala Ser Arg Leu Val Asp
1               5


<210>  33
<211>  10
<212>  PRT
<213>  Artificial Sequence

<220>
<223>  m1720 LCDR3

<400>  33

Leu Lys Tyr Asp Glu Phe Pro Tyr Thr Phe
1               5                   10


<210>  34
<211>  5
<212>  PRT
<213>  Artificial Sequence

<220>
<223>  m1721 HCDR1

<400>  34

Ser Tyr Gly Val Asn
1               5

<210>  35
<211>  16
<212>  PRT
<213>  Artificial Sequence

<220>
<223>  m1721 HCDR2

<400>  35

Val Ile Trp Gly Asp Gly Ser Thr Asn Tyr His Ser Ala Leu Ile Ser
1               5                   10                  15


<210>  36
<211>  14
<212>  PRT
<213>  Artificial Sequence

<220>
<223>  m1721 HCDR3

<400>  36

Asn Tyr Phe Tyr Gly Ser Arg Tyr Gly Tyr Ala Met Asp Ser
1               5                   10


<210>  37
<211>  11
<212>  PRT
<213>  Artificial Sequence

<220>
<223>  m1721 LCDR1

<400>  37

Arg Ala Ser Glu Asn Ile Tyr Ser Ser Leu Ala
1               5                   10


<210>  38
<211>  7
<212>  PRT
<213>  Artificial Sequence

<220>
<223>  m1721 LCDR2

<400>  38

Asn Ala Lys Thr Leu Ile Glu
1               5


<210>  39
<211>  9
<212>  PRT
<213>  Artificial Sequence

<220>
<223>  m1721 LCDR3

<400>  39

Gln His His Tyr Gly Thr Pro Tyr Thr
1               5

<210>  40
<211>  5
<212>  PRT
<213>  Artificial Sequence

<220>
<223>  m1722 HCDR1

<400>  40

Asp Tyr Trp Met His
1               5

<210>  41
<211>  17
<212>  PRT
<213>  Artificial Sequence

<220>
<223>  m1722 HCDR2

<400>  41

Met Leu His Pro Asn Ser Gly Thr Thr Ser Phe Asn Glu Lys Phe Lys
1               5                   10                  15

Ile

<210>  42
<211>  8
<212>  PRT
<213>  Artificial Sequence

<220>
<223>  m1722 HCDR3

<400>  42

Asp Tyr Ser Gly Pro Phe Ala Tyr
1               5

<210>  43
<211>  10
<212>  PRT
<213>  Artificial Sequence

<220>
<223>  m1722 LCDR1

<400>  43

Ser Ala Ser Ser Ser Val Asn Tyr Ile His
1               5                   10

<210>  44
<211>  7
<212>  PRT
<213>  Artificial Sequence

<220>
<223>  m1722 LCDR2

<400>  44

Asp Thr Ser Lys Leu Ala Ser
1               5

<210>  45
<211>  9
<212>  PRT
<213>  Artificial Sequence

<220>
<223>  m1722 LCDR3

<400>  45

Gln Gln Trp Arg Ser Asn Pro Leu Thr
1               5

<210>  46
<211>  119
<212>  PRT
<213>  Artificial Sequence

<220>
<223>  h1718-001VH-CDR graft

<400>  46

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Asn Met Asn Trp Val Arg Gln Ala Pro Gly Gln Arg Leu Glu Trp Met
        35                  40                  45

Gly Val Ile Asn Pro Ser Tyr Gly Ile Thr Asp Tyr Asn Gln Asn Phe
    50                  55                  60

Lys Asp Arg Val Thr Ile Thr Arg Asp Thr Ser Ala Ser Thr Ala Tyr
65                  70                  75                  80

Met Glu Leu Ser Ser Leu Arg Ser Glu Asp Thr Ala Val Tyr Tyr Cys

85                  90                  95

Ala Arg Gln Leu Arg Leu Pro Gly Tyr Phe Asp Val Trp Gly Gln Gly
            100                 105                 110

Thr Thr Val Thr Val Ser Ser
        115

<210>  47
<211>  107
<212>  PRT
<213>  Artificial Sequence

<220>
<223>  h1718-001VL-CDR graft

<400>  47

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Asn Tyr
            20                  25                  30

Leu Asn Trp Phe Gln Gln Lys Pro Gly Lys Ala Pro Lys Ser Leu Ile
        35                  40                  45

Tyr Arg Ala Asn Arg Leu Val Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Lys Tyr Asp Glu Phe Pro Tyr
                85                  90                  95

Thr Phe Gly Gly Gly Thr Lys Val Glu Ile Lys
            100                 105

<210>  48
<211>  107
<212>  PRT
<213>  Artificial Sequence

<220>
<223>  h1718-VL2

<400>  48

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Asn Tyr
            20                  25                  30

Leu Asn Trp Phe Gln Gln Lys Pro Gly Lys Ala Pro Lys Thr Leu Ile
        35                  40                  45

Tyr Arg Ala Asn Arg Leu Val Asp Gly Val Pro Ser Arg Phe Ser Gly
    50                  55                  60

Ser Gly Ser Gly Gln Asp Tyr Thr Leu Thr Ile Ser Ser Leu Gln Pro
65                  70                  75                  80

Glu Asp Phe Ala Thr Tyr Tyr Cys Leu Lys Tyr Asp Glu Phe Pro Tyr
                85                  90                  95

Thr Phe Gly Gly Gly Thr Lys Val Glu Ile Lys
            100                 105

<210> 49
<211> 119
<212> PRT
<213> Artificial Sequence

<220>
<223> h1718-VH2

<400> 49

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Asn Met Asn Trp Val Arg Gln Ala Pro Gly Gln Arg Leu Glu Trp Ile
        35                  40                  45

Gly Val Ile Asn Pro Ser Tyr Gly Ile Thr Asp Tyr Asn Gln Asn Phe
    50                  55                  60

Lys Asp Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gln Leu Arg Leu Pro Gly Tyr Phe Asp Val Trp Gly Gln Gly
            100                 105                 110

Thr Thr Val Thr Val Ser Ser

        115

<210>  50
<211>  119
<212>  PRT
<213>  Artificial Sequence

<220>
<223>  h1718-VH3

<400>  50

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Ile Thr Asp Tyr
            20                  25                  30

Asn Met Asn Trp Val Arg Gln Ala Pro Gly Gln Arg Leu Glu Trp Ile
        35                  40                  45

Gly Val Ile Asn Pro Ser Tyr Gly Ile Thr Asp Tyr Asn Gln Asn Phe
    50                  55                  60

Lys Asp Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Gln Leu Arg Leu Pro Gly Tyr Phe Asp Val Trp Gly Gln Gly
            100                 105                 110

Thr Thr Val Thr Val Ser Ser
        115

<210>  51
<211>  119
<212>  PRT
<213>  Artificial Sequence

<220>
<223>  h1718-VH4

<400>  51

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Ile Thr Asp Tyr
            20                  25                  30

Asn Met Asn Trp Val Lys Gln Ala Pro Gly Gln Arg Leu Glu Trp Ile
        35                  40                  45

Gly Val Ile Asn Pro Ser Tyr Gly Ile Thr Asp Tyr Asn Gln Asn Phe
    50                  55                  60

Lys Asp Lys Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Gln Leu Arg Leu Pro Gly Tyr Phe Asp Val Trp Gly Gln Gly
            100                 105                 110

Thr Thr Val Thr Val Ser Ser
        115

<210>  52
<211>  11
<212>  PRT
<213>  Artificial Sequence

<220>
<223>  General formula of h1718-VL CDR1

<220>
<221>  UNSURE
<222>  (8)..(8)
<223>  The 'Xaa' at location 8 stands for Asn, Thr, Leu or Gln.

<400>  52

Lys Ala Ser Gln Asp Ile Asn Xaa Tyr Leu Asn
1               5                   10

<210>  53
<211>  107
<212>  PRT
<213>  Artificial Sequence

<220>
<223>  h1718.VL1a

<400>  53

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Gln Tyr
            20                  25                  30

Leu Asn Trp Phe Gln Gln Lys Pro Gly Lys Ala Pro Lys Ser Leu Ile

35                  40                  45

Tyr Arg Ala Asn Arg Leu Val Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Lys Tyr Asp Glu Phe Pro Tyr
                85                  90                  95

Thr Phe Gly Gly Gly Thr Lys Val Glu Ile Lys
            100                 105

<210>  54
<211>  107
<212>  PRT
<213>  Artificial Sequence

<220>
<223>  h1718.VL1b

<400>  54

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Thr Tyr
            20                  25                  30

Leu Asn Trp Phe Gln Gln Lys Pro Gly Lys Ala Pro Lys Ser Leu Ile
        35                  40                  45

Tyr Arg Ala Asn Arg Leu Val Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Lys Tyr Asp Glu Phe Pro Tyr
                85                  90                  95

Thr Phe Gly Gly Gly Thr Lys Val Glu Ile Lys
            100                 105

<210>  55
<211>  107
<212>  PRT
<213>  Artificial Sequence

<220>

<223>  h1718.VL1c

<400>  55

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Leu Tyr
            20                  25                  30

Leu Asn Trp Phe Gln Gln Lys Pro Gly Lys Ala Pro Lys Ser Leu Ile
        35                  40                  45

Tyr Arg Ala Asn Arg Leu Val Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Lys Tyr Asp Glu Phe Pro Tyr
                85                  90                  95

Thr Phe Gly Gly Gly Thr Lys Val Glu Ile Lys
            100                 105

<210>  56
<211>  107
<212>  PRT
<213>  Artificial Sequence

<220>
<223>  h1718.VL2a

<400>  56

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Gln Tyr
            20                  25                  30

Leu Asn Trp Phe Gln Gln Lys Pro Gly Lys Ala Pro Lys Thr Leu Ile
        35                  40                  45

Tyr Arg Ala Asn Arg Leu Val Asp Gly Val Pro Ser Arg Phe Ser Gly
    50                  55                  60

Ser Gly Ser Gly Gln Asp Tyr Thr Leu Thr Ile Ser Ser Leu Gln Pro
65                  70                  75                  80

Glu Asp Phe Ala Thr Tyr Tyr Cys Leu Lys Tyr Asp Glu Phe Pro Tyr

85                  90                  95

Thr Phe Gly Gly Gly Thr Lys Val Glu Ile Lys
            100                 105

<210>  57
<211>  107
<212>  PRT
<213>  Artificial Sequence

<220>
<223>  h1718.VL2b

<400>  57

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Thr Tyr
            20                  25                  30

Leu Asn Trp Phe Gln Gln Lys Pro Gly Lys Ala Pro Lys Thr Leu Ile
        35                  40                  45

Tyr Arg Ala Asn Arg Leu Val Asp Gly Val Pro Ser Arg Phe Ser Gly
    50                  55                  60

Ser Gly Ser Gly Gln Asp Tyr Thr Leu Thr Ile Ser Ser Leu Gln Pro
65                  70                  75                  80

Glu Asp Phe Ala Thr Tyr Tyr Cys Leu Lys Tyr Asp Glu Phe Pro Tyr
                85                  90                  95

Thr Phe Gly Gly Gly Thr Lys Val Glu Ile Lys
            100                 105

<210>  58
<211>  107
<212>  PRT
<213>  Artificial Sequence

<220>
<223>  h1718.VL2c

<400>  58

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Leu Tyr
            20                  25                  30

Leu Asn Trp Phe Gln Gln Lys Pro Gly Lys Ala Pro Lys Thr Leu Ile
        35                  40                  45

Tyr Arg Ala Asn Arg Leu Val Asp Gly Val Pro Ser Arg Phe Ser Gly
    50                  55                  60

Ser Gly Ser Gly Gln Asp Tyr Thr Leu Thr Ile Ser Ser Leu Gln Pro
65                  70                  75                  80

Glu Asp Phe Ala Thr Tyr Tyr Cys Leu Lys Tyr Asp Glu Phe Pro Tyr
                85                  90                  95

Thr Phe Gly Gly Gly Thr Lys Val Glu Ile Lys
            100                 105

<210>  59
<211>  122
<212>  PRT
<213>  Artificial Sequence

<220>
<223>  HRP00721VH-CDR graft

<400>  59

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Ser Trp Ile Arg Gln Pro Pro Gly Lys Ala Leu Glu Trp Leu
        35                  40                  45

Ala Val Ile Trp Gly Asp Gly Asn Thr Asn Tyr His Ser Val Leu Ile
    50                  55                  60

Ser Arg Leu Thr Ile Ser Lys Asp Thr Ser Lys Ser Gln Val Val Leu
65                  70                  75                  80

Thr Met Thr Asn Met Asp Pro Val Asp Thr Ala Thr Tyr Tyr Cys Ala
                85                  90                  95

Arg Asn Asn Tyr Tyr Gly Ser Arg Tyr Gly Tyr Pro Met Asp Tyr Trp
            100                 105                 110

Gly Gln Gly Thr Thr Val Thr Val Ser Ser
        115                 120

<210>  60

<211>  107
<212>  PRT
<213>  Artificial Sequence

<220>
<223>  HRP00721VL-CDR graft

<400>  60

Asp Ile Gln Met Thr Gln Ser Pro Ser Ser Val Ser Ala Ser Val Gly
1               5                   10                  15

Asp Arg Val Thr Ile Thr Cys Lys Ala Thr Gln Asp Val Gly Thr Ala
            20                  25                  30

Val Ala Trp Tyr Gln Gln Lys Pro Gly Lys Ala Pro Lys Leu Leu Ile
        35                  40                  45

Tyr Trp Ala Ser Thr Arg His Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Gly Ser Ser Val Leu
                85                  90                  95

Thr Phe Gly Gly Gly Thr Lys Val Glu Ile Lys
            100                 105


<210>  61
<211>  107
<212>  PRT
<213>  Artificial Sequence

<220>
<223>  h1719-VL2

<400>  61

Asp Ile Gln Met Thr Gln Ser Pro Ser Ser Val Ser Ala Ser Val Gly
1               5                   10                  15

Asp Arg Val Thr Ile Thr Cys Lys Ala Thr Gln Asp Val Gly Thr Ala
            20                  25                  30

Val Ala Trp Tyr Gln Gln Lys Pro Gly Lys Ser Pro Lys Leu Leu Ile
        35                  40                  45

Tyr Trp Ala Ser Thr Arg His Thr Gly Val Pro Glu Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Gly Ser Ser Val Leu
                85                  90                  95

Thr Phe Gly Gly Gly Thr Lys Val Glu Ile Lys
            100                 105

<210>  62
<211>  122
<212>  PRT
<213>  Artificial Sequence

<220>
<223>  h1719-VH2

<400>  62

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Ser Trp Val Arg Gln Pro Pro Gly Lys Ala Leu Glu Trp Leu
        35                  40                  45

Ala Val Ile Trp Gly Asp Gly Asn Thr Asn Tyr His Ser Val Leu Ile
    50                  55                  60

Ser Arg Leu Thr Ile Ser Lys Asp Thr Ser Lys Ser Gln Val Val Leu
65                  70                  75                  80

Thr Met Thr Asn Met Asp Pro Val Asp Thr Ala Thr Tyr Tyr Cys Ala
                85                  90                  95

Lys Asn Asn Tyr Tyr Gly Ser Arg Tyr Gly Tyr Pro Met Asp Tyr Trp
            100                 105                 110

Gly Gln Gly Thr Thr Val Thr Val Ser Ser
        115                 120

<210>  63
<211>  122
<212>  PRT
<213>  Artificial Sequence

<220>
<223>  h1719-VH3

<400>  63

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Ser Trp Val Arg Gln Pro Pro Gly Lys Gly Leu Glu Trp Leu
        35                  40                  45

Gly Val Ile Trp Gly Asp Gly Asn Thr Asn Tyr His Ser Val Leu Ile
    50                  55                  60

Ser Arg Leu Thr Ile Ser Lys Asp Thr Ser Lys Ser Gln Val Val Leu
65                  70                  75                  80

Thr Leu Thr Asn Met Asp Pro Val Asp Thr Ala Thr Tyr Tyr Cys Ala
                85                  90                  95

Lys Asn Asn Tyr Tyr Gly Ser Arg Tyr Gly Tyr Pro Met Asp Tyr Trp
            100                 105                 110

Gly Gln Gly Thr Thr Val Thr Val Ser Ser
        115                 120

<210>  64
<211>  14
<212>  PRT
<213>  Artificial Sequence

<220>
<223>  General formula of h1719-VH CDR3

<220>
<221>  UNSURE
<222>  (1)..(1)
<223>  The 'Xaa' at location 1 stands for Gln or Asn.

<220>
<221>  UNSURE
<222>  (2)..(2)
<223>  The 'Xaa' at location 2 stands for Gln, Asn, or Tyr.

<220>
<221>  UNSURE
<222>  (12)..(12)
<223>  The 'Xaa' at location 12 stands for Met, Leu, or Ile.

<400>  64

Xaa Xaa Tyr Tyr Gly Ser Arg Tyr Gly Tyr Pro Xaa Asp Tyr
1               5                   10

<210>  65
<211>  122
<212>  PRT
<213>  Artificial Sequence

<220>
<223>  h1719.VH3a

<400>  65

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Ser Trp Val Arg Gln Pro Pro Gly Lys Gly Leu Glu Trp Leu
        35                  40                  45

Gly Val Ile Trp Gly Asp Gly Asn Thr Asn Tyr His Ser Val Leu Ile
    50                  55                  60

Ser Arg Leu Thr Ile Ser Lys Asp Thr Ser Lys Ser Gln Val Val Leu
65                  70                  75                  80

Thr Leu Thr Asn Met Asp Pro Val Asp Thr Ala Thr Tyr Tyr Cys Ala
                85                  90                  95

Lys Gln Asn Tyr Tyr Gly Ser Arg Tyr Gly Tyr Pro Leu Asp Tyr Trp
            100                 105                 110

Gly Gln Gly Thr Thr Val Thr Val Ser Ser
        115                 120


<210>  66
<211>  122
<212>  PRT
<213>  Artificial Sequence

<220>
<223>  h1719.VH3b

<400>  66

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Ser Trp Val Arg Gln Pro Pro Gly Lys Gly Leu Glu Trp Leu
        35                  40                  45

Gly Val Ile Trp Gly Asp Gly Asn Thr Asn Tyr His Ser Val Leu Ile
    50                  55                  60

Ser Arg Leu Thr Ile Ser Lys Asp Thr Ser Lys Ser Gln Val Val Leu
65                  70                  75                  80

Thr Leu Thr Asn Met Asp Pro Val Asp Thr Ala Thr Tyr Tyr Cys Ala
                85                  90                  95

Lys Asn Tyr Tyr Tyr Gly Ser Arg Tyr Gly Tyr Pro Leu Asp Tyr Trp
            100                 105                 110

Gly Gln Gly Thr Thr Val Thr Val Ser Ser
        115                 120

<210>  67
<211>  122
<212>  PRT
<213>  Artificial Sequence

<220>
<223>  h1719.VH3c

<400>  67

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Ser Trp Val Arg Gln Pro Pro Gly Lys Gly Leu Glu Trp Leu
        35                  40                  45

Gly Val Ile Trp Gly Asp Gly Asn Thr Asn Tyr His Ser Val Leu Ile
    50                  55                  60

Ser Arg Leu Thr Ile Ser Lys Asp Thr Ser Lys Ser Gln Val Val Leu
65                  70                  75                  80

Thr Leu Thr Asn Met Asp Pro Val Asp Thr Ala Thr Tyr Tyr Cys Ala
                85                  90                  95

Lys Asn Gln Tyr Tyr Gly Ser Arg Tyr Gly Tyr Pro Leu Asp Tyr Trp
            100                 105                 110

Gly Gln Gly Thr Thr Val Thr Val Ser Ser
        115                 120

<210>  68
<211>  122
<212>  PRT
<213>  Artificial Sequence

<220>
<223>  h1719.VH3d

<400>  68

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Ser Trp Val Arg Gln Pro Pro Gly Lys Gly Leu Glu Trp Leu
        35                  40                  45

Gly Val Ile Trp Gly Asp Gly Asn Thr Asn Tyr His Ser Val Leu Ile
    50                  55                  60

Ser Arg Leu Thr Ile Ser Lys Asp Thr Ser Lys Ser Gln Val Val Leu
65                  70                  75                  80

Thr Leu Thr Asn Met Asp Pro Val Asp Thr Ala Thr Tyr Tyr Cys Ala
                85                  90                  95

Lys Gln Asn Tyr Tyr Gly Ser Arg Tyr Gly Tyr Pro Ile Asp Tyr Trp
            100                 105                 110

Gly Gln Gly Thr Thr Val Thr Val Ser Ser
        115                 120


<210>  69
<211>  122
<212>  PRT
<213>  Artificial Sequence

<220>
<223>  h1719.VH3e

<400>  69

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Ser Trp Val Arg Gln Pro Pro Gly Lys Gly Leu Glu Trp Leu
        35                  40                  45

Gly Val Ile Trp Gly Asp Gly Asn Thr Asn Tyr His Ser Val Leu Ile
    50                  55                  60

Ser Arg Leu Thr Ile Ser Lys Asp Thr Ser Lys Ser Gln Val Val Leu
65                  70                  75                  80

Thr Leu Thr Asn Met Asp Pro Val Asp Thr Ala Thr Tyr Tyr Cys Ala
                85                  90                  95

Lys Asn Tyr Tyr Tyr Gly Ser Arg Tyr Gly Tyr Pro Ile Asp Tyr Trp
            100                 105                 110

Gly Gln Gly Thr Thr Val Thr Val Ser Ser
        115                 120

<210>  70
<211>  122
<212>  PRT
<213>  Artificial Sequence

<220>
<223>  h1719.VH3f

<400>  70

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Ser Trp Val Arg Gln Pro Pro Gly Lys Gly Leu Glu Trp Leu
        35                  40                  45

Gly Val Ile Trp Gly Asp Gly Asn Thr Asn Tyr His Ser Val Leu Ile
    50                  55                  60

Ser Arg Leu Thr Ile Ser Lys Asp Thr Ser Lys Ser Gln Val Val Leu
65                  70                  75                  80

Thr Leu Thr Asn Met Asp Pro Val Asp Thr Ala Thr Tyr Tyr Cys Ala
                85                  90                  95

Lys Asn Gln Tyr Tyr Gly Ser Arg Tyr Gly Tyr Pro Ile Asp Tyr Trp
            100                 105                 110

Gly Gln Gly Thr Thr Val Thr Val Ser Ser
        115                 120

<210>  71
<211>  122
<212>  PRT
<213>  Artificial Sequence

<220>
<223>  h1721-001 VH-CDR graft

<400>  71

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Asn Trp Ile Arg Gln Pro Pro Gly Lys Ala Leu Glu Trp Leu
        35                  40                  45

Ala Val Ile Trp Gly Asp Gly Ser Thr Asn Tyr His Ser Ala Leu Ile
    50                  55                  60

Ser Arg Leu Thr Ile Ser Lys Asp Thr Ser Lys Ser Gln Val Val Leu
65                  70                  75                  80

Thr Met Thr Asn Met Asp Pro Val Asp Thr Ala Thr Tyr Tyr Cys Ala
                85                  90                  95

Arg Asn Tyr Phe Tyr Gly Ser Arg Tyr Gly Tyr Ala Met Asp Ser Trp
            100                 105                 110

Gly Gln Gly Thr Thr Val Thr Val Ser Ser
        115                 120


<210>  72
<211>  107
<212>  PRT
<213>  Artificial Sequence

<220>
<223>  h1721-001 VL-CDR graft

<400>  72

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Tyr Ser Ser
            20                  25                  30

Leu Ala Trp Tyr Gln Gln Lys Pro Gly Lys Ala Pro Lys Leu Leu Ile
        35                  40                  45

Tyr Asn Ala Lys Thr Leu Ile Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Gly Thr Pro Tyr
                85                  90                  95

Thr Phe Gly Gly Gly Thr Lys Val Glu Ile Lys
            100                 105

<210>  73
<211>  107
<212>  PRT
<213>  Artificial Sequence

<220>
<223>  h1721-VL2

<400>  73

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Tyr Ser Ser
            20                  25                  30

Leu Ala Trp Tyr Gln Gln Lys Pro Gly Lys Ala Pro Lys Leu Leu Val
        35                  40                  45

Tyr Asn Ala Lys Thr Leu Ile Glu Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln His His Tyr Gly Thr Pro Tyr
                85                  90                  95

Thr Phe Gly Gly Gly Thr Lys Val Glu Ile Lys
            100                 105

<210>  74
<211>  107
<212>  PRT
<213>  Artificial Sequence

<220>
<223>  h1721-VL3

<400>  74

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asn Ile Tyr Ser Ser
            20                  25                  30

Leu Ala Trp Tyr Gln Gln Lys Pro Gly Lys Ser Pro Lys Leu Leu Val
        35                  40                  45

Tyr Asn Ala Lys Thr Leu Ile Glu Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln His His Tyr Gly Thr Pro Tyr
                85                  90                  95

Thr Phe Gly Gly Gly Thr Lys Val Glu Ile Lys
            100                 105


<210>  75
<211>  122
<212>  PRT
<213>  Artificial Sequence

<220>
<223>  h1721-VH2

<400>  75

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Asn Trp Ile Arg Gln Pro Pro Gly Lys Ala Leu Glu Trp Leu
        35                  40                  45

Gly Val Ile Trp Gly Asp Gly Ser Thr Asn Tyr His Ser Ala Leu Ile
    50                  55                  60

Ser Arg Leu Thr Ile Ser Lys Asp Thr Ser Lys Ser Gln Val Val Leu
65                  70                  75                  80

Thr Met Thr Asn Met Asp Pro Val Asp Thr Ala Thr Tyr Tyr Cys Ala
                85                  90                  95

Lys Asn Tyr Phe Tyr Gly Ser Arg Tyr Gly Tyr Ala Met Asp Ser Trp
            100                 105                 110

Gly Gln Gly Thr Thr Val Thr Val Ser Ser
        115                 120

<210> 76
<211> 122
<212> PRT
<213> Artificial Sequence

<220>
<223> h1721-VH3

<400> 76

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Asn Trp Ile Arg Gln Pro Pro Gly Lys Ala Leu Glu Trp Leu
        35                  40                  45

Gly Val Ile Trp Gly Asp Gly Ser Thr Asn Tyr His Ser Ala Leu Ile
    50                  55                  60

Ser Arg Leu Thr Ile Ser Lys Asp Thr Ser Lys Ser Gln Val Val Leu
65                  70                  75                  80

Thr Leu Thr Asn Met Asp Pro Val Asp Thr Ala Thr Tyr Tyr Cys Ala
                85                  90                  95

Lys Asn Tyr Phe Tyr Gly Ser Arg Tyr Gly Tyr Ala Met Asp Ser Trp
            100                 105                 110

Gly Gln Gly Thr Thr Val Thr Val Ser Ser
        115                 120

<210> 77
<211> 122
<212> PRT
<213> Artificial Sequence

<220>
<223> h1721-VH4

<400> 77

Glu Val Thr Leu Lys Glu Ser Gly Pro Val Leu Val Lys Pro Thr Glu
1               5                   10                  15

Thr Leu Thr Leu Thr Cys Thr Val Ser Gly Phe Ser Leu Ser Ser Tyr
            20                  25                  30

Gly Val Asn Trp Val Arg Gln Pro Pro Gly Lys Gly Leu Glu Trp Leu
        35                  40                  45

Gly Val Ile Trp Gly Asp Gly Ser Thr Asn Tyr His Ser Ala Leu Ile
    50                  55                  60

Ser Arg Leu Thr Ile Ser Lys Asp Thr Ser Lys Ser Gln Val Val Leu
65                  70                  75                  80

Thr Leu Thr Asn Met Asp Pro Val Asp Thr Ala Thr Tyr Tyr Cys Ala
                85                  90                  95

Lys Asn Tyr Phe Tyr Gly Ser Arg Tyr Gly Tyr Ala Met Asp Ser Trp
            100                 105                 110

Gly Gln Gly Thr Thr Val Thr Val Ser Ser
        115                 120

<210> 78
<211> 117
<212> PRT
<213> Artificial Sequence

<220>
<223> h1722-001 VH-CDR graft

<400> 78

Glu Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asp Tyr
            20                  25                  30

Trp Met His Trp Val Arg Gln Ala Pro Gly Gln Gly Leu Glu Trp Met
        35                  40                  45

Gly Met Leu His Pro Asn Ser Gly Thr Thr Ser Phe Asn Glu Lys Phe
    50                  55                  60

Lys Ile Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Tyr Ser Gly Pro Phe Ala Tyr Trp Gly Gln Gly Thr Leu
            100                 105                 110

Val Thr Val Ser Ser
        115

<210>  79
<211>  106
<212>  PRT
<213>  Artificial Sequence

<220>
<223>  h1722-001 VL-CDR graft

<400>  79

Asp Ile Gln Met Thr Gln Ser Pro Ser Ser Leu Ser Ala Ser Val Gly
1               5                   10                  15

Asp Arg Val Thr Ile Thr Cys Ser Ala Ser Ser Ser Val Asn Tyr Ile
            20                  25                  30

His Trp Tyr Gln Gln Lys Pro Gly Lys Ala Pro Lys Leu Leu Ile Tyr
        35                  40                  45

Asp Thr Ser Lys Leu Ala Ser Gly Val Pro Ser Arg Phe Ser Gly Ser
    50                  55                  60

Gly Ser Gly Thr Asp Phe Thr Leu Thr Ile Ser Ser Leu Gln Pro Glu
65                  70                  75                  80

Asp Phe Ala Thr Tyr Tyr Cys Gln Gln Trp Arg Ser Asn Pro Leu Thr
                85                  90                  95

Phe Gly Gln Gly Thr Lys Leu Glu Ile Lys
            100                 105

<210>  80
<211>  106
<212>  PRT
<213>  Artificial Sequence

<220>
<223>  h1722-VL2

<400>  80

Asp Ile Gln Met Thr Gln Ser Pro Ser Ser Leu Ser Ala Ser Val Gly
1               5                   10                  15

Asp Arg Val Thr Ile Thr Cys Ser Ala Ser Ser Ser Val Asn Tyr Ile
            20                  25                  30

His Trp Tyr Gln Gln Lys Pro Gly Lys Ala Pro Lys Arg Trp Ile Tyr
        35                  40                  45

Asp Thr Ser Lys Leu Ala Ser Gly Val Pro Ser Arg Phe Ser Gly Ser
    50                  55                  60

Gly Ser Gly Thr Asp Phe Thr Leu Thr Ile Ser Ser Leu Gln Pro Glu
65                  70                  75                  80

Asp Phe Ala Thr Tyr Tyr Cys Gln Gln Trp Arg Ser Asn Pro Leu Thr
                85                  90                  95

Phe Gly Gln Gly Thr Lys Leu Glu Ile Lys
            100                 105

<210> 81
<211> 106
<212> PRT
<213> Artificial Sequence

<220>
<223> h1722-VL3

<400> 81

Asp Ile Gln Leu Thr Gln Ser Pro Ser Ser Leu Ser Ala Ser Val Gly
1               5                   10                  15

Asp Arg Val Thr Ile Thr Cys Ser Ala Ser Ser Ser Val Asn Tyr Ile
            20                  25                  30

His Trp Tyr Gln Gln Lys Pro Gly Lys Ala Pro Lys Arg Trp Ile Phe
        35                  40                  45

Asp Thr Ser Lys Leu Ala Ser Gly Val Pro Ser Arg Phe Ser Gly Ser
    50                  55                  60

Gly Ser Gly Thr Asp Tyr Thr Leu Thr Ile Ser Ser Leu Gln Pro Glu
65                  70                  75                  80

Asp Phe Ala Thr Tyr Tyr Cys Gln Gln Trp Arg Ser Asn Pro Leu Thr
                85                  90                  95

Phe Gly Gln Gly Thr Lys Leu Glu Ile Lys
            100                 105

<210> 82
<211> 117
<212> PRT

<213>  Artificial Sequence

<220>
<223>  h1722-VH2

<400>  82

Glu Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asp Tyr
            20                  25                  30

Trp Met His Trp Val Arg Gln Ala Pro Gly Gln Gly Leu Glu Trp Ile
        35                  40                  45

Gly Met Leu His Pro Asn Ser Gly Thr Thr Ser Phe Asn Glu Lys Phe
    50                  55                  60

Lys Ile Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ser Asp Tyr Ser Gly Pro Phe Ala Tyr Trp Gly Gln Gly Thr Leu
            100                 105                 110

Val Thr Val Ser Ser
        115


<210>  83
<211>  117
<212>  PRT
<213>  Artificial Sequence

<220>
<223>  h1722-VH3

<400>  83

Glu Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asp Tyr
            20                  25                  30

Trp Met His Trp Val Lys Gln Ala Pro Gly Gln Gly Leu Glu Trp Ile
        35                  40                  45

Gly Met Leu His Pro Asn Ser Gly Thr Thr Ser Phe Asn Glu Lys Phe
    50                  55                  60

Lys Ile Arg Val Thr Ile Thr Ile Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ser Asp Tyr Ser Gly Pro Phe Ala Tyr Trp Gly Gln Gly Thr Leu
            100                 105                 110

Val Thr Val Ser Ser
        115

<210>  84
<211>  117
<212>  PRT
<213>  Artificial Sequence

<220>
<223>  h1722-VH4

<400>  84

Glu Val Gln Leu Val Gln Ser Gly Ala Glu Val Lys Lys Pro Gly Ser
1               5                   10                  15

Ser Val Lys Val Ser Cys Lys Ala Ser Gly Asn Thr Phe Ser Asp Tyr
            20                  25                  30

Trp Met His Trp Val Lys Gln Ala Pro Gly Gln Gly Leu Glu Trp Ile
        35                  40                  45

Gly Met Leu His Pro Asn Ser Gly Thr Thr Ser Phe Asn Glu Lys Phe
    50                  55                  60

Lys Ile Arg Thr Thr Ile Thr Ile Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ser Asp Tyr Ser Gly Pro Phe Ala Tyr Trp Gly Gln Gly Thr Leu
            100                 105                 110

Val Thr Val Ser Ser
        115

<210>  85
<211>  20
<212>  PRT

<213>  Artificial Sequence

<220>
<223>  positions 17-36 of CD96 extracellular region

<400>  85

Gly Ser Asp Val Asn Leu Thr Cys Gln Thr Gln Thr Val Gly Phe Phe
1               5                   10                  15

Val Gln Met Gln
            20

<210>  86
<211>  20
<212>  PRT
<213>  Artificial Sequence

<220>
<223>  positions 32-51 of CD96 extracellular region

<400>  86

Phe Val Gln Met Gln Trp Ser Lys Val Thr Asn Lys Ile Asp Leu Ile
1               5                   10                  15

Ala Val Tyr His
            20

<210>  87
<211>  20
<212>  PRT
<213>  Artificial Sequence

<220>
<223>  positions 47-66 of CD96 extracellular region

<400>  87

Ile Ala Val Tyr His Pro Gln Tyr Gly Phe Tyr Cys Ala Tyr Gly Arg
1               5                   10                  15

Pro Cys Glu Ser
            20

<210>  88
<211>  20
<212>  PRT
<213>  Artificial Sequence

<220>
<223>  positions 62-81 of CD96 extracellular region

<400>  88

Arg Pro Cys Glu Ser Leu Val Thr Phe Thr Glu Thr Pro Glu Asn Gly
1               5                   10                  15

Ser Lys Trp Thr
            20

<210>  89
<211>  20
<212>  PRT
<213>  Artificial Sequence

<220>
<223>  positions 77-86 of CD96 extracellular region

<400>  89

Gly Ser Lys Trp Thr Leu His Leu Arg Asn Met Ser Cys Ser Val Ser
1               5                   10                  15

Gly Arg Tyr Glu
            20

<210>  90
<211>  20
<212>  PRT
<213>  Artificial Sequence

<220>
<223>  positions 92-111 of CD96 extracellular region

<400>  90

Ser Gly Arg Tyr Glu Cys Met Leu Val Leu Tyr Pro Glu Gly Ile Gln
1               5                   10                  15

Thr Lys Ile Tyr
            20

<210>  91
<211>  20
<212>  PRT
<213>  Artificial Sequence

<220>
<223>  positions 107-126 of CD96 extracellular region

<400>  91

Gln Thr Lys Ile Tyr Asn Leu Leu Ile Gln Thr His Val Thr Ala Asp
1               5                   10                  15

Glu Trp Asn Ser
            20

<210>  92
<211>  20
<212>  PRT

<213>  Artificial Sequence

<220>
<223>  positions 122-141 of CD96 extracellular region

<400>  92

Asp Glu Trp Asn Ser Asn His Thr Ile Glu Ile Glu Ile Asn Gln Thr
1               5                   10                  15

Leu Glu Ile Pro
            20

<210>  93
<211>  20
<212>  PRT
<213>  Artificial Sequence

<220>
<223>  positions 137-156 of CD96 extracellular region

<400>  93

Thr Leu Glu Ile Pro Cys Phe Gln Asn Ser Ser Ser Lys Ile Ser Ser
1               5                   10                  15

Glu Phe Thr Tyr
            20

<210>  94
<211>  22
<212>  PRT
<213>  Artificial Sequence

<220>
<223>  positions 149-170 of CD96 extracellular region

<400>  94

Lys Ile Ser Ser Glu Phe Thr Tyr Ala Trp Ser Val Glu Asp Asn Gly
1               5                   10                  15

Thr Gln Glu Thr Leu Ile
            20

<210>  95
<211>  523
<212>  PRT
<213>  Artificial Sequence

<220>
<223>  Monkey CD96-3Ã\227Flag

<400>  95

Met Glu Phe Gly Leu Ser Trp Leu Phe Leu Val Ala Ile Leu Lys Gly
1               5                   10                  15

Val Gln Cys Val Trp Gly Lys Pro Phe Asn Thr Glu Glu Asn Ile Tyr
            20                  25                  30

Ala Thr Leu Gly Ser Asp Val Asn Leu Thr Cys Gln Thr Gln Ala Lys
        35                  40                  45

Gly Phe Leu Val Gln Met Gln Trp Ser Lys Val Thr Asp Lys Ala Asp
    50                  55                  60

Leu Ile Ala Leu Tyr His Pro Gln Tyr Gly Phe His Cys Ala Tyr Gly
65                  70                  75                  80

Ser Pro Cys Glu Ser Leu Val Thr Phe Thr Gln Thr Pro Glu Asn Gly
                85                  90                  95

Ser Lys Trp Thr Leu His Leu Arg Asn Met Ser Ser Ser Val Ser Gly
            100                 105                 110

Arg Tyr Glu Cys Met Leu Thr Leu Tyr Pro Glu Gly Met Gln Thr Lys
        115                 120                 125

Ile Tyr Asn Leu Leu Ile Gln Thr His Val Thr Pro Asp Glu Trp Lys
    130                 135                 140

Ser Asn His Thr Ile Glu Ile Glu Ile Asn Gln Thr Leu Glu Ile Pro
145                 150                 155                 160

Cys Phe Gln Asn Ser Ser Ser Glu Ile Ser Ser Glu Phe Thr Tyr Ala
                165                 170                 175

Trp Leu Val Glu Asp Asn Gly Thr Gln Gln Thr Leu Ile Ser Gln Asp
            180                 185                 190

His Leu Ile Ser Ser Ser Thr Leu Leu Lys Asp Arg Val Lys Val Gly
        195                 200                 205

Ile Asp Tyr Arg Leu His Leu Ser Pro Val Gln Ile Phe Asp Asp Gly
    210                 215                 220

Arg Lys Phe Ser Cys His Ile Arg Val Gly Pro Asp Lys Ile Leu Arg
225                 230                 235                 240

Ser Ser Thr Thr Ile Lys Val Phe Ala Lys Pro Glu Ile Pro Met Ile
                245                 250                 255

Val Glu Asn Asn Ser Thr Asp Val Leu Val Glu Arg Thr Phe Thr Cys

260                 265                 270

Leu Leu Lys Asn Val Phe Pro Lys Ala Asn Ile Ile Trp Phe Ile Asp
        275                 280                 285

Gly Ser Phe Leu His Asp Glu Lys Glu Gly Ile Tyr Ile Thr Asn Glu
    290                 295                 300

Glu Arg Lys Gly Lys Asp Gly Phe Leu Glu Leu Lys Ser Val Leu Thr
305                 310                 315                 320

Arg Val His Ser Asp Lys Pro Ala Gln Ser Asp Asn Leu Thr Ile Trp
                325                 330                 335

Cys Met Ala Leu Ser Pro Val Pro Gly Asn Lys Val Trp Asn Ile Ser
            340                 345                 350

Ser Glu Lys Ile Thr Phe Leu Leu Gly Ser Glu Met Ser Thr Thr Asp
        355                 360                 365

Leu Pro Pro Ser Val Thr Glu Ser Thr Leu Asp Thr Gln Pro Ser Pro
    370                 375                 380

Ala Ser Ser Val Ser Pro Thr Arg Tyr Pro Ala Thr Ser Ser Val Thr
385                 390                 395                 400

Leu Ala Asp Val Ser Ala Leu Arg Pro Asn Thr Thr Pro Gln Ser Ser
                405                 410                 415

Ser Ser Ser Val Thr Thr Gln Asp Phe Asn Tyr Pro Trp Thr Ser Ser
            420                 425                 430

Gly Thr Asp Ala Lys Lys Ser Phe Ser Gln Ile Pro Ser Glu Thr Tyr
        435                 440                 445

Ser Ser Ser Pro Ser Gly Ala Gly Ser Thr Leu His Asp Asn Val Phe
    450                 455                 460

Thr Ser Thr Thr Arg Ala Leu Ser Glu Val Pro Thr Thr Ala Asn Gly
465                 470                 475                 480

Ser Thr Lys Thr Asn His Val His Ile Thr Gly Ile Val Val Ser Lys
                485                 490                 495

Pro Lys Asp Gly Met Asp Tyr Lys Asp His Asp Gly Asp Tyr Lys Asp
            500                 505                 510

His Asp Ile Asp Tyr Lys Asp Asp Asp Asp Lys
        515                 520

<210>  96
<211>  20
<212>  PRT
<213>  Artificial Sequence

<220>
<223>  positions 166-185 of CD96 extracellular region

<400>  96

Gln Glu Thr Leu Ile Ser Gln Asn His Leu Ile Ser Asn Ser Thr Leu
1               5                   10                  15

Leu Lys Asp Arg
            20

<210>  97
<211>  20
<212>  PRT
<213>  Artificial Sequence

<220>
<223>  positions 181-200 of CD96 extracellular region

<400>  97

Leu Leu Lys Asp Arg Val Lys Leu Gly Thr Asp Tyr Arg Leu His Leu
1               5                   10                  15

Ser Pro Val Gln
            20

<210>  98
<211>  20
<212>  PRT
<213>  Artificial Sequence

<220>
<223>  positions 196-215 of CD96 extracellular region

<400>  98

Leu Ser Pro Val Gln Ile Phe Asp Asp Gly Arg Lys Phe Ser Cys His
1               5                   10                  15

Ile Arg Val Gly
            20

<210>  99
<211>  20
<212>  PRT
<213>  Artificial Sequence

<220>
<223>  positions 211-230 of CD96 extracellular region

<400>  99

His Ile Arg Val Gly Pro Asn Lys Ile Leu Arg Ser Ser Thr Thr Val
1               5                   10                  15

Lys Val Phe Ala
            20

<210>  100
<211>  20
<212>  PRT
<213>  Artificial Sequence

<220>
<223>  positions 220-239 of CD96 extracellular region

<400>  100

Leu Arg Ser Ser Thr Thr Val Lys Val Phe Ala Lys Pro Glu Ile Pro
1               5                   10                  15

Val Ile Val Glu
            20

<210>  101
<211>  20
<212>  PRT
<213>  Artificial Sequence

<220>
<223>  positions 235-254 of CD96 extracellular region

<400>  101

Pro Val Ile Val Glu Asn Asn Ser Thr Asp Val Leu Val Glu Arg Arg
1               5                   10                  15

Phe Thr Cys Leu
            20

<210>  102
<211>  20
<212>  PRT
<213>  Artificial Sequence

<220>
<223>  positions 250-269 of CD96 extracellular region

<400>  102

Arg Phe Thr Cys Leu Leu Lys Asn Val Phe Pro Lys Ala Asn Ile Thr
1               5                   10                  15

Trp Phe Ile Asp
            20

<210>  103
<211>  20
<212>  PRT
<213>  Artificial Sequence

<220>
<223>  positions 265-284 of CD96 extracellular region

<400>  103

Thr Trp Phe Ile Asp Gly Ser Phe Leu His Asp Glu Lys Glu Gly Ile
1               5                   10                  15

Tyr Ile Thr Asn
            20

<210>  104
<211>  20
<212>  PRT
<213>  Artificial Sequence

<220>
<223>  positions 280-299 of CD96 extracellular region

<400>  104

Ile Tyr Ile Thr Asn Glu Glu Arg Lys Gly Lys Asp Gly Phe Leu Glu
1               5                   10                  15

Leu Lys Ser Val
            20

<210>  105
<211>  20
<212>  PRT
<213>  Artificial Sequence

<220>
<223>  positions 295-314 of CD96 extracellular region

<400>  105

Glu Leu Lys Ser Val Leu Thr Arg Val His Ser Asn Lys Pro Ala Gln
1               5                   10                  15

Ser Asp Asn Leu
            20

<210>  106
<211>  20
<212>  PRT
<213>  Artificial Sequence

<220>
<223>  positions 310-329 of CD96 extracellular region

<400>  106

Gln Ser Asp Asn Leu Thr Ile Trp Cys Met Ala Leu Ser Pro Val Pro
1               5                   10                  15

Gly Asn Lys Val
            20

<210>  107
<211>  20
<212>  PRT
<213>  Artificial Sequence

<220>
<223>  positions 325-344 of CD96 extracellular region

<400>  107

Pro Gly Asn Lys Val Trp Asn Ile Ser Ser Glu Lys Ile Thr Phe Leu
1               5                   10                  15

Leu Gly Ser Glu
            20

<210>  108
<211>  11
<212>  PRT
<213>  Artificial Sequence

<220>
<223>  h1718-VL CDR1 mutant

<400>  108

Lys Ala Ser Gln Asp Ile Asn Gln Tyr Leu Asn
1               5                   10

<210>  109
<211>  11
<212>  PRT
<213>  Artificial Sequence

<220>
<223>  h1718-VL CDR1 mutant

<400>  109

Lys Ala Ser Gln Asp Ile Asn Thr Tyr Leu Asn
1               5                   10

<210>  110
<211>  11
<212>  PRT
<213>  Artificial Sequence

<220>
<223>  h1718-VL CDR1 mutant

<400>  110

Lys Ala Ser Gln Asp Ile Asn Leu Tyr Leu Asn
1               5                   10


<210>  111
<211>  14
<212>  PRT
<213>  Artificial Sequence

<220>
<223>  h1719-VH CDR3 mutant

<400>  111

Gln Asn Tyr Tyr Gly Ser Arg Tyr Gly Tyr Pro Leu Asp Tyr
1               5                   10


<210>  112
<211>  14
<212>  PRT
<213>  Artificial Sequence

<220>
<223>  h1719-VH CDR3 mutant

<400>  112

Asn Tyr Tyr Tyr Gly Ser Arg Tyr Gly Tyr Pro Leu Asp Tyr
1               5                   10


<210>  113
<211>  14
<212>  PRT
<213>  Artificial Sequence

<220>
<223>  h1719-VH CDR3 mutant

<400>  113

Asn Gln Tyr Tyr Gly Ser Arg Tyr Gly Tyr Pro Leu Asp Tyr
1               5                   10


<210>  114
<211>  14
<212>  PRT
<213>  Artificial Sequence

<220>
<223>  h1719-VH CDR3 mutant

<400>  114

Gln Asn Tyr Tyr Gly Ser Arg Tyr Gly Tyr Pro Ile Asp Tyr

1               5                   10


<210>  115
<211>  14
<212>  PRT
<213>  Artificial Sequence

<220>
<223>  h1719-VH CDR3 mutant

<400>  115

Asn Tyr Tyr Tyr Gly Ser Arg Tyr Gly Tyr Pro Ile Asp Tyr
1               5                   10


<210>  116
<211>  14
<212>  PRT
<213>  Artificial Sequence

<220>
<223>  h1719-VH CDR3 mutant

<400>  116

Asn Gln Tyr Tyr Gly Ser Arg Tyr Gly Tyr Pro Ile Asp Tyr
1               5                   10


<210>  117
<211>  327
<212>  PRT
<213>  Artificial Sequence

<220>
<223>  Heavy chain constant region

<400>  117

Ala Ser Thr Lys Gly Pro Ser Val Phe Pro Leu Ala Pro Cys Ser Arg
1               5                   10                  15


Ser Thr Ser Glu Ser Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
    50                  55                  60


Leu Ser Ser Val Val Thr Val Pro Ser Ser Ser Leu Gly Thr Lys Thr
65                  70                  75                  80


Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210> 118

<211>  107
<212>  PRT
<213>  Artificial Sequence

<220>
<223>  Light chain constant region

<400>  118


<210>  119
<211>  17
<212>  PRT
<213>  Artificial Sequence

<220>
<223>  General formula of h1722-VH CDR2


<220>
<221>  UNSURE
<222>  (5)..(5)

<400>  119

Met Leu His Pro Xaa Ser Gly Thr Thr Ser Phe Asn Glu Lys Phe Lys
1               5                   10                  15


Ile



<210>  120
<211>  17
<212>  PRT

<213>  Artificial Sequence

<220>
<223>  h1722-VH CDR2 mutant

<400>  120

Met Leu His Pro Lys Ser Gly Thr Thr Ser Phe Asn Glu Lys Phe Lys
1               5                   10                  15

Ile

<210>  121
<211>  17
<212>  PRT
<213>  Artificial Sequence

<220>
<223>  h1722-VH CDR2 mutant

<400>  121

Met Leu His Pro Gln Ser Gly Thr Thr Ser Phe Asn Glu Lys Phe Lys
1               5                   10                  15

Ile

<210>  122
<211>  117
<212>  PRT
<213>  Artificial Sequence

<220>
<223>  h1722-VH2a (N54K)

<400>  122

Glu Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asp Tyr
            20                  25                  30

Trp Met His Trp Val Arg Gln Ala Pro Gly Gln Gly Leu Glu Trp Ile
        35                  40                  45

Gly Met Leu His Pro Lys Ser Gly Thr Thr Ser Phe Asn Glu Lys Phe
    50                  55                  60

Lys Ile Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ser Asp Tyr Ser Gly Pro Phe Ala Tyr Trp Gly Gln Gly Thr Leu
            100                 105                 110

Val Thr Val Ser Ser
        115

<210>  123
<211>  117
<212>  PRT
<213>  Artificial Sequence

<220>
<223>  h1722-VH2b (N54Q)

<400>  123

Glu Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asp Tyr
            20                  25                  30

Trp Met His Trp Val Arg Gln Ala Pro Gly Gln Gly Leu Glu Trp Ile
        35                  40                  45

Gly Met Leu His Pro Gln Ser Gly Thr Thr Ser Phe Asn Glu Lys Phe
    50                  55                  60

Lys Ile Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ser Asp Tyr Ser Gly Pro Phe Ala Tyr Trp Gly Gln Gly Thr Leu
            100                 105                 110

Val Thr Val Ser Ser
        115

## Claims

1. A monoclonal antibody or an antigen-binding fragment thereof that specifically binds to human CD96, wherein the monoclonal antibody comprises a heavy chain variable region and a light chain variable region, wherein:
(i) the heavy chain variable region comprises HCDR variants selected from the group consisting of 3, 2, 1 or 0 amino acid mutations on the basis of the HCDR1, HCDR2 and HCDR3, respectively, wherein the HCDR1, HCDR2 and HCDR3 are shown in SEQ ID NO: 16, 17 and 18, respectively; the light chain variable region comprises LCDR variants selected from the group consisting of 3, 2, 1 or 0 amino acid mutations on the basis of the LCDR1, LCDR2 and LCDR3, respectively, wherein the LCDR1, LCDR2 and LCDR3 are shown in SEQ ID NO: 19, 20 and 21, respectively; or
(ii) the heavy chain variable region comprises HCDR variants selected from the group consisting of 3, 2, 1 or 0 amino acid mutations on the basis of the HCDR1, HCDR2 and HCDR3, respectively, wherein the HCDR1, HCDR2 and HCDR3 are shown in SEQ ID NO: 22, 23 and 24, respectively; the light chain variable region comprises LCDR variants selected from the group consisting of 3, 2, 1 or 0 amino acid mutations on the basis of the LCDR1, LCDR2 and LCDR3, respectively, wherein the LCDR1, LCDR2 and LCDR3 are shown in SEQ ID NO: 25, 26 and 27, respectively; or
(iii) the heavy chain variable region comprises HCDR variants selected from the group consisting of 3, 2, 1 or 0 amino acid mutations on the basis of the HCDR1, HCDR2 and HCDR3, respectively, wherein the HCDR1, HCDR2 and HCDR3 are shown in SEQ ID NO: 28, 29 and 30, respectively; the light chain variable region comprises LCDR variants selected from the group consisting of 3, 2, 1 or 0 amino acid mutations on the basis of the LCDR1, LCDR2 and LCDR3, respectively, wherein the LCDR1, LCDR2 and LCDR3 are shown in SEQ ID NO: 31, 32 and 33, respectively; or
(iv) the heavy chain variable region comprises HCDR variants selected from the group consisting of 3, 2, 1 or 0 amino acid mutations on the basis of the HCDR1, HCDR2 and HCDR3, respectively, wherein the HCDR1, HCDR2 and HCDR3 are shown in SEQ ID NO: 34, 35 and 36, respectively; the light chain variable region comprises LCDR variants selected from the group consisting of 3, 2, 1 or 0 amino acid mutations on the basis of the LCDR1, LCDR2 and LCDR3, respectively, wherein the LCDR1, LCDR2 and LCDR3 are shown in SEQ ID NO: 37, 38 and 39, respectively; or
(v) the heavy chain variable region comprises HCDR variants selected from the group consisting of 3, 2, 1 or 0 amino acid mutations on the basis of the HCDR1, HCDR2 and HCDR3, respectively, wherein the HCDR1, HCDR2 and HCDR3 are shown in SEQ ID NO: 40, 41 and 42, respectively; the light chain variable region comprises LCDR variants selected from the group consisting of 3, 2, 1 or 0 amino acid mutations on the basis of the LCDR1, LCDR2 and LCDR3, respectively, wherein the LCDR1, LCDR2 and LCDR3 are shown in SEQ ID NO: 43, 44 and 45, respectively.

2. The monoclonal antibody or the antigen-binding fragment thereof according to claim 1, wherein:
(vi) the heavy chain variable region comprises HCDR1, HCDR2 and HCDR3 having the amino acid sequences of SEQ ID NO: 16, 17 and 18, respectively; the light chain variable region comprises LCDR1, LCDR2 and LCDR3 having the amino acid sequences of SEQ ID NO: 52, 20 and 21, respectively, wherein the LCDR1 is preferably selected from any one of SEQ ID NO: 19, 108, 109 and 110; or
(vii) the heavy chain variable region comprises HCDR1, HCDR2 and HCDR3 regions having the amino acid sequences of SEQ ID NO: 22, 23 and 64, respectively; the light chain variable region comprises LCDR1, LCDR2 and LCDR3 regions having the amino acid sequences of SEQ ID NO: 25, 26 and 27, respectively, wherein the HCDR3 is preferably selected from any one of SEQ ID NO: 24, 111, 112, 113, 114, 115 and 116; or
(viii) the heavy chain variable region comprises the HCDR1, HCDR2 and HCDR3 regions having the amino acid sequences of SEQ ID NO: 28, 29 and 30, respectively; the light chain variable region comprises the LCDR1, LCDR2 and LCDR3 regions having the amino acid sequences of SEQ ID NO: 31, 32 and 33, respectively; or
(ix) the heavy chain variable region comprises the HCDR1, HCDR2 and HCDR3 regions having the amino acid sequences of SEQ ID NO: 34, 35 and 36, respectively; the light chain variable region comprises the LCDR1, LCDR2 and LCDR3 regions having the amino acid sequences of SEQ ID NO: 37, 38 and 39, respectively; or
(x) the heavy chain variable region comprises the HCDR1, HCDR2 and HCDR3 regions having the amino acid sequences of SEQ ID NO: 40, 119 and 42, respectively; the light chain variable region comprises the LCDR1, LCDR2 and LCDR3 regions having the amino acid sequences of SEQ ID NO: 43, 44 and 45, respectively, wherein the HCDR2 is preferably as shown in SEQ ID NO: 41, 120 or 121.

3. The monoclonal antibody or the antigen-binding fragment thereof according to claim 1 or 2, wherein the monoclonal antibody or the antigen-binding fragment thereof is a recombinant antibody, preferably a murine antibody, a chimeric antibody or a humanized antibody.

4. The monoclonal antibody or the antigen-binding fragment thereof according to claim 3, comprising:
a. the heavy chain variable region having the sequence of any one of SEQ ID NO: 6, 46, 49, 50 and 51, or a variant thereof, and/or the light chain variable region having the sequence of any one of SEQ ID NO: 7, 47, 48, 53, 54, 55, 56, 57 and 58, or a variant thereof; or
b. the heavy chain variable region having the sequence of any one of SEQ ID NO: 8, 59, 62, 63, 65, 66, 67, 68, 69 and 70, or a variant thereof, and/or the light chain variable region having the sequence of any one of SEQ ID NO: 9, 60 and 61, or a variant thereof; or
c. the heavy chain variable region having the sequence of any one of SEQ ID NO: 10, or a variant thereof, and/or the light chain variable region having the sequence of any one of SEQ ID NO: 11, or a variant thereof; or
d. the heavy chain variable region having the sequence of any one of SEQ ID NO: 12, 71, 75, 76 and 77, or a variant thereof, and/or the light chain variable region having the sequence of any one of SEQ ID NO: 13, 72, 73 and 74, or a variant thereof; or
e. the heavy chain variable region having the sequence of any one of SEQ ID NO: 14, 78, 82, 83, 84, 122 and 123, or a variant thereof, and/or the light chain variable region having the sequence of any one of SEQ ID NO: 15, 79, 80 and 81, or a variant thereof;
wherein the variants in a to e have 1 to 10 amino acid mutations in the sequence of the framework region of the light chain variable region or the heavy chain variable region, and the mutation is preferably a back mutation.

5. The monoclonal antibody or the antigen-binding fragment thereof according to claim 4, wherein the antibody or the antigen-binding fragment thereof comprises:
f. the heavy chain variable region having the sequence selected from any one of SEQ ID NO: 6, 46, 49, 50 and 51 and the light chain variable region having the sequence selected from any one of SEQ ID NO: 7, 47, 48, 53, 54, 55, 56, 57 and 58; or
g. the heavy chain variable region having the sequence selected from any one of SEQ ID NO: 8, 59, 62, 63, 65, 66, 67, 68, 69 and 70 and the light chain variable region having the sequence selected from any one of SEQ ID NO: 9, 60 and 61; or
h. the heavy chain variable region having the sequence of SEQ ID NO: 10 and the light chain variable region having the sequence of SEQ ID NO: 11; or
i. the heavy chain variable region having the sequence selected from any one of SEQ ID NO: 12, 71, 75, 76 and 77 and the light chain variable region having the sequence selected from any one of SEQ ID NO: 13, 72, 73 and 74; or
j. the heavy chain variable region having the sequence selected from any one of SEQ ID NO: 14, 78, 82, 83, 84, 122 and 123 and the light chain variable region having the sequence selected from any one of SEQ ID NO: 15, 79, 80 and 81.

6. The monoclonal antibody or antigen-binding fragment thereof according to any one of claims 1 to 5, wherein the antibody is a full-length antibody, further comprising a human antibody constant region, preferably comprising a human antibody heavy chain constant region of SEQ ID NO: 117 and/or a human antibody light chain constant region of SEQ ID NO: 118.

7. The monoclonal antibody or the antigen-binding fragment thereof according to any one of claims 1 to 5, wherein the antigen-binding fragment is selected from the group consisting of Fab, Fab', F(ab')2, single chain variable fragment (scFv), dimerized domain V (diabody), disulfide stabilized Fv (dsFv) and CDR-containing peptides.

8. The monoclonal antibody or the antigen-binding fragment thereof according to any one of claims 1 to 7, wherein the antibody or the antigen-binding fragment binds to human CD96 with an affinity of a KD value of 1 × 10⁻⁷M to 1 × 10⁻¹²M as determined by, for example, surface plasmon resonance (BIACORE).

9. An isolated monoclonal antibody or the antigen-binding fragment thereof, competing with the monoclonal antibody or the antigen-binding fragment according to any one of claims 1 to 8 to bind to human CD96.

10. The monoclonal antibody or the antigen-binding fragment thereof according to claim 9, having at least one of the following characteristics:
i. binding to human CD96 with an affinity of a KD value of 1 × 10⁻⁷M to 1 × 10⁻¹²M as determined by, for example, surface plasmon resonance (BIACORE);
ii. cross-reacting with CD96 of cynomolgus monkey or rhesus monkey;
iii. blocking the binding of human CD96 to human CD155;
iv. increased activation of NK cells and/or T cells;
v. blocking the inhibition of NK cell activation induced by the binding of CD96 to CD155.

11. The monoclonal antibody or the antigen-binding fragment thereof according to claim 9 or 10, wherein the monoclonal antibody or the antigen-binding fragment thereof binds to a region in the extracellular region of human CD96 as shown by IAVYHPQYGFYCAYGRPCES.

12. A multispecific antibody comprising the light chain variable region and/or the heavy chain variable region of the monoclonal antibody or the antigen-binding fragment thereof according to any one of claims 1 to 11.

13. A single chain antibody comprising the light chain variable region and/or the heavy chain variable region of the monoclonal antibody or the antigen-binding fragment thereof according to any one of claims 1 to 11.

14. A pharmaceutical composition, which comprises a therapeutically effective amount of the monoclonal antibody or the antigen-binding fragment thereof according to any one of claims 1 to 11, or the multispecific antibody according to claim 12, or the single chain antibody according to claim 13, and one or more pharmaceutically acceptable carriers, diluents, buffers, or excipients.

15. An isolated nucleic acid molecule encoding the monoclonal antibody or the antigen-binding fragment thereof according to any one of claims 1 to 11, or the multispecific antibody according to claim 12, or the single chain antibody according to claim 13.

16. A recombinant vector comprising the isolated nucleic acid molecule according to claim 15.

17. A host cell transformed with the recombinant vector according to claim 16, wherein the host cell is selected from the group consisting of a prokaryotic cell and a eukaryotic cell, preferably a eukaryotic cell, more preferably a mammalian cell.

18. A method for producing the monoclonal antibody or the antigen-binding fragment thereof according to any one of claims 1 to 11, or the multispecific antibody according to claim 12, or the single chain antibody according to claim 13, wherein the method comprises culturing the host cell according to claim 15 in a medium to produce and accumulate the monoclonal antibody or the antigen-binding fragment thereof according to any one of claims 1 to 11, or the multispecific antibody according to claim 12, or the single chain antibody according to claim 13, and harvesting the monoclonal antibody or the antigen-binding fragment thereof, the multispecific antibody, or the single chain antibody from the culture.

19. A method for detecting or measuring human CD96 *in vitro,* wherein the method comprises using the monoclonal antibody or the antigen-binding fragment thereof according to any one of claims 1 to 11, or the multispecific antibody according to claim 12, or the single chain antibody according to claim 13.

20. A use of the monoclonal antibody or the antigen-binding fragment thereof according to any one of claims 1 to 11, or the multispecific antibody according to claim 12, or the single chain antibody according to claim 13 in the preparation of a reagent for detecting or measuring human CD96.

21. A method for reducing or alleviating immunosuppression, wherein the method comprises administering to a subject a therapeutically effective amount of the monoclonal antibody or the antigen-binding fragment thereof according to any one of claims 1 to 11, or the multispecific antibody according to claim 12, or the single chain antibody according to claim 13, or the pharmaceutical composition according to claim 14, or the isolated nucleic acid molecule according to claim 15.

22. A method for enhancing the activity of NK cells, wherein the method comprises a step of reducing CD96 activity using the monoclonal antibody or the antigen-binding fragment thereof according to any one of claims 1 to 11, or the multispecific antibody according to claim 12, or the single chain antibody according to claim 13, or the pharmaceutical composition according to claim 14, or the isolated nucleic acid molecule according to claim 15.

23. A method for treating diseases associated with human CD96, wherein the method comprises administering to a subject a therapeutically effective amount of the monoclonal antibody or the antigen-binding fragment thereof according to any one of claims 1 to 11, or the multispecific antibody according to claim 12, or the single chain antibody according to claim 13, or the pharmaceutical composition according to claim 14, or the isolated nucleic acid molecule according to claim 15, to treat diseases associated with human CD96, wherein the disease is preferably a tumor, a cancer or an infectious disease.

24. A use of the monoclonal antibody or the antigen-binding fragment thereof according to any one of claims 1 to 11, or the multispecific antibody according to claim 12, or the single chain antibody according to claim 13, or the pharmaceutical composition according to claim 14, or the isolated nucleic acid molecule according to claim 15 or the combination thereof in the preparation of a medicament for treating or preventing diseases or disorders, wherein the diseases or disorders is diseases associated with human CD96, preferably a tumor, a cancer or an infectious disease.

25. A medicament of the monoclonal antibody or the antigen-binding fragment thereof according to any one of claims 1 to 11, or the multispecific antibody according to claim 12, or the single chain antibody according to claim 13, or the pharmaceutical composition according to claim 14, or the isolated nucleic acid molecule according to claim 15.

26. The medicament of the monoclonal antibody or the antigen-binding fragment thereof according to any one of claims 1 to 11, or the multispecific antibody according to claim 12, or the single chain antibody according to claim 13, or the pharmaceutical composition according to claim 14, or the isolated nucleic acid molecule according to claim 15, according to claim 25, wherein the medicament is used for the treatment of diseases associated with human CD96, wherein the disease is preferably a tumor, a cancer or an infectious disease.
